# EUROPEAN PATENT SPECIFICATION

(11) **EP 0 852 626 B1**
(45) Date of publication and mention of the grant of the patent: **03.04.2002**
(21) Application number: 97907549.6
(22) Date of filing: 29.01.1997
(51) Int. Cl.: C12Q 1/02, C12N 15/49, C12N 15/64, C12N 15/86

(54) **COMPOSITIONS AND METHODS FOR DETERMINING ANTI-VIRAL DRUG SUSCEPTIBILITY AND RESISTANCE AND ANTI-VIRAL DRUG SCREENING**
VERFAHREN UND ZUSAMMENSETZUNGEN ZUR BESTIMMUNG DER EMPFINDLICHKEIT UND RESISTENZ GEGEN ANTIVIRALE WIRKSTOFFE UND SCREENING FÜR ANTIVIRALE WIRKSTOFFE
COMPOSITIONS ET PROCEDES POUR DETERMINER LA SENSIBILITE ET LA RESISTANCE VIS-A-VIS DE MEDICAMENTS ANTIVIRAUX ET CRIBLAGE DE MEDICAMENTS ANTIVIRAUX

(30) Priority: 29.01.1996 US 593009
(43) Date of publication of application: 15.07.1998
(60) Divisional application: 01117002.4
(73) Proprietor: Virologic, Inc., South San Francisco, CA 94080 (US)
(72) Inventor: CAPON, Daniel, J., Hillsborough, CA 94010 (US); PETROPOULOUS, Christos, John, Half Moon Bay, CA 94019 (US)
(74) Representative: Armitage, Ian Michael
(86) International application number: US9701609
(87) International publication number: WO9727319

(56) References cited:
- EP-A- 0 291 893
- EP-A- 0 361 749
- EP-A- 0 372 904
- EP-A- 0 414 475
- WO-A-95/05453
- WO-A-95/22622
- WO-A-95/29703
- WO-A-95/30763
- WO-A-99/06597
- FR-A- 2 700 169
- US-A- 5 439 809
- US-A- 5 837 464
- MORGENSTERN J P ET AL: "ADVANCED MAMMALIAN GENE TRANSFER: HIGH TITRE RETROVIRAL VECTORS WITH MULTIPLE DRUG SELECTION MARKERS AND A COMPLEMENTARY HELPER -FREE PACKAGING CELL LINE" NUCLEIC ACIDS RESEARCH, vol. 18, no. 12, 1 January 1990 (1990-01-01), pages 3587-3596, XP002073969
- KELLAM P ET AL.: "Fifth mutation in human immunodeficiency virus type 1 reverse transcriptase contributes to the development of high-level resistance to zidovudine" PROCEEDINGS OF THE NATIONAL ACADEMY OF SCIENCES USA, vol. 89, 1992, pages 1934-1938, XP002107338
- CONDRA J H ET AL.: "In vivo emergence of HIV-1 variants resistant to multiple protease inhibitors" NATURE, vol. 374, 1995, pages 569-571, XP002107339
- VIROLOGY, 1993, Vol. 197, SALTARELLI M.J. et al., "The CAEV tat Gene Trans-activates the Viral LTR and is Necessary for Efficient Viral Replication", pages 35-44.
- ANTIMICROBIAL AGENTS AND CHEMOTHERAPY, January 1994, Vol. 38, KELLAM P. et al., "Recombinant Virus Assay: a Rapid, Phenotypic Assay for Assessment of Drug Susceptibility of Human Immunodeficiency Virus Type 1 Isolates", pages 23-30.

## Description

### Technical Field

This invention relates to anti-viral drug susceptibility and resistance tests to be used in identifying effective drug regimens for the treatment of viral infections. The invention further relates to novel vectors, host cells and compositions for carrying out these novel anti-viral drug susceptibility and resistance tests. This invention also relates to the screening of candidate drugs for their capacity to inhibit selected viral sequences and/or viral proteins. More particularly, the invention relates to the use of recombinant DNA technology to first construct a resistance test vector comprising a patient-derived segment and an indicator gene, then introducing the resistance test vector into a host cell, and determining the expression or inhibition of the indicator gene product in a target host cell in the presence of an anti-viral drug. This invention is also related to the means and methods of identifying anti-viral drugs which have distinct patterns of resistance when compared with existing anti-viral drugs. This invention also relates to methods and compositions for the identification and assessment of the biological effectiveness of potential therapeutic compounds. This invention is more particularly related to drug susceptibility and resistance tests useful in providing an optimal therapeutic regimen for the treatment of various viral diseases, including for example, HIV/AIDS and hepatitis.

### Background of the Invention

### Viral Drug Resistance

The use of anti-viral compounds for chemotherapy and chemoprophylaxis of viral diseases is a relatively new development in the field of infectious diseases, particularly when compared with the more than 50 years of experience with antibacterial antibiotics. The design of anti-viral compounds is not straightforward because viruses present a number of unique problems. Viruses must replicate intracellularly and often employ host cell enzymes, macromolecules, and organelles for the synthesis of virus particles. Therefore, safe and effective anti-viral compounds must be able to discriminate with a high degree of efficiency between cellular and virus-specific functions. In addition, because of the nature of virus replication, evaluation of the *in vitro* sensitivity of virus isolates to anti-viral compounds must be carried out in a complex culture system consisting of living cells (e.g. tissue culture). The results from such assay systems vary widely according to the type of tissue culture cells which are employed and the conditions of assay. Despite these complexities nine drugs have been approved for AIDS therapy, five reverse transcriptase inhibitors AZT, ddI, ddC, d4T, 3TC, one non-nucleoside reverse transcriptase inhibitor, nevirapine and three protease inhibitors saquinavir, ritonavir and indinovir and several additional anti-viral drug candidates have been recently developed such as nelfinavir, delaviridine, VX-478 and 1592.

Viral drug resistance is a substantial problem given the high rate of viral replication and mutation frequencies. Drug resistant mutants were first recognized for poxviruses with thiosemicarbazone (Appleyard and Way (1966) *Brit. J. Exptl*. *Pathol.* **47**, 144-51), for poliovirus with guanidine (Melnick et al. (1961) *Science* **134**, 557), for influenza A virus with amantadine (Oxford et al. (1970) *Nature* **226**, 82-83; Cochran et al. (1965) *Ann. NY Acad Sci* **130**, 423-429) and for herpes simplex virus with iododeoxyuridine (Jawetz et al. (1970) *Ann. NY Acad Sci* **173**, 282-291). Approximately 75 HIV drug resistance mutations to various anti-viral agents have been identified to date (Mellors et al. (1995) *Intnl. Antiviral News,* supplement and Condra, J.H. et al. (1996) *J Virol.* **70**, 8270-8276).

The small and efficient genomes of viruses have lent themselves to the intensive investigation of the molecular genetics, structure and replicative cycles of most important human viral pathogens. As a consequence, the sites and mechanisms have been characterized for both the activity of and resistance to anti-viral drugs more precisely than have those for any other class of drugs. (Richman (1994) *Trends Microbiol*. **2**, 401-407). The likelihood that resistant mutants will emerge is a function of at least four factors: 1) the viral mutation frequency; 2) the intrinsic mutability of the viral target site with respect to a specific anti-viral; 3) the selective pressure of the anti-viral drug; and, 4) the magnitude and rate of virus replication. With regard to the first factor, for single stranded RNA viruses, whose genome replication lacks a proofreading mechanism, the mutation frequencies are approximately 3x10⁻⁵ per base-pair per replicative cycle (Holland et al. (1992) *Curr. Topics Microbiol Immunol*. **176**, 1-20; Mansky et al. (1995) *J Virol*. **69**, 5087-94; Coffin (1995) *Science* **267**, 483-489). Thus, a single 10 kilobase genome, like that of human immunodeficiency virus (HIV), would be expected to contain on average one mutation for every three progeny viral genomes. As to the second factor, the intrinsic mutability of the viral target site in response to a specific anti-viral agent can significantly affect the likelihood of resistant mutants. For example, zidovudine (AZT) selects for mutations in the reverse transcriptase of HIV more readily *in vitro* and *in vivo* than does the other approved thymidine analog d4T (stavudine).

One, perhaps inevitable consequence of the action of an anti-viral drug is that it confers sufficient selective pressure on virus replication to select for drug-resistant mutants (Herrmann et al. (1977) *Ann NY Acad Sci* **284,** 632-7). With respect to the third factor, with increasing drug exposure, the selective pressure on the replicating virus population increases to promote the more rapid emergence of drug resistant mutants. For example, higher doses of AZT tend to select for drug resistant virus more rapidly than do lower doses (Richman et al. (1990) *J. AIDS.* **3**, 743-6). This selective pressure for resistant mutants increases the likelihood of such mutants arising as long as significant levels of virus replication are sustained.

The fourth factor, the magnitude and rate of replication of the virus population, has major consequences on the likelihood of emergence of resistant mutants. Many virus infections are characterized by high levels of virus replication with high rates of virus turnover. This is especially true of chronic infections with HIV as well as hepatitis B and C viruses. The likelihood of emergence of AZT resistance increases in HIV-infected patients with diminishing CD4 lymphocyte counts which are associated with increasing levels of HIV replication (Ibid).

Higher levels of virus increase the probability of preexisting mutants. It has been shown that the emergence of a resistant population results from the survival and selective proliferation of a previously existing subpopulation that randomly emerges in the absence of selective pressure. All viruses have a baseline mutation rate. With calculations of approximately 10¹⁰ new virions being generated daily during HIV infection (Ho et al. (1995) Nature **373,** 123-126), a mutation rate of 10⁻⁴ to 10⁻⁵ per nucleotide guarantees the preexistence of almost any mutation at any time point during HIV infection. Evidence is accumulating that drug resistant mutants do in fact exist in subpopulations of HIV infected individuals (Najera et al. (1994) *AIDS Res Hum Retroviruses* **10**, 1479-88; Najera et al. (1995) *J Virol*. **69**, 23-31). The preexistence of drug resistant picornavirus mutants at a rate of approximately 10⁻⁵ is also well documented (Ahmad et al. (1987) *Antiviral Res.* **8**, 27-39).

### Human Immunodeficiency Virus (HIV)

Acquired immune deficiency syndrome (AIDS) is a fatal human disease, generally considered to be one of the more serious diseases to ever affect humankind. Globally, the numbers of human immunodeficiency virus (HIV) infected individuals and of AIDS cases increase relentlessly and efforts to curb the course of the pandemic, some believe, are of limited effectiveness. Two types of HIV are now recognized: HIV-1 and HIV-2. By December 31, 1994 a total of 1,025,073 AIDS cases had been reported to the World Health Organization. This is only a portion of the total cases, and WHO estimates that as of late 1994, allowing for underdiagnosis, underreporting and delays in reporting, and based on the estimated number of HIV infections, there have been over 4.5 million cumulative AIDS cases worldwide (Mertens et al. (1995) *AIDS* **9** (Suppl A). S259-S272). Since HIV began its spread in North America, Europe and sub-Saharan Africa, over 19.5 million men, women and children are estimated to have been infected (Ibid). One of the distinguishing features of the AIDS pandemic has been its global spread within the last 20 years, with about 190 countries reporting AIDS cases today. The projections of HIV infection worldwide by the WHO are staggering. The projected cumulative total of adult AIDS cases by the year 2000 is nearly 10 million. By the year 2000, the cumulative number of HIV-related deaths in adults is predicted to rise to more than 8 million from the current total of around 3 million.

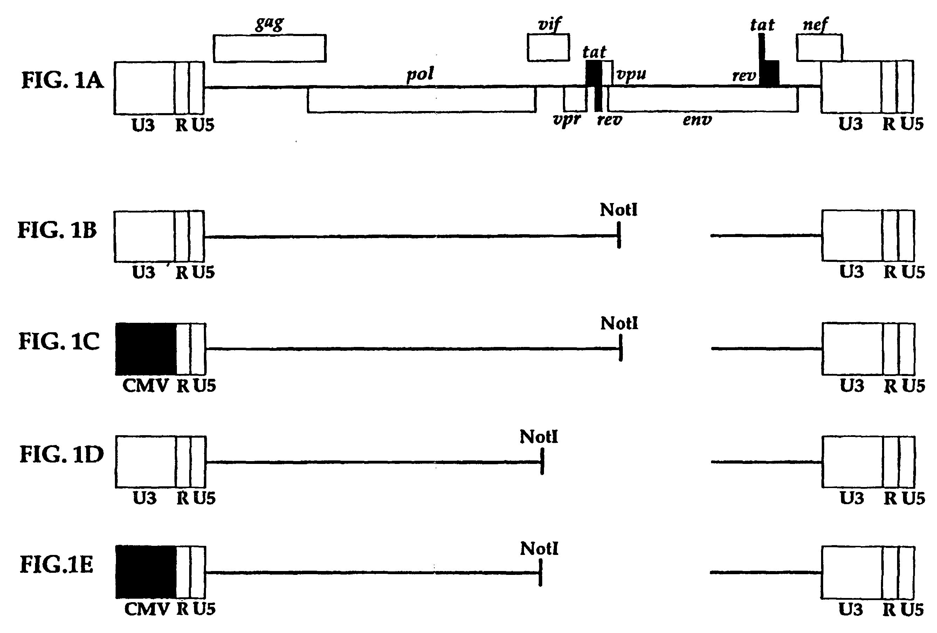

HIV-1 and HIV-2 are enveloped retroviruses with a diploid genome having two identical RNA molecules. The molecular organization of HIV is (5') U3-R-U5-*gag-pol-env*-U3-R-U5 (3') as shown in Fig. 1a. The U3, R, and U5 sequences form the long terminal repeats (LTR) which are the regulatory elements that promote the expression of the viral genes and sometimes nearby cellular genes in infected hosts. The internal regions of the viral RNA code for the structural proteins: gag (p55, p17, p24 and p7 core proteins), *pol* (p10 protease, p66 and p51 reverse transcriptase and p32 integrase) and *env* (gp120 and gp41 envelope glycoproteins). *Gag* codes for a polyprotein precursor that is cleaved by a viral protease into three or four structural proteins; *pol* codes for reverse transcriptase (RT) and the viral protease and integrase; *env* codes for the transmembrane and outer glycoprotein of the virus. The *gag* and *pol* genes are expressed as a genomic RNA while the *env* gene is expressed as a spliced subgenomic RNA. In addition to the *env* gene there are other HIV genes produced by spliced subgenomic RNAs that contribute to the replication and biologic activities of the virus. These genes include: tat which encodes a protein that activates the expression of viral and some cellular genes; *rev* which encodes a protein that promotes the expression of unspliced or single-spliced viral mRNAs; *nef* which encodes a myristylated protein that appears to modulate viral production under certain conditions; *vif* which encodes a protein that affects the ability of virus particles to infect target cells but does not appear to affect viral expression or transmission by cell-to-cell contact; *vpr* which encodes a virion-associated protein; and vpu which encodes a protein that appears to promote the extracellular release of viral particles.

No disease better exemplifies the problem of viral drug resistance than AIDS. Drug resistant HIV isolates have been identified for nucleoside and non-nucleoside reverse transcriptase inhibitors and for protease inhibitors. The emergence of HIV isolates resistant to AZT is not surprising since AZT and other reverse transcriptase inhibitors only reduce virus replication by about 90%. High rates of virus replication in the presence of the selective pressure of drug treatment provide ideal conditions for the emergence of drug-resistant mutants. Patients at later stages of infection who have higher levels of virus replication develop resistant virus with AZT treatment more quickly than those at early stages of infection (Richman et al. (1990) *J AIDS* **3***,* 743-6). The initial description of the emergence of resistance to AZT identified progressive and stepwise reductions in drug susceptibility (Larder et al. (1989) *Science* **243,** 1731-1734). This was explained by the recognition of multiple mutations in the gene for reverse transcriptase that contributed to reduced susceptibility (Larder et al. (1989) *Science* **246,** 1155-1158). These mutations had an additive or even synergistic contribution to the phenotype of reduced susceptibility (Kellam et al. (1992) *Proc. Natl. Acad. Sci.* **89**, 1934-1938). The cumulative acquisition of such mutations resulted in progressive decreases in susceptibility. Similar effects have been seen with non-nucleoside reverse transcriptase inhibitors (Nunberg et al. (1991) *J Virol* **65**, 4887-4892; Sardanna et al. (1992) *J Biol Chem* **267**, 17526-17530). Studies of protease inhibitors have found that the selection of HIV strains with reduced drug susceptibility occurs within weeks (Ho et al. (1994) *J Virol* **68**, 2016-2020; Kaplan et al. (1994) *Proc. Natl. Acad. Sci.* **91**, 5597-5601). While recent studies have shown protease inhibitors to be more powerful than reverse transcriptase inhibitors, nevertheless resistance has developed. (Condra et al., Id. and Report 3rd Conference on Retroviruses and Opportunistic Infections, March 1996). Subtherapeutic drug levels, whether caused by reduced dosing, drug interactions, malabsorption or reduced bioavailability due to other factors, or self-imposed drug holidays, all permit increased viral replication and increased opportunity for mutation and resistance. (Id.)

The selective pressure of drug treatment permits the outgrowth of preexisting mutants. With continuing viral replication in the absence of completely suppressive anti-viral drug activity, the cumulative acquisition of multiple mutations can occur over time, as has been described for AZT and protease inhibitors of HIV. Indeed viral mutants multiply resistant to different drugs have been observed (Larder et al. (1989) Science **243**, 1731-1734; Larder et al. (1989) *Science* **246**, 1155-1158; Condra et al. (1995) *Nature* **374**, 569-71). With the inevitable emergence of resistance in many viral infections, as with HIV for example, strategies must be designed to optimize treatment in the face of resistant virus populations. Ascertaining the contribution of drug resistance to drug failure is a difficult problem because patients who are more likely to develop drug resistance are more likely to have other confounding factors that will predispose them to a poor prognosis (Richman (1994) *AIDS Res Hum Retroviruses* **10,** 901-905). In addition patients contain mixtures of viruses with different susceptibilities.

### Presently Available Viral Resistance Assays

The definition of viral drug susceptibility is generally understood to be the concentration of the anti-viral agent at which a given percentage of viral replication is inhibited (e.g. the IC₅₀ for an anti-viral agent is the concentration at which 50% of virus replication is inhibited). Thus, a decrease in viral drug susceptibility is the hallmark that an anti-viral has selected for mutant virus that is resistant to that anti-viral drug. Viral drug resistance is generally defined as a decrease in viral drug susceptibility in a given patient over time. In the clinical context, viral drug resistance is evidenced by the anti-viral drug being less effective or no longer being clinically effective in a patient.

At present the tools available to the researcher and clinician to assess anti-viral drug susceptibility and resistance are inadequate. The classical test for determining the resistance and sensitivity of HIV to an anti-viral agent is complex, time-consuming, expensive, and is hazardous in that it requires the culture of pathogenic virus from each and every patient (Barre-Sinoussi et al (1983) *Science* **220**, 868-871; Popovic et al. (1984) *Science* **224**, 497-500). In this procedure, the patient's peripheral blood mononuclear cells (PBMC) are first cultured to establish a viral stock of known multiplicity of infection (moi), and the viral stock thereby produced is used to infect a target indicator cell line. The resulting burst of viral replication is then typically measured in the presence and absence of an anti-viral agent by determining the production of viral antigens in the cell culture. Such tests can be performed reliably only in the hands of expert investigators, and may take two to three months to carry out at a cost of thousands of dollars per patient for each agent tested. Furthermore, as viral stocks of sufficient moi cannot be established from the PBMC of some HIV patients, the classical test for HIV resistance cannot be performed on all HIV-infected individuals. More significantly, in the course of generating the viral stock by passage of the virus in culture, the characteristics of the viruses themselves can change and may therefore obscure the true nature of the patient's virus. Thus, the application of the classical test has been limited to gathering information about trends in clinical trials and has not been available for a prospective analysis which could be used to custom tailor anti-viral therapy for a given patient. Notwithstanding these limitations, the classical test has two important qualities: it is *specific* for the agent under evaluation, and it provides information on the *phenotype* of the patient's own virus, that is, the concentration of the drug which inhibits 50% of viral replication (IC₅₀).

A number of attempts have been made to improve upon the classical test, but each of these has serious shortcomings. The first type of these tests can be described as *nonspecific* in that they do not determine the characteristics of a patient's own virus at all, but rather provide an independent measure of the course of the infection. Among these tests are those which measure the patient's CD4⁺ T cell count, the hallmark of HIV disease progression (Goedert et al. (1987) *JAMA* **257,** 331-334), those which measure viral antigen levels (e.g., p24 core antigen (Allain et al. (1987) *N. Engl. J. Med.* **317**, 1114-1121)), and those which measure viral RNA and DNA levels (e.g., quantitative polymerase chain reaction and branched DNA assays (Piatak et al. (1993) *Science* **259**, 1749-1754; Urdea (1993) *Clin. Chem*. **39**, 725-726)). The primary disadvantage of such nonspecific tests is that they do not provide any information on viral drug resistance per se, but rather attempt to infer this information from the apparent course of the patient's disease. In addition, many factors other than viral drug resistance can affect the level of the parameter under consideration. In other words, CD₄⁺ T cell counts, p24 antigen levels and HIV viral RNA levels can vary for reasons other than drug resistance during the course of disease.

Another modified classical test amplifies the viral gene that is the target of the anti-viral agent. In this test the viral gene from a given patient is amplified and then recombined into a biologically active proviral clone of HIV. This proviral clone is transfected into human cells to generate a viral stock of known moi which can then be used to infect a target indicator cell line. In the manner of the classical test, one then determines the production of viral antigens in the presence or absence of the anti-viral agent. One such assay described by Kellam and Larder (1994) *Antimicrobial Agents and Chemo*. **38**, 23-30, involves PCR amplification of reverse transcriptase coding sequences from a patient, which is then introduced into a proviral DNA clone by homologous recombination to reconstitute the complete viral genome including the reverse transcriptase gene which was deleted. The resulting recombinant virus produced from such clones is then cultured in T-cell lines, and the drug sensitivity is tested in the HeLa CD4* plaque reduction assay. However, this class of test still requires the culturing of virus to determine drug resistance, and is thus difficult, lengthy and costly and requires the laboratory investigator to handle hazardous viral cultures. Furthermore, given the attendant variation of the virus itself during the culture process, the results may be correspondingly inaccurate.

A second class of test attempts to provide specific information on the genotype of the patient's HIV, with the ultimate goal of correlating this genotypic information with the virus' drug resistant phenotype. Indeed, specific amino acid substitutions within viral genes such as reverse transcriptase and protease genes have been shown to correspond to specific levels of viral resistance to reverse transcriptase and protease inhibitors, respectively (Larder et al. (1994) *J. Gen Virol.* **75,** 951-957). A major shortcoming associated with such an analysis is that it is indirect and can be obfuscated by secondary mutations which have been shown to add to or counter the effects of the first mutation. It is the complex interplay of all amino acid residues within a given viral polypeptide which ultimately determines the gene product's activity in the presence or absence of an inhibitor. Thus, a database of vast and impractical proportions would be necessary to interpret the status of drug resistance or sensitivity of a given genotype, given the number of potential amino acid changes in the HIV genome.

A third class of test, a recently developed bacterial-based assay makes use of a molecularly cloned viral gene (specifically, the reverse transcriptase gene) which has been inserted into a bacterial expression vector. Upon transformation of special strains of *E. coli* which are deficient in the bacterial DNA polymerase I, the cloned reverse transcriptase gene can rescue the growth of the bacteria under selected growth conditions. In making *E.* *coli* dependent upon reverse transcriptase for their growth, one can ascertain the effects of certain reverse transcriptase inhibitors on the activity of the viral gene (PCT Application No. WO 95/22622). A major shortcoming with this approach, however, is that the inhibitor may be transported across the cell membrane and metabolized differently by the bacteria than it is by a human cell, and as a result the concentration of the true metabolic inhibitor of the reverse transcriptase may be grossly different in the bacterium than it would be in a relevant human cell target of infection, or the true inhibitor may be absent altogether. Indeed, nucleoside metabolism is known to differ markedly between human and bacterial cells. Another significant shortcoming of this approach is that the assay measures DNA-dependent DNA polymerase activity of reverse transcriptase but not the RNA-dependent DNA polymerase, strand transfer or RNAse H activities of the reverse transcriptase. Thus, an anti-viral compound which acts, at least in part, on these other activities would not have its full inhibitory activity in this assay. Yet another difficulty with this approach is that it is a growth-based test; thus if an inhibitor (eg., a nucleoside analog) also blocks bacterial growth for reasons other than its effects on reverse transcriptase, it can not be adequately tested in this system.

### Viral Vectors

Viral vectors and particularly retroviral vectors have been used for modifying mammalian cells because of the high efficiency with which retroviral vectors infect target cells and integrate into the target cell genome. Because of their ability to insert into the genome of mammalian cells much attention has focused on retroviral vectors for use in gene therapy. Details on retroviral vectors and their use can be found in patents and patent publications including European Patent Application EPA 0 178 220, U.S. Patent 4,405,712, PCT Application WO 92/07943, U.S. Patent 4,980,289, U.S. Patent 5,439,809 and PCT Application WO 89/11539.

One consequence of the emphasis on retroviral vector technology has been the development of packaging cell lines. A major problem with the use of retroviruses is the possibility of the spread of replication-competent retrovirus. There is thus a need for producing helper vectors which could not be processed into virions. As a result packaging defective vectors and packaging cell lines were developed. Details on packaging-defective vectors and packaging cell lines can be found in patents and patent publications, including U.S. Patent 5,124,263, European Patent Application Pub. No. 0386 882, PCT Application No. WO 91/19798, PCT Application No. WO 88/08454, PCT Application No. WO 93/03143, U.S. Patent No. 4,650,764, U.S. Patent 4,861,719 and U.S. Patent 5,278,056.

It is an object of this invention to provide a drug susceptibility and resistance test capable of showing whether a viral population in a patient is resistant to a given prescribed drug. Another object of this invention is to provide a test that will enable the physician to substitute one or more drugs in a therapeutic regimen for a patient that has become resistant to a given drug or drugs after a previous course of therapy. Yet another object of this invention is to provide a test that will enable selection of an effective drug regimen for the treatment of virus infections. Yet another object of this invention is to provide a safe, standardized, affordable, rapid, precise and reliable assay of drug susceptibility and resistance for clinical and research application. Still another object of this invention is to provide a test and methods for evaluating the biological effectiveness of candidate drug compounds which act on specific viral genes and/or viral proteins particularly with respect to viral drug resistance and cross resistance. It is also an object of this invention to provide the means and compositions for evaluating viral drug resistance and susceptibility. This and other objects of this invention will be apparent from the specification as a whole.

### Summary of the Invention

Objects of the present invention are accomplished by a novel test for determining susceptibility for an anti-HIV drug comprising: (a) introducing a resistance test vector comprising a HIV patient-derived segment and an indicator gene into a host cell; (b) culturing the host cell from step (a); (c) measuring expression of the indicator gene in a target host cell wherein the expression of the indicator gene is dependent upon the HIV patient-derived segment; and (d) comparing the expression of the indicator gene from step (c) with the expression of the indicator gene measured when steps (a)-(c) are carried out in the absence of the anti-HIV drug, wherein a test concentration of the anti-HIV drug is present at steps (a)-(c); at steps (b)-(c); or at step (c).

This invention also provides a method for determining anti-HIV drug resistance in a patient comprising:(a) developing a standard curve of drug susceptibility for an anti-HIV drug; (b) determining anti-HIV drug susceptibility in the patient using the susceptibility test described above; and (c) comparing the anti-HIV drug susceptibility in step (b) with the standard curve determined in step (a), wherein a decrease in anti-HIV susceptibility indicates development of anti-HIV drug resistance in the patient.

This invention further provides a method for determining anti-HIV drug resistance in a patient comprising: (a) determining anti-HIV drug susceptibility in the patient at a first time according to the above method, wherein the patient-derived segment is obtained from the patient at said time; (b) determining anti-HIV drug susceptibility of the same patient at a later time; and (c) comparing the anti-HIV drug susceptibilities determined in step (a) and (b), wherein a decrease in anti-HIV drug susceptibility at the later time compared to the first time indicates the development or progression of anti-HIV drug resistance in the patient.

The assay of this invention enables a physician to assess whether a viral gene encoding a viral protein or a functional viral sequence, each of which may be the target of an anti-viral agent, has mutated to render the drug less effective. More particularly, the novel assay of this invention enables one to determine whether a virus has become resistant to a particular anti-viral drug. Furthermore, this invention enables a physician to assess drug susceptibility and resistance of combination therapy. In addition the assay enables one to alter a therapeutic regimen prospectively by testing particular drug(s) or combinations of drugs and determining whether these drugs, alone or in combination, inhibit one or more viral gene(s) and/or viral protein(s). This invention provides significant advantages over presently available assays by providing a safer, more affordable, more reliable, more rapid and more effective drug susceptibility and resistance assay to assess the therapeutic efficacy of particular anti-viral drug(s) or combinations of drugs enabling a physician to optimize treatment. The assay of this invention has the significant advantage of enabling the evaluation of resistance and susceptibility at all stages of drug development: 1) during preclinical evaluation of candidate compounds; 2) during clinical evaluation of new drugs;. 3) during patient therapy enabling design of an effective therapeutic regimen to overcome the problem of drug resistance; and 4) as part of epidemiologic surveillance, evaluating the prevalence of resistance during the use of approved and experimental drugs.

The present invention is directed to the methods and compositions of assessing drug susceptibility and resistance, including: a) the assay method of determining the susceptibility and resistance of a patient-derived segment to an anti-HIV drug; b) compositions including resistance test vectors comprising a HIV patient-derived segment and an indicator gene; and c) host cells containing the resistance test vectors. This invention is further directed to the compositions and methods of constructing the vectors and host cells which are used in the drug susceptibility and resistance assay of this invention.

In one aspect of the invention there is provided a method for determining susceptibility for an anti-HIV drug comprising: (a) introducing a resistance test vector comprising a patient-derived segment and an indicator gene into a host cell; (b) culturing the host cell from step (a) ; (c) measuring expression of the indicator gene in a target host cell wherein the expression of the indicator gene is dependent upon the HIV patient derived segment; and (d) comparing the expression of the indicator gene from step (c) with the expression of the indicator gene measured when steps (a)-(c) are carried out in the absence of the anti-HIV drug, wherein a test concentration of the anti-HIV drug is present at steps (a) - (c) ; at steps (b) - (c); or at step (c).

In one aspect of the invention there is provided a method for determining susceptibility for an anti-HIV drug comprising: (a) introducing a resistance test vector comprising a patient-derived segment and an indicator gene into a host cell; (b) culturing the host cell from step (a) ; (c) measuring an indicator in a target host cell wherein a change in the indicator is dependent upon the HIV patient-derived segment; and wherein said indicator is a DNA or RNA structure; and (d) comparing the measurement of the indicator from step (c) with the measurement of the indicator when steps (a)-(c) are carried out in the absence of the anti-HIV drug, wherein a test concentration of the anti-HIV drug is present at steps (a)-(c); at steps (b)-(c); or at step (c).

This invention also provides a method for determining anti-HIV drug resistance in a patient comprising:(a) developing a standard curve of drug susceptibility for an anti-HIV drug; (b) determining anti-HIV drug susceptibility in the patient using the susceptibility test described above; and (c) comparing the anti-HIV drug susceptibility in step (b) with the standard curve determined in step (a), wherein a decrease in anti-HIV susceptibility indicates development of anti-HIV drug resistance in the patient.

This invention also provides a method for evaluating the biological effectiveness of a candidate anti-HIV drug compound comprising; (a) introducing a resistance test vector comprising a HIV patient-derived segment and an indicator gene into a host cell; (b) culturing the host cell from step (a); (c) measuring expression of the indicator gene in a target host cell wherein the expression of the indicator gene is dependent upon the HIV patient derived segment; and (d) comparing the expression of the indicator gene from step (c) with the expression of the indicator gene measured when steps (a)-(c) are carried out in the absence of the candidate anti-HIV drug compound, wherein a test concentration of the candidate anti-HIV drug compound is present at steps (a)-(c); at steps (b)-(c); or at step (c).

This invention also provides a method for evaluating the biological effectiveness of a candidate anti-HIV drug compound comprising: (a) introducing a resistance test vector comprising a HIV patient-derived segment and an indicator gene into a host cell; (b) culturing the host cell from step (a); (c) measuring an indicator in a target host cell wherein a change in the indicator is dependent upon the HIV patient-derived segment; wherein said indicator is a DNA or RNA structure; and (d) comparing the measurement of the indicator from step (c) with the measurement of the indicator when steps (a)-(c) are carried out in the absence of the candidate anti-HIV drug compound, wherein a test concentration of the candidate anti-HIV drug compound is present at steps (a)-(c); at steps (b)-(c); or at step (c).

The resistance test vector comprising the HIV patient-derived viral segment(s) and the indicator gene may additionally include one or more non-patient-derived segments. In one embodiment the resistance test vector is constructed from a genomic viral vector which may include a deletion in one or more genes. For example, *env* is deleted in a resistance test vector which otherwise preserves the mRNA expression and processing characteristics of the complete virus. Alternatively, the resistance test vector is constructed from a subgenomic viral vector which may include only one or a few viral genes which are typically the target of the anti-viral drug. The resistance test vector further comprises either the native enhancer/promoter of the particular virus or a foreign enhancer/promoter for the expression of the anti-viral target genes.

The expression of the indicator gene in the resistance test vector in the target cell is ultimately dependent upon the action of the patient-derived segment sequences. The indicator gene may be functional or non-functional. In the case of a non-functional indicator gene, the indicator gene is not efficiently expressed in a host cell transfected by the resistance test vector until it is converted into a functional indicator gene through the action of one or more of the patient-derived segment products. In one embodiment, the indicator gene is rendered non-functional through use of a permuted promoter, i.e. a promoter that, although in the same transcriptional orientation as the indicator gene, follows rather than precedes the coding sequence of the indicator gene. In addition, the orientation of the non-functional indicator gene is opposite to that of the viral promoters or LTRs. Thus the coding sequence of the non-functional indicator gene can neither be transcribed by the permuted promoter nor by the viral promoters. The indicator gene is rearranged as a consequence of reverse transcription so that the permuted promoter now precedes the indicator gene sequence, which as a result can be functionally expressed. In a second embodiment, the indicator gene is rendered non-functional through use of a permuted coding region, i.e. an indicator gene coding region in which the 5' portion of the coding region follows rather than precedes the 3' portion of the coding region. In this configuration, no mRNA is expressed which can give rise to a functional indicator gene product. The indicator gene is rearranged as a consequence of reverse transcription so that the 5' coding region of the indicator gene now precedes the 3' coding region, and as a result the indicator gene can be functionally expressed. In a third embodiment, the indicator gene is rendered non-functional through use of an inverted intron, i.e. an intron inserted into the coding sequence of the indicator gene with a transcriptional orientation opposite to that of the indicator gene. In addition, the indicator gene itself contains a functional promoter with the entire transcriptional unit oriented opposite to the viral promoters. Thus the non-functional indicator gene is in the wrong orientation to be transcribed by the viral LTRs and it cannot be functionally transcribed by its own promoter, as the inverted intron cannot be properly excised by splicing. However, transcription by the viral promoters result in the formation of mRNA in which removal of the inverted intron can occur by splicing. In retroviruses, as a consequence of reverse transcription of the resulting spliced transcript and the integration of the resulting provirus into the host cell chromosome, the indicator gene can now be functionally transcribed by its own promoter.

Resistance test vectors comprising a non-functional indicator gene can be used to carry out resistance tests in either a particle-based or non-particle-based assay. The particle based assay is based on resistance test vector viral particles, which are replication defective, being produced by the resistance test vector host cells. The trans-acting factors necessary for production of the resistance test vector viral particles are provided by the packaging expression vectors which are transfected into the packaging host cell. In contrast the non-particle based resistance test is performed by transfection of a single host cell with a resistance test vector in the absence of packaging expression vectors.

In the case of the functional indicator gene, the functional indicator gene is efficiently expressed in a first host cell transfected by the resistance test vector (referred to herein as a resistance test vector host cell). Thus, the function of the indicator gene in the resistance test vector host cell is not dependent on the patient-derived segment. However, the capacity of the indicator gene to be expressed in a second host cell (referred to herein as a target host cell) is dependent on the production of functional resistance test vector viral particles in the resistance test vector host cell. Thus, the activity of the indicator gene in the target host cells is dependent on the activity of the patient-derived segments.

In another aspect this invention is directed to anti-viral drug susceptibility and resistance tests for HIV/AIDS. Particular resistance test vectors of the invention for use in the HIV/AIDS anti-viral drug susceptibility and resistance test are identified as well as resistance test vector host cells.

In yet another aspect this invention provides for the identification and assessment of the biological effectiveness of potential therapeutic anti-HIV compounds for the treatment of viral diseases. In still another aspect the invention is directed to a host cell transfected with one or more vectors to assess drug susceptibility. In another aspect, the invention is directed to a novel resistance test vector comprising a HIV patient-derived viral gene(s) and an indicator gene.

### Brief Description of the Drawings

Fig. 1.
   A. Diagrammatic representation of the DNA genomic structure of HIV-1. Viral proteins are encoded in each of the three reading frames by the *gag, pol, vif, vpr, tat, rev, vpu, env and nef* genes. The RNA is transcribed from viral DNA and processed by viral and cellular enzymes, giving rise to both genomic viral RNA and mRNA. The U3, R and U5 elements of the viral long terminal repeat (LTR) are indicated.
   B. Generalized diagrammatic representation of the HIV genomic viral vector which contains the following elements in a 5' to 3' orientation: 1) an HIV-LTR U3 region, 2) an HIV-LTR R region, 3) an HIV-LTR U5 region , 4) the coding regions of the HIV *gag-pol, vif, vpr, tat, rev, vpu,* deleted *env,* and *nef* genes, and 5) a 3' HIV-LTR.
   C. Generalized diagrammatic representation of the HIV genomic viral vector which contains the following elements in a 5' to 3' orientation: 1) a CMV IE enhancer-promoter, 2) an HIV-LTR R region, 3) an HIV-LTR U5 region, 4) the coding regions of the HIV *gag-pol, vif, vpr, tat, rev, vpu,* deleted *env,* and *nef* genes, and 5) a 3' HIV-LTR.
   D. Generalized diagrammatic representation of the HIV subgenomic viral vector which contains the following elements in a 5' to 3' orientation: 1) an HIV-LTR U3 region, 2) an HIV-LTR R region, 3) an HIV-LTR U5 region, 4) the coding region of the HIV *gag-pol* gene, and 5) a 3' HIV- LTR.
   E. Generalized diagrammatic representation of the HIV subgenomic viral vector which contains the following elements in a 5' to 3' orientation: 1) a CMV IE enhancer-promoter, 2) an HIV-LTR R region, 3) an HIV-LTR U5 region, 4) the coding region of the HIV *gag-pol* gene, and 5) a 3' HIV-LTR.
Fig. 2.
   A. Diagrammatic representation of the DNA genomic structure of HIV-1.
   B. Diagrammatic representation of the resistance test vector comprising a nonfunctional indicator gene comprising a permuted promoter having the following elements in a 5' to 3' orientation: 1) an HIV-LTR U3 region (pLG-lucPP-HS and pLG-lucPP-PB) or a CMV IE enhancer-promoter (pCG-lucPP-HS and pCG-lucPP-PB), 2) an HIV-LTR R region, 3) an HIV-LTR U5 region containing an inserted T7 phage RNA polymerase promoter (herein referred to as T7 promoter) with a transcriptional orientation opposite to that of the LTRs, 4) the coding regions of the HIV *gag-pol, vif, vpr, tat, rev, vpu*, deleted *env*, and *nef* genes, 5) a patient-derived segment(s) inserted into the patient sequence acceptor sites, 6) an indicator gene cassette inserted into the deleted env gene, and 7) a 3' HIV-LTR.
   C. Diagrammatic representation showing the conversion of the non-functional indicator gene (permuted promoter) in the resistance test vector, described in 2(a), to a functional indicator gene by reverse transcriptase. The conversion to a functional indicator gene results from the repositioning of the T7 promoter relative to the indicator gene coding region.
Fig. 3.
   A. Diagrammatic representation of the DNA genomic structure of HIV-1.
   B. Diagrammatic representation of the packaging expression vector pLTR-HIV3' which provides the *vif, vpr, tat, rev, vpu* and *nef* genes, each of which is expressed as a spliced subgenomic mRNA transcribed from the HIV LTR U3 region.
   C. Diagrammatic representation of the packaging expression vector pCMV-HIV3' which provides the *vif, vpr, tat, rev, vpu* and *nef* genes, each of which is expressed as a spliced subgenomic mRNA transcribed from the CMV IE enhancer-promoter.
   D. Diagrammatic representation of the packaging expression vector pVL-*env*4070A [pCXAS (4070A *env*)] which provides the amphotrophic MLV *env* gene product, by transcription from the CMV IE enhancer-promoter.
Fig. 4.
   A. Diagrammatic representation of the DNA genomic structure of HIV-1.
   B. A generalized diagrammatic representation of the resistance test vectors comprising a nonfunctional indicator gene comprising a permuted coding region containing the following elements in a 5' to 3' orientation: 1) an HIV-LTR U3 region (pLG-lucPC-HS and pLG-lucPC-PB) or a first CMV IE enhancer-promoter (pCG-lucPC-HS and pCG-lucPC-PB), 2) the HIV-LTR R and U5 regions, 3) the coding regions of the HIV *gag-pol, vif, vpr, tat, rev, vpu,* deleted *env,* and *nef* genes, 4) a patient-derived segment(s) inserted into the patient sequence acceptor sites, 5) a first indicator gene cassette containing the 5' coding region of the luciferase gene, inserted into the deleted *env* gene, 6) a second indicator gene cassette containing the 3' coding region of the luciferase gene, inserted into a deleted 3' HIV-LTR U3 region, and 7) a 3' HIV-LTR R and U5 region.
   C. Diagrammatic representation showing the conversion of the non-functional indicator gene (permuted coding region) in the resistance test vector, described in 4(a), to a functional indicator gene by reverse transcriptase. Following reverse transcription and strand transfer, the luciferase 3' coding region is copied from the 3' LTR to the 5' LTR, permitting the transcription of mRNA which can be spliced to generate a functional luciferase coding region.
Fig. 5.
   A. Diagrammatic representation of the DNA genomic structure of HIV-1.
   B. A generalized diagrammatic representation of the resistance test vectors comprising a nonfunctional indicator gene comprising an inverted intron containing the following elements in a 5' to 3' orientation: 1) an HIV-LTR U3 region (pLG-lucII-HS and pLG-lucII-PB) or a first CMV IE enhancer-promoter (pCG-lucII-HS and pCG-lucII-PB), 2) the HIV-LTR R and U5 regions, 3) the coding regions of the HIV gag-*pol, vif, vpr, tat*, *rev, vpu,* deleted *env,* and *nef* genes, 4) patient-derived segment(s) inserted into the patient sequence acceptor site, 5) an indicator gene cassette inserted into the deleted *env* gene, and 5) a 3' HIV-LTR.
   C. Diagrammatic representation showing the conversion of the non-functional indicator gene (inverted intron) in the resistance test vector, described in 5(a), to a functional indicator gene by reverse transcriptase. The overall transcriptional orientation of the indicator gene cassette is opposite to that of the first CMV enhancer-promoter and viral LTRs, while the orientation of the artificial intron is the same as the latter elements. Transcription of the indicator gene by the second CMV enhancer-promoter does not lead to the production of functional transcripts as the inverted intron cannot be spliced in this orientation. Transcription of the indicator gene by the 5' viral LTR or the first CMV IE enhancer-promoter, however, leads to the removal of the inverted intron by RNA splicing, although the indicator gene is still not functionally expressed as the resulting transcript has an antisense orientation. Following the reverse transcription of this transcript and integration of the resultant proviral DNA, the indicator gene can be functionally transcribed by the second CMV enhancer-promoter as the inverted intron has been previously removed.
Fig. 6.
   A. Diagrammatic representation of the DNA genomic structure of HIV-1 is shown above (a) and (b).
   B. A generalized diagrammatic representation of the resistant test vectors comprising a functional indicator gene having the following elements in a 5' to 3' orientation: 1) an HIV-LTR U3 region (pLG-luc-HS-1 and pLG-luc-PB-1) or a first CMV IE enhancer-promoter (pCG-luc-HS-1 and pCG-luc-PB-1), 2) the HIV-LTR R and U5 regions, 3) the coding region of the HIV *gag-pol, vif, vpr, tat, rev, vpu*, deleted *env*, and *nef* genes, 4) a functional indicator gene cassette inserted into the deleted *env* gene, with a transcriptional orientation opposite to the viral LTRs and 5) a 3' HIV-LTR.
   C. A generalized diagrammatic representation of the resistance test vectors comprising a functional indicator gene cassette (pLG-luc-HS-2, pLG-luc-PB-2, pCG-luc-HS-2 and pCG-luc-PB-2) in which the transcriptional orientation of the indicator gene cassette is the same as the viral LTRs.
Fig. 7.
   A. Demonstration of drug susceptibility using the resistance test vectors, pCG-CXCN(F-lucP)2-AA and pCG-CXAT(F-lucP)2-AA. Data are presented as luciferase gene activity in target host cells as Relative Light Units (RLU) in the absence of AZT or in the presence of 5 mM AZT.
   B. Drug susceptibility and resistance test performed with the resistance test vector, pCG-CXCN(F-lucP)2-AA containing pre-AZT treatment and post-AZT treatment "test" patient-derived segments. The resistance test vectors are derived from the genomic indicator gene viral vector, pCG-CXCN(F-lucP)2-AA. Data are presented as percent inhibition of luciferase gene activity in target host cells vs. AZT concentration (log₁₀). pCG-CXCN(F-lucP)2-AA is plotted as solid boxes. pCG-CXCN(F-lucP)2-AA containing patient-derived segments prior to AZT treatment is plotted as solid circles and post-AZT treatment as solid triangles.
   C. Drug susceptibility and resistance test performed with the resistance test vector, pCG-CXCN(F-lucP)2-AA containing the reverse transcriptase segment derived from the biologically active proviral clone, pNL4-3. Data are presented as percent inhibition of luciferase gene activity in target host cells vs. nevirapine concentration (log₁₀) and is plotted as solid boxes.
   D. Drug susceptibility and resistance test performed with the resistance test vector, pCG-CXCN(F-lucP)2-AA containing the protease segment derived from the biologically active proviral clone, pNL4-3. Data are presented as percent inhibition of luciferase gene activity in target host cells vs. indinavir concentration (log₁₀)and is plotted as solid boxes.

### Detailed Description of the Invention

In order that the invention described herein may be more fully understood, the following description is set forth.

The present invention provides a novel drug susceptibility and resistance assay comprising the steps of: (a) introducing a resistance test vector comprising a HIV patient-derived segment and an indicator gene into a host cell; (b) culturing the host cell from step (a); (c) measuring expression of the indicator gene in a target host cell wherein the expression of the indicator gene is dependent upon the HIV patient-derived segment; and (d) comparing the expression of the indicator gene from step (c) with the expression of the indicator gene measured when steps (a)-(c) are carried out in the absence of the anti-HIV drug, wherein a test concentration of the anti-HIV drug is present at steps (a)-(c); at steps (b)-(c); or at step (c).

In one aspect of the invention there is provided a method for determining susceptibility for an anti-HIV drug comprising: (a) introducing a resistance test vector comprising a HIV patient-derived segment and an indicator gene into a host cell; (b) culturing the host cell from step (a); (c) measuring an indicator in a target host cell wherein a change in the indicator is dependent upon the HIV patient-derived segment; and wherein said indicator is a DNA or RNA structure; and (d) comparing the measurement of the indicator from step (c) with the measurement of the indicator when steps (a)-(c) are carried out in the absence of the anti-HIV drug, wherein a test concentration of the anti-HIV drug is present at steps (a)-(c); at steps (b)-(c); or at step (c).

This invention also provides a method for determining anti-HIV drug resistance in a patient comprising:(a) developing a standard curve of drug susceptibility for an anti-HIV drug; (b) determining anti-HIV drug susceptibility in the patient using either of the susceptibility tests described above; and (c) comparing the anti-HIV drug susceptibility in step (b) with the standard curve determined in step (a), wherein a decrease in anti-HIV susceptibility indicates development of anti-HIV drug resistance in the patient.

This invention also provides a method for determining anti-HIV drug resistance in a patient comprising: (a) determining anti-HIV drug susceptibility in the patient at a first time according to either of the above methods, wherein the HIV patient-derived segment is obtained from the patient at about said time; (b) determining anti-HIV drug susceptibility of the same patient at a later time; and (c) comparing the anti-HIV drug susceptibilities determined in step (a) and (b), wherein a decrease in anti-HIV drug susceptibility at the later time compared to the first time indicates development or progression of anti-HIV drug resistance in the patient.

The assay of this invention can be used for HIV, where anti-viral drug susceptibility and resistance is a concern.

The structure, life cycle and genetic elements of the HIV virus which could be tested in the drug susceptibility and resistance test of this invention would be known to one of ordinary skill in the art. It is useful to the practice of this invention, for example, to understand the life cycle of a retrovirus, as well as the viral genes required for retrovirus rescue and infectivity. Retrovirally infected cells shed a membrane virus containing a diploid RNA genome. The virus, studded with an envelope glycoprotein (which serves to determine the host range of infectivity), attaches to a cellular receptor in the plasma membrane of the cell to be infected. After receptor binding, the virus is internalized and uncoated as it passes through the cytoplasm of the host cell. Either on its way to the nucleus or in the nucleus, the reverse transcriptase molecules resident in the viral core drive the synthesis of the double-stranded DNA provirus, a synthesis that is primed by the binding of a tRNA molecule to the genomic viral RNA. The double-stranded DNA provirus is subsequently integrated in the genome of the host cell, where it can serve as a transcriptional template for both mRNAs encoding viral proteins and virion genomic RNA, which will be packaged into viral core particles. On their way out of the infected cell, core particles move through the cytoplasm, attach to the inside of the plasma membrane of the newly infected cell, and bud, taking with them tracts of membrane containing the virally encoded envelope glycoprotein gene product. This cycle of infection - reverse transcription, transcription, translation, virion assembly, and budding - repeats itself over and over again as infection spreads.

The viral RNA and, as a result, the proviral DNA encode several cis-acting elements that are vital to the successful completion of the viral lifecycle. The virion RNA carries the viral promoter at its 3' end. Replicative acrobatics place the viral promoter at the 5' end of the proviral genome as the genome is reverse transcribed. Just 3' to the 5' retroviral LTR lies the viral packaging site. The retroviral lifecycle requires the presence of virally encoded transacting factors. The viral-RNA-dependent DNA polymerase (*pol*)-reverse transcriptase is also contained within the viral core and is vital to the viral life cycle in that it is responsible for the conversion of the genomic RNA to the integrative intermediate proviral DNA. The viral envelope glycoprotein, *env*, is required for viral attachment to the uninfected cell and for viral spread. There are also transcriptional trans-activating factors, so called transactivators, that can serve to modulate the level of transcription of the integrated parental provirus. Typically, replication-competent (non-defective) viruses are self-contained in that they encode all of these trans-acting factors. Their defective counterparts are not self-contained.

The following flow chart illustrates certain of the various vectors and host cells which may be used in this invention. It is not intended to be all inclusive.

### Vectors

### Host Cells

Packaging Host Cell - transfected with packaging expression vectors
Resistance Test Vector Host Cell - a packaging host cell transfected with a resistance test vector
Target Host Cell - a host cell to be infected by a resistance test vector viral particle produced by the resistance test vector host cell

### Resistance Test Vector

"Resistance test vector" means one or more vectors which taken together contain DNA or RNA comprising a patient-derived segment and an indicator gene. In the case where the resistance test vector comprises more than one vector the patient-derived segment may be contained in one vector and the indicator gene in a different vector. Such a resistance test vector comprising more than one vector is referred to herein as a resistance test vector system for purposes of clarity but is nevertheless understood to be a resistance test vector. The DNA or RNA of a resistance test vector may thus be contained in one or more DNA or RNA molecules. In one embodiment, the resistance test vector is made by insertion of a patient-derived segment into an indicator gene viral vector. In another embodiment, the resistance test vector is made by insertion of a patient-derived segment into a packaging vector while the indicator gene is contained in a second vector, for example an indicator gene viral vector. As used herein, "patient-derived segment" refers to one or more viral segments obtained directly from a patient using various means, for example, molecular cloning or polymerase chain reaction (PCR) amplification of a population of patient-derived segments using viral DNA or complementary DNA (cDNA) prepared from viral RNA, present in the cells (e.g. peripheral blood mononuclear cells, PBMC), serum or other bodily fluids of infected patients. When a viral segment is "obtained directly" from a patient it is obtained without passage of the virus through culture, or if the virus is cultured, then by a minimum number of passages to essentially eliminate the selection of mutations in culture. The term "viral segment" refers to any functional viral sequence or viral gene encoding a gene product (e.g., a protein) that is the target of an anti-viral drug. The term "functional viral sequence" as used herein refers to any nucleic acid sequence (DNA or RNA) with functional activity such as enhancers, promoters, polyadenylation sites, sites of action of trans-acting factors, such as *tar* and RRE, packaging sequences, integration sequences, or splicing sequences. If a drug were to target more than one functional viral sequence or viral gene product then patient-derived segments corresponding to each said viral gene would be inserted in the resistance test vector. In the case of combination therapy where two or more anti-virals targeting two different functional viral sequences or viral gene products are being evaluated, patient-derived segments corresponding to each functional viral sequence or viral gene product would be inserted in the resistance test vector. The patient-derived segments are inserted into unique restriction sites or specified locations, called patient sequence acceptor sites, in the indicator gene viral vector or for example, a packaging vector depending on the particular construction being used as described herein.

As used herein, "patient-derived segment" encompasses segments derived from human and various animal species. Such species include, but are not limited to chimpanzees, horses, cattles, cats and dogs.

Patient-derived segments can also be incorporated into resistance test vectors using any of several alternative cloning techniques. For example, cloning via the introduction of class II restriction sites into both the plasmid backbone and the patient-derived segments or by uracil DNA glycosylase primer cloning (refs).

The patient-derived segment may be obtained by any method of molecular cloning or gene amplification, or modifications thereof, by introducing patient sequence acceptor sites, as described below, at the ends of the patient-derived segment to be introduced into the resistance test vector. For example, in a gene amplification method such as PCR, restriction sites corresponding to the patient-sequence acceptor sites can be incorporated at the ends of the primers used in the PCR reaction. Similarly, in a molecular cloning method such as cDNA cloning, said restriction sites can be incorporated at the ends of the primers used for first or second strand cDNA synthesis, or in a method such as primer-repair of DNA, whether cloned or uncloned DNA, said restriction sites can be incorporated into the primers used for the repair reaction. The patient sequence acceptor sites and primers are designed to improve the representation of patient-derived segments. Sets of resistance test vectors having designed patient sequence acceptor sites provide representation of patient-derived segments that would be underrepresented in one resistance test vector alone.

Resistance test vectors are prepared by modifying an indicator gene viral vector (described below) by introducing patient sequence acceptor sites, amplifying or cloning patient-derived segments and inserting the amplified or cloned sequences precisely into indicator gene viral vectors at the patient sequence acceptor sites. The resistance test vectors are constructed from indicator gene viral vectors which are in turn derived from genomic viral vectors or subgenomic viral vectors and an indicator gene cassette, each of which is described below. Resistance test vectors are then introduced into a host cell. Alternatively, a resistance test vector (also referred to as a resistance test vector system) is prepared by introducing patient sequence acceptor sites into a packaging vector, amplifying or cloning patient-derived segments and inserting the amplified or cloned sequences precisely into the packaging vector at the patient sequence acceptor sites and co-transfecting this packaging vector with an indicator gene viral vector.

In one preferred embodiment, the resistance test vector may be introduced into packaging host cells together with packaging expression vectors, as defined below, to produce resistance test vector viral particles that are used in drug resistance and susceptibility tests that are referred to herein as a "particle-based test." In an alternative preferred embodiment, the resistance test vector may be introduced into a host cell in the absence of packaging expression vectors to carry out a drug resistance and susceptibility test that is referred to herein as a "non-particle-based test." As used herein a "packaging expression vector" provides the factors, such as packaging proteins (e.g. structural proteins such as core and envelope polypeptides), transacting factors, or genes required by replication-defective retrovirus or hepadnavirus. In such a situation, a replication-competent viral genome is enfeebled in a manner such that it cannot replicate on its own. This means that, although the packaging expression vector can produce the trans-acting or missing genes required to rescue a defective viral genome present in a cell containing the enfeebled genome, the enfeebled genome cannot rescue itself.

### Indicator or Indicator Gene

"Indicator or indicator gene" refers to a nucleic acid encoding a protein, DNA or RNA structure that either directly or through a reaction gives rise to a measurable or noticeable aspect, e.g. a color or light of a measurable wavelength or in the case of DNA or RNA used as an indicator a change or generation of a specific DNA or RNA structure. Preferred examples of an indicator gene is the *E. coli lac*Z gene which encodes beta-galactosidase, the *luc* gene which encodes luciferase either from, for example, *Photonis pyralis* (the firefly) or *Renilla reniformis* (the sea pansy), the *E. coli phoA* gene which encodes alkaline phosphatase, green fluorescent protein and the bacterial CAT gene which encodes chloramphenicol acetyltransferase. Additional preferred examples of an indicator gene are secreted proteins or cell surface proteins that are readily measured by assay, such as radioimmunoassay (RIA), or fluorescent activated cell sorting (FACS), including, for example, growth factors, cytokines and cell surface antigens (e.g. growth hormone, Il-2 or CD4, respectively). "Indicator gene" is understood to also include a selection gene, also referred to as a selectable marker. Examples of suitable selectable markers for mammalian cells are dihydrofolate reductase (DHFR), thymidine kinase, hygromycin, neomycin, zeocin or *E. coli* gpt. In the case of the foregoing examples of indicator genes, the indicator gene and the patient-derived segment are discrete, i.e. distinct and separate genes. In some cases a patient-derived segment may also be used as an indicator gene. In one such embodiment in which the patient-derived segment corresponds to more than one viral gene which is the target of an anti-viral, one of said viral genes may also serve as the indicator gene. For example, a viral protease gene may serve as an indicator gene by virtue of its ability to cleave a chromogenic substrate or its ability to activate an inactive zymogen which in turn cleaves a chromogenic substrate, giving rise in each case to a color reaction. In all of the above examples of indicator genes, the indicator gene may be either "functional" or "non-functional" but in each case the expression of the indicator gene in the target cell is ultimately dependent upon the action of the patient-derived segment.

### Functional Indicator Gene

In the case of a "functional indicator gene" the indicator gene may be capable of being expressed in a "packaging host cell/resistance test vector host cell" as defined below, independent of the patient-derived segment, however the functional indicator gene could not be expressed in the target host cell, as defined below, without the production of functional resistance test vector particles and their effective infection of the target host cell. In one embodiment of a functional indicator gene, the indicator gene cassette, comprising control elements and a gene encoding an indicator protein, is inserted into the indicator gene viral vector with the same or opposite transcriptional orientation as the native or foreign enhancer/promoter of the viral vector. One example of a functional indicator gene in the case of HIV or HBV, places the indicator gene and its promoter (a CMV IE enhancer/promoter) in the same or opposite transcriptional orientation as the HIV-LTR or HBV enhancer-promoter, respectively, or the CMV IE enhancer/promoter associated. with the viral vector.

### Non-Functional Indicator Gene

Alternatively the indicator gene, may be "non-functional" in that the indicator gene is not efficiently expressed in a packaging host cell transfected with the resistance test vector, which is then referred to a resistance test vector host cell, until it is converted into a functional indicator gene through the action of one or more of the patient-derived segment products. An indicator gene is rendered non-functional through genetic manipulation according to this invention.
1. Permuted Promoter In one embodiment an indicator gene is rendered non-functional due to the location of the promoter, in that, although the promoter is in the same transcriptional orientation as the indicator gene, it follows rather than precedes the indicator gene coding sequence. This misplaced promoter is referred to as a "permuted promoter." In addition to the permuted promoter the orientation of the non-functional indicator gene is opposite to that of the native or foreign promoter/enhancer of the viral vector. Thus the coding sequence of the non-functional indicator gene can neither be transcribed by the permuted promoter nor by the viral promoters. The non-functional indicator gene and its permuted promoter is rendered functional by the action of one or more of the viral proteins. One example of a non-functional indicator gene with a permuted promoter in the case of HIV, places a T7 phage RNA polymerase promoter (herein referred to as T7 promoter) promoter in the 5' LTR in the same transcriptional orientation as the indicator gene. The indicator gene cannot be transcribed by the T7 promoter as the indicator gene cassette is positioned upstream of the T7 promoter. The non-functional indicator gene in the resistance test vector is converted into a functional indicator gene by reverse transcriptase upon infection of the target cells, resulting from the repositioning of the T7 promoter, by copying from the 5' LTR to the 3' LTR, relative to the indicator gene coding region. Following the integration of the repaired indicator gene into the target cell chromosome by HIV integrase, a nuclear T7 RNA polymerase expressed by the target cell transcribes the indicator gene.
   A permuted promoter may be any eukaryotic or prokaryotic promoter which can be transcribed in the target host cell. Preferably the promoter will be small in size to enable insertion in the viral genome without disturbing viral replication. More preferably, a promoter that is small in size and is capable of transcription by a single subunit RNA polymerase introduced into the target host cell, such as a bacteriophage promoter, will be used. Examples of such bacteriophage promoters and their cognate RNA polymerases include those of phages T7, T3 and Sp6. A nuclear localization sequence (NLS) may be attached to the RNA polymerase to localize expression of the RNA polymerase to the nucleus where they may be needed to transcribed the repaired indicator gene. Such an NLS may be obtained from any nuclear-transported protein such as the SV40 T antigen. If a phage RNA polymerase is employed, an internal ribosome entry site (IRES) such as the EMC virus 5' untranslated region (UTR) may be added in front of the indicator gene, for translation of the transcripts which are generally uncapped. The permuted promoter itself can be introduced at any position within the 5' LTR that is copied to the 3' LTR during reverse transcription so long as LTR function is not disrupted, preferably within the U5 and R portions of the LTR, and most preferably outside of functionally important and highly conserved regions of U5 and R.
   Blocking sequences may be added at the ends of the resistance test vector should there be inappropriate expression of the non-functional indicator gene due to transfection artifacts (DNA concatenation). In the HIV example of the permuted T7 promoter given above, such a blocking sequence may consist of a T7 transcriptional terminator, positioned to block readthrough transcription resulting from DNA concatenation, but not transcription resulting from repositioning of the permuted T7 promoter from the 5' LTR to the 3' LTR during reverse transcription.
2. Permuted Coding Region In a second embodiment, an indicator gene is rendered non-functional due to the relative location of the 5' and 3' coding regions of the indicator gene, in that, the 3' coding region precedes rather than follows the 5' coding region. This misplaced coding region is referred to as a "permuted coding region." The orientation of the non-functional indicator gene may be the same or opposite to that of the native or foreign promoter/enhancer of the viral vector, as mRNA coding for a functional indicator gene will be produced in the event of either orientation. The non-functional indicator gene and its permuted coding region is rendered functional by the action of one or more of the patient-derived segment products. A second example of a non-functional indicator gene with a permuted coding region places a 5' indicator gene coding region with an associated promoter in the 3' LTR U3 region and a 3' indicator gene coding region in an upstream location of the HIV genome, with each coding region having the same transcriptional orientation as the viral LTRs. In both examples, the 5' and 3' coding regions may also have associated splice donor and acceptor sequences, respectively, which may be heterologous or artificial splicing signals. The indicator gene cannot be functionally transcribed either by the associated promoter or viral promoters, as the permuted coding region prevents the formation of functionally spliced transcripts. The non-functional indicator gene in the resistance test vector is converted into a functional indicator gene by reverse transcriptase upon infection of the target cells, resulting from the repositioning of the 5' and 3' indicator gene coding regions relative to one another, by copying of the 3' LTR to the 5' LTR. Following transcription by the promoter associated with the 5' coding region, RNA splicing can join the 5' and 3' coding regions to produce a functional indicator gene product.
3. Inverted Intron In a third embodiment, the indicator gene is rendered non-functional through use of an "inverted intron," i.e. an intron inserted into the coding sequence of the indicator gene with a transcriptional orientation opposite to that of the indicator gene. The overall transcriptional orientation of the indicator gene cassette including its own, linked promoter, is opposite to that of the viral control elements, while the orientation of the artificial intron is the same as the viral control elements. Transcription of the indicator gene by its own linked promoter does not lead to the production of functional transcripts as the inverted intron cannot be spliced in this orientation. Transcription of the indicator gene by the viral control elements does, however, lead to the removal of the inverted intron by RNA splicing, although the indicator gene is still not functionally expressed as the resulting transcript has an antisense orientation. Following the reverse transcription of this transcript and integration of the resultant retroviral DNA, or the circularization of hepadnavirus DNA, the indicator gene can be functionally transcribed using its own linked promoter as the inverted intron has been previously removed. In this case, the indicator gene itself may contain its own functional promoter with the entire transcriptional unit oriented opposite to the viral control elements. Thus the non-functional indicator gene is in the wrong orientation to be transcribed by the viral control elements and it cannot be functionally transcribed by its own promoter, as the inverted intron cannot be properly excised by splicing However, in the case of a retrovirus and HIV specifically, transcription by the viral promoters (HIV LTR) results in the removal of the inverted intron by splicing. As a consequence of reverse transcription of the resulting spliced transcript and the integration of the resulting provirus into the host cell chromosome, the indicator gene can now be functionally transcribed by its own promoter. The inverted intron, consisting of a splice donor and acceptor site to remove the intron, is preferably located in the coding region of the indicator gene in order to disrupt translation of the indicator gene. The splice donor .and acceptor may be any splice donor and acceptor. A preferred splice donor-receptor is the CMV IE splice donor and the splice acceptor of the second exon of the human alpha globin gene ("intron A").

### Indicator Gene Viral Vector - Construction

As used herein, "indicator gene viral vector" refers to a vector(s) comprising an indicator gene and its control elements and one or more viral genes. The indicator gene viral vector is assembled from an indicator gene cassette and a "viral vector," defined below. The indicator gene viral vector may additionally include an enhancer, splicing signals, polyadenylation sequences, transcriptional terminators, or other regulatory sequences. Additionally the indicator gene viral vector may be functional or nonfunctional. In the event that the viral segments which are the target of the anti-viral drug are not included in the indicator gene viral vector they are provided in a second vector. An "indicator gene cassette" comprises an indicator gene and control elements. "Viral vector" refers to a vector comprising some or all of the following: viral genes encoding a gene product, control sequences, viral packaging sequences, and in the case of a retrovirus, integration sequences. The viral vector may additionally include one or more viral segments one or more of which may be the target of an anti-viral drug. Two examples of a viral vector which contain viral genes are referred to herein as an "genomic viral vector" and a "subgenomic viral vector." A "genomic viral vector" is a vector which may comprise a deletion of a one or more viral genes to render the virus replication incompetent, but which otherwise preserves the mRNA expression and processing characteristics of the complete virus. In one embodiment for an HIV drug susceptibility and resistance test, the genomic viral vector comprises the HIV *gag-pol, vif, vpr, tat, rev, vpu*, and *nef* genes (some, most or all of *env* may be deleted). A "subgenomic viral vector" refers to a vector comprising the coding region of one or more viral genes which may encode the proteins that are the target(s) of the anti-HIV drug. A preferred embodiment is a subgenomic viral vector comprising the HIV *gag-pol* gene. Two examples of proviral clones used for viral vector construction are: HXB2 (Fisher et al., (1986) *Nature,* **320**, 367-371) and NL4-3, (Adachi et al., (1986) *J. Virol*., **59**, 284-291). The viral coding genes may be under the control of a native enhancer/promoter or a foreign viral or cellular enhancer/promoter. A preferred embodiment for an HIV drug susceptibility and resistance test, is to place the genomic or subgenomic viral coding regions under the control of the native enhancer/promoter of the HIV-LTR U3 region or the CMV immediate-early (IE) enhancer/promoter. In the case of an indicator gene viral vector that contains one or more viral genes which are the targets or encode proteins which are the targets of an anti-viral drug(s) then said vector contains the patient sequence acceptor sites. The patient-derived segments are inserted in the patient sequence acceptor site in the indicator gene viral vector which is then referred to as the resistance test vector, as described above.

"Patient sequence acceptor sites" are sites in a vector for insertion of patient-derived segments and said sites may be: 1) unique restriction sites introduced by site-directed mutagenesis into a vector; 2) naturally occurring unique restriction sites in the vector; or 3) selected sites into which a patient-derived segment may be inserted using alternative cloning methods (e.g. UDG cloning). In one embodiment the patient sequence acceptor site is introduced into the indicator gene viral vector. The patient sequence acceptor sites are preferably located within or near the coding region of the viral protein which is the target of the anti-viral drug. The viral sequences used for the introduction of patient sequence acceptor sites are preferably chosen so that no change, or a conservative change, is made in the amino acid coding sequence found at that position. Preferably the patient sequence acceptor sites are located within a relatively conserved region of the viral genome to facilitate introduction of the patient-derived segments. Alternatively, the patient sequence acceptor sites are located between functionally important genes or regulatory sequences. Patient-sequence acceptor sites may be located at or near regions in the viral genome that are relatively conserved to permit priming by the primer used to introduce the corresponding restriction site into the patient-derived segment. To improve the representation of patient-derived segments further, such primers may be designed as degenerate pools to accommodate viral sequence heterogeneity, or may incorporate residues such as deoxyinosine (I) which have multiple base-pairing capabilities. Sets of resistance test vectors having patient sequence acceptor sites that define the same or overlapping restriction site intervals may be used together in the drug resistance and susceptibility tests to provide representation of patient-derived segments that contain internal restriction sites identical to a given patient sequence acceptor site, and would thus be underrepresented in either resistance test vector alone.

### Host Cells

The resistance test vector is introduced into a host cell. Suitable host cells are mammalian cells. Preferred host cells are derived from human tissues and cells which are the principle targets of viral infection. These include human cells such as human T cells, monocytes, macrophage, dendritic cells, Langerhans cells, hematopoeitic stem cells or precursor cells, and other cells. Human derived host cells will assure that the anti-viral drug will enter the cell efficiently and be converted by the cellular enzymatic machinery into the metabolically relevant form of the anti-viral inhibitor. Host cells are referred to herein as a "packaging host cells," "resistance test vector host cells," or "target host cells." A "packaging host cell" refers to a host cell that provides the trans-acting factors and viral packaging proteins required by the replication defective viral vectors used herein, such as the resistance test vectors, to produce resistance test vector viral particles. The packaging proteins may be provided for by the expression of viral genes contained within the resistance test vector itself, a packaging expression vector(s), or both. A packaging host cell is a host cell which is transfected with one or more packaging expression vectors and when transfected with a resistance test vector is then referred to herein as a "resistance-test vector host cell" and is sometimes referred to as a packaging host cell/resistance test vector host cell. Preferred host cells for use as packaging host cells for HIV include 293 human embryonic kidney cells (293, Graham, F.L. et al., J. Gen Virol. 36: 59, 1977), BOSC23 (Pear et al., *Proc. Natl. Acad. Sci.* **90**, 8392, 1993), tsa54 and tsa201 cell lines (Heinzel et al., *J.Virol.* **62**, 3738, 1988). A "target host cell" refers to a cell to be infected by resistance test vector viral particles produced by the resistance test vector host cell in which expression or inhibition of the indicator gene takes place. Preferred host cells for use as target host cells include human T cell leukemia cell lines including Jurkat (ATCC T1B-152), H9 (ATCC HTB-176), CEM (ATCC CCL-119), HUT78 (ATCC T1B-161), and derivatives thereof.

### Drug Susceptibility and Resistance Tests

The drug susceptibility and resistance tests of this invention may be carried out in one or more host cells. Viral drug susceptibility is determined as the concentration of the anti-viral agent at which a given percentage of indicator gene expression is inhibited (e.g. the IC₅₀ for an anti-viral agent is the concentration at which 50% of indicator gene expression is inhibited). A standard curve for drug susceptibility of a given anti-viral drug can be developed for a viral segment that is either a standard laboratory viral segment or from a drug-naive patient (i.e. a patient who has not received any anti-viral drug) using the method of this invention. Correspondingly, viral drug resistance is a decrease in viral drug susceptibility for a given patient either by comparing the drug susceptibility to such a given standard or by making sequential measurement in the same patient over time, as determined by increased inhibition of indicator gene expression (i.e. decreased indicator gene expression).

In the first type of drug susceptibility and resistance test, resistance test vector viral particles are produced by a first host cell (the resistance test vector host cell) that is prepared by transfecting a packaging host cell with the resistance test vector and packaging expression vector(s). The resistance test vector viral particles are then used to infect a second host cell (the target host cell) in which the expression of the indicator gene is measured. Such a two cell system comprising a packaging host cell which is transfected with a resistance test vector, which is then referred to as a resistance test vector host cell, and a target cell are used in the case of either a functional or non-functional indicator gene. Functional indicator genes are efficiently expressed upon transfection of the packaging host cell and would require infection of a target host cell with resistance test vector host cell supernatant to carry out the test of this invention. Non-functional indicator genes with a permuted promoter, a permuted coding region, or an inverted intron are not efficiently expressed upon transfection of the packaging host cell and thus the infection of the target host cell can be achieved either by co-cultivation by the resistance test vector host cell and the target host cell or through infection of the target host cell using the resistance test vector host cell supernatant. In the second type of drug susceptibility and resistance test, a single host cell (the resistance test vector host cell) also serves as a target host cell. The packaging host cells are transfected and produce resistance test vector viral particles and some of the packaging host cells also become the target of infection by the resistance test vector particles. Drug susceptibility and resistance tests employing a single host cell type are possible with viral resistance test vectors comprising a non-functional indicator gene with a permuted promoter, a permuted coding region, or an inverted intron. Such indicator genes are not efficiently expressed upon transfection of a first cell, but are only efficiently expressed upon infection of a second cell, and thus provide an opportunity to measure the effect of the anti-viral agent under evaluation. In the case of a drug susceptibility and resistance test using a resistance test vector comprising a functional indicator gene, neither the co-cultivation procedure nor the resistance and susceptibility test using a single cell type can be used for the infection of target cells. A resistance test vector comprising a functional indicator gene requires a two cell system using filtered supernatants from the resistance test vector host cells to infect the target host cell.

In one embodiment of the invention, a particle-based resistance tests are carried out with resistance test vectors derived from genomic viral vectors, i.e., pLG-lucPP-HS, pCG-lucPP-HS, pLG-lucPP-PB, pCG-lucPP-PB, pLG-lucPC-HS, pCG-lucPC-HS, pLG-lucPC-PB, pCG-lucPC-PB, pLG-lucII-HS, pCG-lucII-HS, pLG-lucII-PB, pCG-lucII-PB, and pCG-CXCN(F-lucP)2-AA which are cotransfected with the packaging expression vector pVL-env4070A (also referred to as pCXAS-4070A*env*). Alternatively, a particle-based resistance test may be carried out with resistance test vectors derived from subgenomic viral vectors, i.e., pLS-lucPP-HS, pCS-lucPP-HS, pLS-lucPP-PB, pCS-luc-PP-PB, pLS-lucPC-HS, pCS-lucPC-HS, pLS-lucPC-PB, pCS-luc-PC-PB, pLS-lucII-HS, pCS-lucII-HS, pLS-lucII-PB, and pCS-luc-II-PB) which are cotransfected with the packaging expression vector pVL-env4070A and either pLTR-HIV3' or pCMV-HIV3'. In another embodiment of the invention, non-particle-based resistance tests are carried out using each of the above described resistance test vectors by transfection of selected host cells in the absence of packaging expression vectors.

In the case of the particle-based susceptibility and resistance test, resistance test vector viral particles are produced by a first host cell (the resistance test vector host cell) that is prepared by transfecting a packaging host cell with the resistance test vector and packaging expression vector(s). The resistance test vector viral particles are then used to infect a second host cell (the target host cell) in which the expression of the indicator gene is measured. In a second type of particle-based susceptibility and resistance test, a single host cell type (the resistance test vector host cell) serves both purposes: some of the packaging host cells in a given culture are transfected and produce resistance test vector viral particles and some of the host cells in the same culture are the target of infection by the resistance test vector particles thus produced. Resistance tests employing a single host cell type are possible with resistance test vectors comprising a non-functional indicator gene with a permuted promoter since such indicator genes are efficiently expressed upon infection of a permissive host cell, they are not efficiently expressed upon transfection of the same host cell type, and thus provide an opportunity to measure the effect of the anti-viral agent under evaluation. For similar reasons, resistance tests employing two cell types may be carried out by co-cultivating the two cell types as an alternative to infecting the second cell type with viral particles obtained from the supernatants of the first cell type.

In the case of the non-particle-based susceptibility and resistance test, resistance tests are performed by transfection of a single host cell with the resistance test vector in the absence of packaging expression vectors. Non-particle based resistance tests are carried out using the resistance test vectors comprising non-functional indicator genes with either permuted promoters, permuted coding regions or inverted introns. These non-particle based resistance tests are performed by transfection of a single host cell type with each resistance test vector in the absence of packaging expression vectors. Although the non-functional indicator genes contained within these resistance test vectors are not efficiently expressed upon transfection of the host cells, there is detectable indicator gene expression resulting from non-viral particle-based reverse transcription. Reverse transcription and strand transfer results in the conversion of the permuted, non-functional indicator gene to a non-permuted, functional indicator gene. As reverse transcription is completely dependent upon the expression of the *pol* gene contained within each resistance test vector, anti-viral agents may be tested for their ability to inhibit the *pol* gene products encoded by the patient-derived segments contained within the resistance test vectors. In the case of HIV, reverse transcription and strand transfer results in the conversion of the non-functional indicator gene to a functional indicator gene. As reverse transcription is completely dependent upon the expression of the patient-derived segment contained within each resistance test vector, anti-viral agents may be tested for their ability to inhibit the gene products encoded by the patient-derived segments contained within the resistance test vectors.

The packaging host cells are transfected with the resistance test vector and the appropriate packaging expression vector(s) to produce resistance test vector host cells. Individual anti-viral agents, including the reverse transcriptase inhibitors AZT, ddI, ddC, d4T and 3TC, and the protease inhibitors saquinavir, ritonavir and indinavir, as well as combinations thereof, are added to individual plates of packaging host cells at the time of their transfection, at an appropriate range of concentrations. Twenty-four to 48 hours after transfection, target host cells are infected by co-cultivation with resistance test vector host cells or with resistance test vector viral particles obtained from filtered supernatants of resistance test vector host cells. Each anti-viral agent, or combination thereof, is added to the target host cells prior to or at the time of infection to achieve the same final concentration of the given agent, or agents, present during the transfection.

Determination of the expression or inhibition of the indicator gene in the target host cells infected by co-cultivation or with filtered viral supernatants is made by assay of indicator gene expression, for example in the case where the indicator gene is the firefly *luc* gene, by measuring luciferase activity. The reduction in luciferase activity observed for target host cells infected with a given preparation of resistance test vector viral particles in the presence of a given antiviral agent, or agents, as compared to a control run in the absence of the antiviral agent, generally relates to the log of the concentration of the antiviral agent as a sigmoidal curve. This inhibition curve is used to calculate the apparent inhibitory concentration (IC) of that agent, or combination of agents, for the viral target product encoded by the patient-derived segments present in the resistance test vector.

In the case of a one cell susceptibility and resistance test, host cells are transfected with the resistance test vector and the appropriate packaging expression vector(s) to produce resistance test vector host cells. Individual antiviral agents, or combinations thereof, are added to individual plates of transfected cells at the time of their transfection, at an appropriate range of concentrations. Twenty-four to 72 hours after transfection, cells are collected and assayed for firefly luciferase activity. As transfected cells in the culture do not efficiently express the indicator gene, transfected cells in the culture, as well superinfected cells in the culture, can serve as target host cells for indicator gene expression. The reduction in luciferase activity observed for cells transfected in the presence of a given antiviral agent, or agents as compared to a control run in the absence of the antiviral agent(s), generally relates to the log of the concentration of the antiviral agent as a sigmoidal curve. This inhibition curve is used to calculate the apparent inhibitory concentration (IC) of an agent, or combination of agents, for the viral target product encoded by the patient-derived segments present in the resistance test vector.

### Anti-HIV Drugs/Drug Candidates

The anti-HIV drugs being added to the test system are added at selected times depending upon the target of the anti-HIV drug. For example, in the case of HIV protease inhibitors, including saquinavir, ritonavir, indinavir, and nelfinavir, they are added to individual plates of packaging host cells at the time of their transfection with a resistance test vector, at an appropriate range of concentrations. HIV protease inhibitors are also added to the target host cells at the time of infection to achieve the same final concentration added during transfections. HIV reverse transcriptase inhibitors, including AZT, ddI, ddC, d4T, 3TC and nevaripine, are added to individual plates of target host cells at the time of infection by the resistance test vector viral particles, at a test concentration. Alternatively, the antiviral drugs may be present throughout the assay. The test concentration is selected from a range of concentrations which is typically between about 0.1nM and about 100 (M and more specifically for each of the following drugs: AZT, from about 1nM to about 5(M; ddI, from about 1nM to about 25(M; 3TC, from about 1nM to about 50(M; d4T, from about 1nM to about 25(M; and, nevaripine, from about 1nM to about 100(M.

In another embodiment of this invention, a candidate antiviral compound is tested in the drug susceptibility and resistance test of this invention. The candidate antiviral compound is added to the test system at an appropriate concentration and at selected times depending upon the protein target of the candidate anti-viral. Alternatively, more than one candidate antiviral compound may be tested or a candidate antiviral compound may be tested in combination with an approved antiviral drug such as AZT, ddI, ddC, d4T, 3TC, saquinavir or a compound which is undergoing clinical trials such as ritonavir, or indinovir. The effectiveness of the candidate antiviral will be evaluated by measuring the expression or inhibition of the indicator gene. In another aspect of this embodiment, the drug susceptibility and resistance test may be used to screen for viral mutants. Following the identification of resistant mutants to either known anti-virals or candidate anti-virals the resistant mutants are isolated and the DNA is analyzed. A library of viral resistant mutants can thus be assembled enabling the screening of candidate anti-virals, alone or in combination. This will enable one of ordinary skill to identify effective anti-virals and design effective therapeutic regimens.

### General Materials and Methods

Most of the techniques used to construct vectors, and transfect and infect cells, are widely practiced in the art, and most practitioners are familiar with the standard resource materials which describe specific conditions and procedures. However, for convenience, the following paragraphs may serve as a guideline.

"Plasmids" and "vectors" are designated by a lower case p followed by letters and/or numbers. The starting plasmids herein are either commercially available, publicly available on an unrestricted basis, or can be constructed from available plasmids in accord with published procedures. In addition, equivalent plasmids to those described are known in the art and will be apparent to the ordinarily skilled artisan.

Construction of the vectors of the invention employs standard ligation and restriction techniques which are well understood in the art (see Ausubel et al., (1987) Current Protocols in Molecular Biology, Wiley - Interscience or Maniatis et al., (1992) in Molecular Cloning: A laboratory Manual, Cold Spring Harbor Laboratory, N.Y.). Isolated plasmids, DNA sequences, or synthesized oligonucleotides are cleaved, tailored, and relegated in the form desired. The sequences of all DNA constructs incorporating synthetic DNA were confirmed by DNA sequence analysis (Sanger et al. (1977) *Proc. Natl. Acad. Sci.* **74**, 5463-5467).

"Digestion" of DNA refers to catalytic cleavage of the DNA with a restriction enzyme that acts only at certain sequences, restriction sites, in the DNA. The various restriction enzymes used herein are commercially available and their reaction conditions, cofactors and other requirements are known to the ordinarily skilled artisan. For analytical purposes, typically 1(g of plasmid or DNA fragment is used with about 2 units of enzyme in about 20(l of buffer solution. Alternatively, an excess of restriction enzyme is used to insure complete digestion of the DNA substrate. Incubation times of about one hour to two hours at about 37°C are workable, although variations can be tolerated. After each incubation, protein is removed by extraction with phenol/chloroform, and may be followed by ether extraction, and the nucleic acid recovered from aqueous fractions by precipitation with ethanol. If desired, size separation of the cleaved fragments may be performed by polyacrylamide gel or agarose gel electrophoresis using standard techniques. A general description of size separations is found in Methods of Enzymology 65:499-560 (1980).

Restriction cleaved fragments may be blunt ended by treating with the large fragment of E. coli DNA polymerase I (Klenow) in the presence of the four deoxynucleotide triphosphates (dNTPs) using incubation times of about 15 to 25 minutes at 20°C in 50 mM Tris (ph7.6) 50 mM NaCl, 6 mM MgCl₂, 6 mM DTT and 5-10 mM dNTPs. The Klenow fragment fills in at 5' sticky ends but chews back protruding 3' single strands, even though the four dNTPs are present. If desired, selective repair can be performed by supplying only one of the dNTPs, or with selected dNTPs, within the limitations dictated by the nature of the sticky ends. After treatment with Klenow, the mixture is extracted with phenol/chloroform and ethanol precipitated. Treatment under appropriate conditions with S1 nuclease or Bal-31 results in hydrolysis of any single-stranded portion.

Ligations are performed in 15-50 (l volumes under the following standard conditions and temperatures: 20 mM Tris-Cl pH 7.5, 10 mM MgCl₂, 10 mM DTT, 33 mg/ml BSA, 10 mM-50 mM NaCl, and either 40 mM ATP, 0.01-0.02 (Weiss) units T4 DNA ligase at 0°C (for "sticky end" ligation) or 1mM ATP, 0.3 - 0.6 (Weiss) units T4 DNA ligase at 14°C (for "blunt end" ligation). Intermolecular "sticky end" ligations are usually performed at 33-100 (g/ml total DNA concentrations (5-100 mM total end concentration). Intermolecular blunt end ligations (usually employing a 10-30 fold molar excess of linkers) are performed at 1 mM total ends concentration.

"Transient expression" refers to unamplified expression within about one day to two weeks of transfection. The optimal time for transient expression of a particular desired heterologous protein may vary depending on several factors including, for example, any transacting factors which may be employed, translational control mechanisms and the host cell. Transient expression occurs when the particular plasmid that has been transfected functions, i.e., is transcribed and translated. During this time the plasmid DNA which has entered the cell is transferred to the nucleus. The DNA is in a nonintegrated state, free within the nucleus. Transcription of the plasmid taken up by the cell occurs during this period. Following transfection the plasmid DNA may become degraded or diluted by cell division. Random integration within the cell chromatin occurs.

In general, vectors containing promoters and control sequences which are derived from species compatible with the host cell are used with the particular host cell. Promoters suitable for use with prokaryotic hosts illustratively include the beta-lactamase and lactose promoter systems, alkaline phosphatase, the tryptophan (trp) promoter system and hybrid promoters such as tac promoter. However, other functional bacterial promoters are suitable. In addition to prokaryotes, eukaryotic microbes such as yeast cultures may also be used. *Saccharomyces cerevisiae,* or common baker's yeast is the most commonly used eukaryotic microorganism, although a number of other strains are commonly available. Promoters controlling transcription from vectors in mammalian host cells may be obtained from various sources, for example, the genomes of viruses such as: polyoma, simian virus 40 (SV40), adenovirus, retroviruses, hepatitis B virus and preferably cytomegalovirus, or from heterologous mammalian promoters, e.g. b-actin promoter. The early and late promoters of the SV 40 virus are conveniently obtained as an SV40 restriction fragment which also contains the SV40 viral origin of replication. The immediate early promoter of the human cytomegalovirus is conveniently obtained as a HindIII E restriction fragment. Of course, promoters from the host cell or related species also are useful herein.

The vectors used herein may contain a selection gene, also termed a selectable marker. A selection gene encodes a protein, necessary for the survival or growth of a host cell transformed with the vector. Examples of suitable selectable markers for mammalian cells include the dihydrofolate reductase gene (DHFR), the ornithine decarboxylase gene, the multi-drug resistance gene (mdr), the adenosine deaminase gene, and the glutamine synthase gene. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. The second category is referred to as dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin (Southern and Berg (1982) *J. Molec. Appl. Genet.* **1**, 327), mycophenolic acid (Mulligan and Berg (1980) *Science* **209,** 1422), or hygromycin (Sugden et al. (1985) *Mol. Cell. Biol.* **5**, 410-413). The three examples given above employ bacterial genes under eukaryotic control to convey resistance to the appropriate drug neomycin (G418 or genticin), xgpt (mycophenolic acid) or hygromycin, respectively.

"Transfection" means introducing DNA into a host cell so that the DNA is expressed, whether functionally expressed or otherwise; the DNA may also replicate either as an extrachromosomal element or by chromosomal integration. Unless otherwise provided, the method used herein for transformation of the host cells is the calcium phosphate co-precipitation method of Graham and van der Eb (1973) *Virology* **52**, 456-457. Alternative methods for transfection are electroporation, the DEAE-dextran method, lipofection and biolistics (Kriegler (1990) Gene Transfer and Expression: A Laboratory Manual, Stockton Press).

Host cells may be transfected with the expression vectors of the present invention and cultured in conventional nutrient media modified as is appropriate for inducing promoters, selecting transformants or amplifying genes. Host cells are cultured in F12:DMEM (Gibco) 50:50 with added glutamine and without antibiotics. The culture conditions, such as temperature, pH and the like, are those previously used with the host cell selected for expression, and will be apparent to the ordinarily skilled artisan.

The following examples merely illustrate the best mode now known for practicing the invention, but should not be construed to limit the invention. All literature references are expressly incorporated by reference.

### EXAMPLE 1

### HIV Drug Susceptibility And Resistance Test Using Resistance Test Vectors Comprising Patient-derived Segment(s) And A Non-Functional Indicator Gene With A Permuted Promoter.

### Indicator Gene Viral Vector - Construction

Indicator gene viral vectors containing a non-functional indicator gene with a permuted promoter were designed using both HIV genomic and subgenomic viral vectors comprising viral genes which are the target(s) of anti-viral drugs. The indicator gene viral vectors pLG-lucPP and pCG-lucPP are based on the genomic viral vectors pLG and pCG; each bears a deletion in the HIV *env* gene. Resistance test vectors derived from the genomic indicator gene viral vectors, pLG-lucPP and pCG-lucPP, contain a patient sequence acceptor site for insertion of the patient-derived segment and are used in conjunction with a packaging expression vector encoding the amphotrophic MLV 4070A env gene product. The indicator gene viral vectors pLS-lucPP and pCS-lucPP are based on the subgenomic viral vectors pLS and pCS; each encodes the HIV *gag-pol* gene only. Resistance test vectors derived from the subgenomic indicator gene viral vectors, pLS-lucPP and pCS-lucPP, contain a sequence acceptor site for insertion of patient-derived segment and are used in conjunction with a first packaging expression vector encoding the HIV *vif, vpr, tat, rev, vpu* and *nef* genes and a second packaging vector encoding the amphotrophic MLV 4070A *env* gene product.

### HIV Viral Vectors - Genomic and Subgenomic

HIV viral vectors were designed using the sequences of the biologically active proviral clone, HXB2 (Fisher et al. (1986) *Nature* **320,** 367-371). Two types of viral vector were designed: genomic viral vectors with deletions in a single gene such as *env*, but which otherwise preserve the mRNA expression and processing characteristics of the complete virus, and subgenomic viral vectors which may include only one or a few genes that are typically the specific targets of susceptibility and resistance testing, such as *gag-pol*, or which may lack viral genes altogether. Both types of vectors have a unique restriction site within the viral genome for the insertion of an indicator gene cassette as well as patient sequence acceptor sites, i.e. additional unique restriction sites near or within the anti-viral target gene (eg., *pol*) to permit the insertion of patient-derived HIV sequences. In addition, both types of vector were designed to incorporate either the native enhancer-promoter of the HIV-LTR U3 region, or a foreign enhancer-promoter from the CMV immediate-early (IE) region. Standard methods are employed for the construction of plasmid DNAs (Ausubel et al. (1987) Current Protocols in Molecular Biology, Wiley-Interscience). The sequences of all DNA constructs incorporating synthetic DNA are confirmed by DNA sequence analysis (Sanger et al. (1977) *Proc. Natl. Acad. Sci.* **74**, 5463-5467).

HIV sequences are obtained from plasmid pBS-HIV (Page et al. (1990) *J. Virol*. **64**, 5270-5276) which contains the HXB2 proviral DNA sequence on an HpaI to XbaI restriction fragment inserted into the polylinker of the pBluescript KS (+) plasmid cloning vector (Stratagene, San Diego, CA). As this proviral clone contains uncharacterized, flanking human DNA from the site of proviral integration, two steps of site-directed mutagenesis are employed to remove such sequences using plasmid pBS-HIV as a template. In step one, human sequences adjacent to the 5' LTR are removed using oligonucleotide 1 which contains the following sequences in a 5' to 3' direction: 1) a sequence complementary to the first 18 nucleotides of the integrated provirus, within the left U3 region, 2) a six nucleotide.SmaI site, and 3) an 18 nucleotide sequence complementary to the region in pBluescript KS (+) just beyond the polylinker and phage T3 promoter. In step two, human sequences adjacent to the 5' LTR are removed using oligonucleotide 2 which contains the following sequences (5' to 3'): 1) an 18 nucleotide sequence complementary to the region in pBluescript KS (+) just beyond the PvuI site, within the LacZ gene, 2) a six nucleotide XbaI site, and 3) a sequence complementary to the last 18 nucleotides of the integrated provirus, within the right U5 region. The resulting plasmid is called pBS-HXB2.

Genomic and subgenomic viral vectors employing the HIV-LTR U3 region as an enhancer-promoter for the expression of anti-viral target genes (Fig. 1) are each derived from plasmid pBS-HXB2 by a single step of site-directed mutagenesis. The genomic viral vector pLG, which is deleted for the *env* gene, is prepared using oligonucleotide 3 which contains the following sequences (5' to 3'): 1) an 18 nucleotide sequence complementary to positions 7626 to 7643 of HXB2 within the *env* gene (all coordinates for HXB2 are by reference to GenBank, accession number K03455), 2) an 8 nucleotide NotI site, and 3) an 18 nucleotide sequence complementary to positions 6384 to 6401 of HXB2 within the *env* gene. The subgenomic viral vector pLS, which is deleted for the *env, tat, rev, vif, vpr* and *vpu* genes, is prepared using oligonucleotide 4 which contains the following sequences (5' to 3'): 1) an 18 nucleotide sequence complementary to positions 7626 to 7643 of HXB2 within the *env* gene, 2) an 8 nucleotide NotI site, and 3) an 18 nucleotide sequence complementary to positions 5109 to 5126 of HXB2 within the *vif* gene.

Genomic and subgenomic viral vectors which employ the CMV IE region as an enhancer-promoter for the expression of anti-viral target genes are derived from plasmids pLG and pLS, and are called pCG and pCS, respectively (Fig. 1). The genomic viral vector pCG is prepared in two steps. In the first step, an intermediate plasmid is prepared from two DNA fragments: 1) a vector fragment 11.2 kB prepared from digesting plasmid pLG with SmaI and treating the vector with alkaline phosphatase, and 2) a DNA fragment of 0.9 kB containing the CMV IE enhancer-promoter prepared by digesting plasmid pVL-1 (described below) with SmaI. Plasmids containing the CMV IE region in the same transcriptional orientation as the viral LTRs are identified by restriction mapping. In the second step, plasmid pCG is prepared from this intermediate plasmid by site-directed mutagenesis to join the CMV IE enhancer-promoter to the 5'-LTR R region at a position permitting transcription initiation to occur at the beginning of the R region, using oligonucleotide 5 which contains the following sequences (5' to 3'): 1) an 18 nucleotide sequence complementary to positions 455 to 472 of HXB2 at the beginning of the R region, and 2) an 18 nucleotide sequence complementary to positions -18 to -1 of the CMV IE enhancer-promoter (coordinates referenced to Boshart et al. (1985) *Cell* **41**, 521-530). The subgenomic viral vector pCS is derived from plasmid pCG and is prepared from two DNA fragments: 1) a vector DNA of 9.1 kB prepared by digesting plasmid pLS with SmaI and ClaI, and 2) a DNA fragment of 1.3 kB prepared by digesting plasmid pCG with SmaI and ClaI.

### Genomic Indicator Gene Viral vector- Permuted Promoter

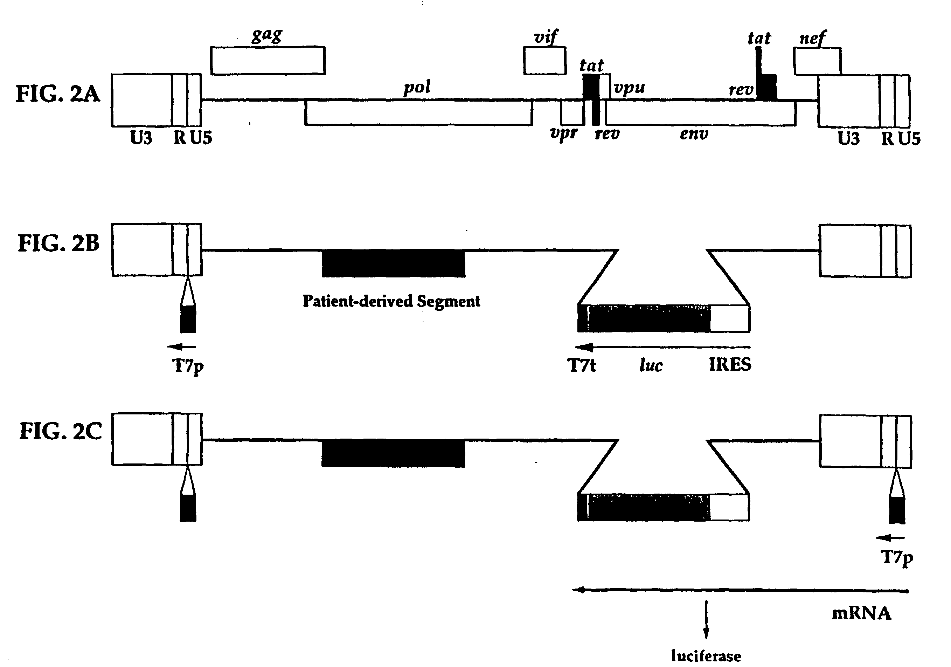

The indicator gene viral vectors pLG-lucPP and pCG-lucPP, and resistance test vectors derived therefrom, contain the following elements in a 5' to 3' orientation (Fig 2B): 1) an HIV-LTR U3 region (pLG-lucPP) or a CMV IE enhancer-promoter (pCG-lucPP), 2) an HIV-LTR R region, 3) an HIV-LTR U5 region containing an inserted T7 promoter with a transcriptional orientation opposite to that of the LTRs, 4) the coding regions of the HIV *gag-pol, vif, vpr, tat, rev, vpu,* deleted *env*, and *nef* genes, 5) an indicator gene cassette inserted into the deleted *env* gene, and 6) a 3' HIV-LTR. The same indicator gene cassette is inserted in pLG-lucPP and pCG-lucPP and contains the following elements: 1) an EMC 5'-UTR region which permits internal ribosome entry, 2) the complete coding region of the luciferase (luc) gene, and 3) a T7 transcriptional terminator. The indicator gene has a transcriptional orientation opposite to the HIV-LTR or CMV IE enhancer-promoter and therefore cannot be functionally transcribed by these elements. The indicator gene also cannot be transcribed by the T7 promoter as the indicator gene cassette is positioned upstream of the T7 promoter. Following reverse transcription and strand transfer, the T7 promoter is copied from the 5' LTR to the 3' LTR, permitting functional transcription of the indicator gene from the newly created T7 promoter by T7 RNA polymerase (Fig 2C).

Plasmid plucPP, which contains the indicator gene cassette, is prepared in three steps. In the first step, plasmid pVL-EMC/T7 which contains a cassette flanked by unique NotI sites comprising the EMC 5'-UTR element and T7 transcriptional terminator, is prepared from two DNA fragments: 1) a vector DNA of 3.0 kB prepared by digesting plasmid pVL (described below) with NotI and treating the vector with alkaline phosphatase, and 2) a DNA fragment of 0.8 kB containing the EMC 5' UTR and T7 terminator prepared by PCR using plasmid pTM1 (Moss et al. (1990) *Nature* **348,** 91-92) as a template and oligonucleotides 6 and 7 as primers, followed by digestion with NotI. Oligonucleotides 6 and 7 each incorporate a NotI restriction site. In the second step, plasmid pVL-luc, which contains the coding region of the firefly luciferase gene inserted into the mammalian expression vector pVL-1 (described below), is prepared from two DNA fragments: a vector DNA of 4.1 kB prepared by digesting plasmid pVL-1 with NruI and BglII, and 2) a DNA fragment of 1.7 kB containing the complete luciferase coding region, prepared by PCR using plasmid pGEM-luc (Promega, Madison, WI) as a template and oligonucleotides 8 and 9 as primers followed by digestion with NruI and BglII. Oligonucleotides 8 and 9 incorporate NruI and NcoI, and BglII and XhoI restriction sites, respectively. In the third step, plasmid plucPP, which contains the coding region of the luciferase gene inserted between the EMC 5'-UTR element and T7 transcriptional terminator, is prepared from two DNA fragments: 1) a vector DNA of 3.8 kB prepared by digesting plasmid pVL-EMC/T7 with NcoI and SalI, and 2) a DNA fragment of 1.7 kB containing the complete luciferase coding region, prepared by digesting plasmid pVL-luc with NcoI and XhoI.

Plasmid pLG-lucPP is prepared in two steps. In the first step, plasmid pLG-T7 is prepared by inserting a phage T7 promoter into the upstream HIV-LTR U5 region in plasmid pLG by site-directed mutagenesis using oligonucleotide 10 which contains the following sequences (5' to 3'): 1) an 18 nucleotide sequence complementary to positions 552 to 569 of HXB2 within the U5 region, 2) a 20 nucleotide sequence complementary to the T7 promoter, and 3) an 18 nucleotide sequence complementary to positions 534 to 551 of HXB2 within the U5 region. In the second step, plasmid pLG-lucPP is prepared from two DNA fragments: 1) a vector DNA of 11.2 kB prepared by digesting plasmid pLG-T7 with NotI and treating the resulting vector with alkaline phosphatase, and 2) a DNA fragment of 2.5 kB containing the luciferase indicator gene cassette prepared by digesting plasmid plucPP with NotI. Clones corresponding to pLG-lucPP, which contain the indicator gene cassette inserted into the viral vector with a transcriptional orientation.opposite to that of the viral LTRs, are identified by restriction mapping.

Plasmid pCG-lucPP is prepared in two steps. In the first step, plasmid pCG-T7 is prepared by inserting a phage T7 promoter into the upstream HIV-LTR U5 region in plasmid pCG by site-directed mutagenesis using oligonucleotide 10. In the second step, plasmid pCG-lucPP is prepared from two DNA fragments: 1) a vector DNA of 11.7 kB prepared by digesting plasmid pCG-T7 with NotI and treating the resulting vector with alkaline phosphatase, and 2) a DNA fragment of 2.5 kB containing the luciferase indicator gene cassette prepared by digesting plasmid plucPP with NotI. Clones corresponding to pCG-lucPP, which contain the indicator gene cassette inserted into the viral vector with a transcriptional orientation opposite to that of the CMV IE enhancer-promoter and viral LTRs, are identified by restriction mapping.

### Subgenomic Indicator Gene Viral Vector - Permuted Promoter

The indicator gene viral vectors pLS-lucPP and pCS-lucPP, and resistance test vectors derived therefrom, contain the following elements in a 5' to 3' orientation (Fig 2B): 1) an HIV-LTR U3 region (pLS-lucPP) or a CMV IE enhancer-promoter (pCS-lucPP), 2) an HIV-LTR R region, 3) an HIV-LTR U5 region containing an inserted T7 promoter with a transcriptional orientation opposite to that of the LTRs, 4) the coding region of the HIV *gag-pol* gene, 5) the indicator gene cassette, 6) an RRE element from the HIV *env* gene containing a viral packaging sequence, and 7) a 3' HIV- LTR. The indicator gene cassette of pLS-lucPP and pCS-lucPP is the same as in pLG-lucPP and pCG-lucPP. As for the latter vectors, the indicator genes of pLS-lucPP and pCS-lucPP cannot be functionally transcribed until reverse transcription and strand transfer results in the copying of the T7 promoter from the 5' LTR to the 3' LTR (Fig 2C). Plasmid pLS-lucPP is prepared in two steps. In the first step, plasmid pLS-T7, which contains a phage T7 promoter inserted into the upstream HIV-LTR U5 region of plasmid pLS, is prepared from two DNA fragments: 1) a vector DNA of 9.1 kB prepared by digesting plasmid pLS with SmaI and ClaI, and 2) a DNA fragment of 0.8 containing the HIV-LTR with an R5 region containing an inserted T7 promoter, prepared by digesting plasmid pLG-T7 with SmaI and ClaI. In the second step, plasmid pLS-lucPP is prepared from two DNA fragments: 1) a vector DNA of 9.9 kB prepared by digesting plasmid pLS-T7 with NotI and treating the resulting vector with alkaline phosphatase, and 2) a DNA fragment of 2.5 kB containing the luciferase indicator gene cassette prepared by digesting plasmid plucPP with NotI. Clones corresponding to pLS-lucPP, which contain the indicator gene cassette inserted into the viral vector with a transcriptional orientation opposite to that of the viral LTRs, are identified by restriction mapping.

Plasmid pCS-lucPP is prepared from two DNA fragments: 1) a vector DNA of 11.6 kB prepared by digesting plasmid pLS-lucPP with SmaI and ClaI, and 2) a DNA fragment of 1.3 containing the CMV IE promoter fused to the R5 region with an inserted T7 promoter, prepared by digesting plasmid pCG-T7 with SmaI and ClaI.

### Resistance Test Vectors - Construction

Resistance test vectors are prepared by 1) modifying the indicator gene viral vectors pLG-lucPP, pCG-lucPP, pLS-lucPP and pCS-lucPP by introducing unique restriction sites, called patient sequence acceptor sites, in or near the *pol* gene, 2) amplifying patient-derived segments corresponding to the HIV protease and reverse transcriptase coding regions by PCR using complementary DNA (cDNA) prepared from viral RNA or DNA present in the serum or cells of infected patients, and 3) inserting the amplified sequences precisely into indicator gene viral vectors at patient sequence acceptor sites (Fig. 2B). Two sets of patient sequence acceptor sites are introduced by site-directed mutagenesis into each of the four indicator gene viral vectors. The first set of patient sequence acceptor sites consist of a HpaI site and a Sail site which define an interval comprising the entire protease coding region and most of the reverse transcriptase coding region, resulting in plasmids pLG-lucPP-HS, pCG-lucPP-HS, pLS-lucPP-HS and pCS-lucPP-HS. The second set of patient sequence acceptor sites consist of a PvuI site and a BamHI site which define the same interval, resulting in plasmids pLG-lucPP-PB, pCG-lucPP-PB, pLS-lucPP-PB and pCS-lucPP-PB, respectively. Cognate pairs of resistance test vectors which define the same restriction site interval (eg., those derived from pLG-lucPP-HS and pLG-lucPP-PB) are used together in some resistance tests to improve the representation of those patient-derived segments that contain internal restriction sites identical to a given patient sequence acceptor site, and would thus be underrepresented in either resistance test vector alone.

Plasmid pLG-lucPP-HS is prepared by three consecutive steps of site-directed mutagenesis using plasmid pLG-lucPP as a template. The first two steps are for the purpose of introducing two new restriction sites, one of which (HpaI) is unique to, and one of which (SalI) is already is present once in each indicator gene viral vector. The third step is for the purpose of deleting the pre-existing SalI site in each vector to make the introduced SalI site unique. In step 1, a HpaI site is introduced immediately upstream of the mature coding region of the HIV protease at position 2243 using oligonucleotide 11 which contains the following sequences (5' to 3'): 1) an 18 nucleotide sequence complementary to positions 2249 to 2266 of HXB2, 2) a six nucleotide HpaI site, which leaves the gag protein sequence at this position unaltered and introduces a conservative amino acid change (Phe to Val) into the *pol* precursor sequence, and 3) an 18 nucleotide sequence complementary to positions 2225 to 2242 of HXB2. In step 2, a SalI site is introduced at the carboxy-terminal coding region of the HIV reverse transcriptase at position 4190 using oligonucleotide 12 which contains the following sequences (5' to 3'): 1) an 18 nucleotide sequence complementary to positions 4196 to 4213 of HXB2, 2) a six nucleotide SalI site which leaves the reverse transcriptase protein sequence at this position unaltered, and 3) an 18 nucleotide sequence complementary to positions 4172 to 4189 of HXB2. In step 3, the pre-existing SalI site within the *vpr* coding region at position 5785 of HXB2 is deleted using oligonucleotide 13 which contains the following sequences (5' to 3'): 1) an 18 nucleotide sequence complementary to positions 5791 to 5808 of HXB2, 2) the 6 nucleotide sequence GCCGAC which ablates the Sall site but leaves the *vpr* protein sequence at this position unaltered, 3) an 18 nucleotide sequence complementary to positions 5767 to 5784 of HXB2.

Plasmids pCG-lucPP-HS, pLS-lucPP-HS and pCS-lucPP-HS are derived from pLG-lucPP-HS as follows. Plasmid pCG-lucPP-HS is prepared from two DNA fragments: 1) a vector DNA of 12.9 kB prepared by digesting plasmid pLG-lucPP-HS with SmaI and ClaI, and 2) a DNA fragment of 1.3 kB prepared by digesting plasmid pCG-lucPP with SmaI and ClaI. Plasmid pLS-lucPP-HS is prepared from two DNA fragments: 1) a vector DNA of 11.1 kB prepared by digesting plasmid pLG-lucPP-HS with NdeI and XhoI, and 2) a DNA fragment of 1.3 kB prepared by digesting plasmid pLS-lucPP with NdeI and XhoI. Plasmid pCS-lucPP-HS is prepared from two DNA fragments: 1) a vector DNA of 11.6 kB prepared by digesting plasmid pLS-lucPP-HS with SmaI and ClaI, and 2) a DNA fragment of 1.3 kB prepared by digesting plasmid pCS-lucPP with SmaI and ClaI.

Plasmid pLG-lucPP-PB is prepared by four consecutive steps of site-directed mutagenesis using plasmid pLG-lucPP as a template. The first two steps are for the purpose of introducing two new restriction sites (PvuI and BamHI), each of which is already present once in each indicator gene viral vector. The third and fourth steps are for the purpose of deleting the pre-existing PvuI and BamHI sites in each vector to make the newly introduced sites unique. In step 1, a PvuI site is introduced immediately upstream of the mature coding region of the HIV protease at position 2221 using oligonucleotide 14 which contains the following sequences (5' to 3'): 1) an 18 nucleotide sequence complementary to positions 2227 to 2244 of HXB2, 2) a six nucleotide PvuI site, which leaves the *gag* and *pol* precursor protein sequences at this position unaltered, and 3) an 18 nucleotide sequence complementary to positions 2203 to 2220 of HXB2. In step 2, a BamHI site is introduced at the carboxy-terminal coding region of the HIV reverse transcriptase at position 4212 using oligonucleotide 15 which contains the following sequences (5' to 3'): 1) an 18 nucleotide sequence complementary to positions 4218 to 4235 of HXB2, 2) a six nucleotide BamHI site which leaves the reverse transcriptase protein sequence at this position unaltered, and 3) an 18 nucleotide sequence complementary to positions 4194 to 4211 of HXB2. In step 3, the pre-existing PvuI site within the b-lactamase coding region at position 2413 (coordinates references to GenBank, accession number X52331) is deleted using oligonucleotide 16 which contains the following sequences (5' to 3'): 1) an 18 nucleotide sequence complementary to positions 2395 to 2412 of pBluescript KS (+) , 2) the 6 nucleotide sequence CAATCG which ablates the PvuI site but leaves the b-lactamase protein sequence at this position unaltered, and 3) an 18 nucleotide sequence complementary to positions 2419 to 2436 of pBluescript KS (+). In step 4, the pre-existing BamHI site within the HIV *rev* coding region at position 8474 of HXB2 is deleted using oligonucleotide 17 which contains the following sequences (5' to 3'): 1) an 18 nucleotide sequence complementary to positions 8480 to 8497 of HXB2, 2) the 6 nucleotide sequence GGATTC which ablates the BamHI site but leaves the HIV *rev* protein sequence at this position unaltered, and 3) an 18 nucleotide sequence complementary to positions 8456 to 8473 of HXB2.

Plasmids pCG-lucPP-PB, pLS-lucPP-PB and pCS-lucPP-PB are derived from pLG-lucPP-PB as follows. Plasmid pCG-lucPP-PB is prepared from two DNA fragments: 1) a vector DNA of 12.9 kB prepared by digesting plasmid pLG-lucPP-PB with SmaI and ClaI, and 2) a DNA fragment of 1.3 kB prepared by digesting plasmid pCG-lucPP with SmaI and ClaI. Plasmid pLS-lucPP-PB is prepared from three DNA fragments: 1) a vector DNA of 11.1 kB prepared by digesting plasmid pLG-lucPP-PB with NdeI and XhoI, 2) a DNA fragment of 0.5 kB prepared by digesting plasmid pLS-lucPP with NdeI and HindIII and 3) a DNA fragment of 0.8 kB prepared by digesting plasmid pLG-lucPP-PB with HindIII and XhoI. Plasmid pCS-lucPP-PB is prepared from two DNA fragments: 1) a vector DNA of 11.6 kB prepared by digesting plasmid pLS-lucPP-PB with SmaI and ClaI, and 2) a DNA fragment of 1.3 kB prepared by digesting plasmid pCS-lucPP with SmaI and ClaI.

Patient-derived segment (s) corresponding to the HIV protease and reverse transcriptase coding regions are amplified by the reverse transcription-polymerase chain reaction method (RT-PCR), using viral RNA isolated from the serum of HIV-infected patients. Two RT-PCR protocols are used as described. In the first method (Piatak et al. (1993) *Science* **259**, 1749-1754), separate enzymes, Moloney murine leukemia virus reverse transcriptase (BRL, Bethesda, MD) and *Taq* DNA polymerase (Roche Molecular Diagnostics, Ontario, Canada), are used for the preparation of cDNA and for the PCR reaction, respectively. In the second method (Mulder et al. (1994) J. Clin. Microbiol. **32**, 292-300), a single enzyme, *Thermus thermophilus* (*Tth*) DNA polymerase, is used to carry both cDNA synthesis and the PCR reaction. Two primer pairs, consisting of oligonucleotides 18 and 19, and oligonucleotides 20 and 21, are employed for the amplification of patient-derived segments that can be inserted precisely into the indicator gene viral vectors containing the HpaI/SalI and PvuI/BamHI patient acceptor sites, respectively.

A first set of four resistance test vectors incorporating the first primer pair is constructed from the following two DNA preparations: 1) a vector DNA prepared from plasmid pLG-lucPP-HS, pCG-lucPP-HS, pLS-lucPP-HS or pCS-lucPP-HS, digested with HpaI and SalI, and 2) an amplified DNA product of 2.0 kB prepared by RT-PCR using viral RNA isolated from the serum of an HIV-infected individual as a template and oligonucleotides 18 and 19 as primers, followed by digestion with HpaI and SalI. A second set of four resistance test M vectors incorporating the second primer pair are constructed from the following two DNA preparations: 1) a vector DNA prepared from plasmid pLG-lucPP-PB, pCG-lucPP-PB, pLS-lucPP-PB or pCS-lucPP-PB, digested with PvuI and BamHI, and 2) an amplified DNA product of 2.0 kB prepared by RT-PCR using viral RNA isolated from the serum of an HIV-infected individual as a template and oligonucleotides 18 and 19 as primers, followed by digestion with PvuI and BamHI. Oligonucleotides 18, 19, 20 and 21 incorporate HpaI, SalI, PvuI and BamHI restriction sites, respectively. To ensure that the plasmid DNA corresponding to each of the eight resulting resistance test vectors comprises a representative sample of the HIV viral quasi-species present in the serum of a given patient, at least one hundred independent E. coli transformants obtained in the construction of a given resistance test vector are used for the preparation of plasmid DNA.

To improve the representation of patient-derived segments, a third and fourth set of resistance test vectors are prepared using partially degenerate PCR primer pools, called oligonucleotides 22, 23, 24 and 25, which are based on the sequences of oligonucleotides 18, 19, 20 and 21, respectively. Each primer pool is synthesized in a manner that incorporates more than one nucleotide base (G, A, T or C) at the each of the 18 nucleotide positions located at the 3' end of the parent primer that display sequence variations among the different patient isolates cataloged in the Los Alamos HIV sequence database (Myers et al. (1993) Human Retroviruses and AIDS 1993, Los Alamos National Laboratory, Los Alamos, NM). The third set of four resistance test vectors is constructed using vectors prepared from plasmid pLG-lucPP-HS, pCG-lucPP-HS, pLS-lucPP-HS or pCS-lucPP-HS, with amplified patient sequences prepared with oligonucleotides 22 and 23; the fourth set of four resistance test vectors is constructed using vectors prepared from plasmid pLG-lucPP-PB, pCG-lucPP-PB, pLS-lucPP-PB or pCS-lucPP-PB, with amplified patient sequences prepared with oligonucleotides 24 and 25. Oligonucleotides 22, 23, 24 and 25 incorporate HpaI, SalI, PvuI and BamHI restriction sites, respectively.

### Host Cells - Preparation

### Packaging Host Cells and Resistance Test Vector Host Cells

Resistance test vectors are used to prepare resistance test vector host cells from packaging host cells expressing viral packaging proteins. The packaging proteins may be provided for by the expression of viral genes contained within the resistance test vector itself, a packaging expression vector(s), or both. Either transient or stable transfection of the packaging host cell may be employed to produce the packaging proteins. A packaging expression vector encoding an amphotrophic MLV *env* gene product enables production in a resistance test vector host cell of resistance test vector viral particles which can efficiently infect human target cells. Resistance test vectors derived from plasmids pLG-lucPP-HS, pLG-lucPP-PB, pCG-lucPP-HS and pCG-lucPP-PB encode all HIV genes with the exception of *env,* and are used to produce resistance test vector host cells. The pVL-env4070A packaging expression vector which encodes the amphotrophic MLV 4070A *env* gene product is used with the foregoing genomic-based resistance test vectors to enable production in the resistance test vector host cell of resistance test vector viral particles. Resistance test vectors derived from plasmids pLS-lucPP-HS, pLS-lucPP-PB, pCS-lucPP-HS and pCS-lucPP-PB encode the HIV *gag-pol* gene products only, and are used to prepare resistance test vector host cells. The pVL-env4070A which provides *env*, and either the pLTR-HIV3' or the pCMV-HIV3' packaging expression vectors, each of which provides the HIV *vif, vpr, tat, rev, vpu* and *nef* genes are used with the foregoing subgenomic based resistance test vectors to enable production in the resistance test vector host cells of resistance test vector viral particles.

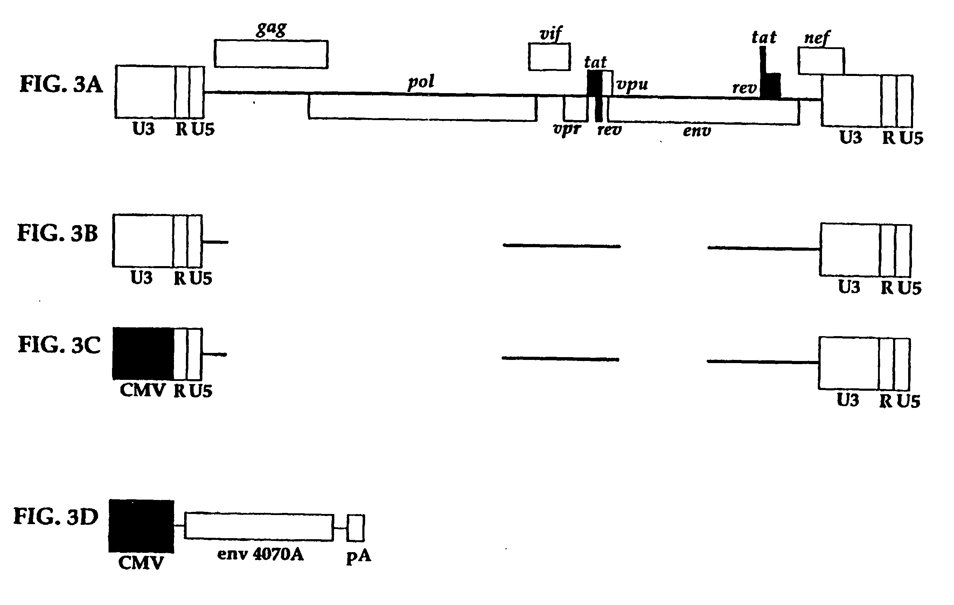

Plasmids pLTR-HIV3' and pCMV-HIV3' are each derived by removing most of the *gag-pol* coding region from the genomic viral vectors pLG and pCG, respectively. Plasmid pLTR-HIV3' (Fig. 3B) is prepared by site-directed mutagenesis using plasmid pLG as a template with oligonucleotide 26 which contains the following sequences (5' to 3'): 1) an 18 nucleotide sequence complementary to positions 4712 to 4729 of HXB2 within the *pol* gene, and 2) an 18 nucleotide sequence complementary to positions 925 to 942 of HXB2 within the *gag* gene. Plasmid pCMV-HIV3' (Fig. 3C) is prepared from two DNA fragments: 1) a vector fragment of 6.8 kB prepared by digesting plasmid pLTR-HIV3' with SmaI and ClaI, and 2) a DNA fragment of 1.3 kB prepared by digesting plasmid pCG with SmaI and ClaI.

Plasmid pVL-env4070A (Fig. 3D) is constructed from two DNA fragments: 1) a vector fragment of 4.3 kB prepared by digestion of the pVL-2 mammalian expression vector with NruI and BglII, and 2) a DNA fragment of 2.0 kB containing the complete coding region of the MLV4070A *env* gene product (nucleotides 37 to 2001, coordinates given in GenBank, accession number M33469, Ott et al. (1990) *J. Virol.* **64**, 757-766) prepared by PCR using plasmid pCRIPam*gag*-2 (Danos and Mulligan (1988) *Proc. Natl. Acad. Sci*. **85,** 6460) as a template with oligonucleotides 27 and 28 as primers, followed by digestion with NruI and BglII. Oligonucleotide 27 incorporates a unique NruI site followed by a consensus sequence for mammalian translation initiation (e.g., Kozak (1991) *J. Biol. Chem.* **266**, 19867-19870), while oligonucleotide 28 incorporates a unique BglII site.

The mammalian expression vector pVL-2 contains the following elements in a 5' to 3' direction: the CMV IE promoter/enhancer, the CMV IE first exon splice donor, the human (1 globin second exon splice acceptor, a cloning site polylinker, the polyadenylation site of the SV40 T antigen gene, and the SV40 origin of replication. Plasmid pVL-2 is constructed in four steps as follows. In the first step, plasmid pVL is prepared by replacing the cloning site polylinker and phage T7 and T3 promoters of plasmid pBluescript II KS (+) with a cloning site polylinker containing BssHII, NotI, SmaI, HindIII, SphI, SmaI, EcoRI, NruI, ApaI, BglII, NheI, NotI, XhoI, and BssHII restriction sites. pVL is constructed from two DNA fragments: 1) a vector fragment of 3.0 kB prepared by cutting plasmid pBluescript II KS (+) with BssHII, and treating the resulting vector with alkaline phosphatase, and 2) a DNA fragment prepared by annealing overlapping oligonucleotides 29 and 30, extending with Klenow DNA polymerase and digesting with BssHII. Plasmids containing the HindIII to XhoI sites in a 5' to 3' order relative to the pBluescript II KS (+) plasmid map (GenBank accession number X52327) are identified by restriction mapping analysis. In the second step, an intermediate plasmid is prepared from plasmid pVL by inserting the CMV IE enhancer-promoter and first exon splice donor, and the human (1 globin second exon splice acceptor. This intermediate plasmid is prepared from three DNA fragments: 1) a vector fragment of 3.0 kB prepared by digesting plasmid pVL with HindIII and EcoRI, 2) a DNA fragment of 0.9 kB containing a CMV IE promoter-enhancer and first exon splice donor (nucleotides -674 to -19, coordinates referenced to Boshart et al. (1985) *Cell* **41,** 521-530), prepared by PCR using the plasmid pCM5027 containing the PstI m-fragment from HCMV strain AD169 (Boshart et al., Ibid) as template with oligonucleotides 31 and 32 as primers, followed by digestion with HindIII and SphI, and 3) a DNA fragment of 0.1 kB containing the human (1 globin second exon splice acceptor (nucleotides 6808 to 6916, coordinates by reference to GenBank, accession number J00153) prepared by PCR using plasmid ppSVaHP (Treisman et al. (1983) *Proc. Natl. Acad. Sci*. **80**, 7428-7432) as a template with oligonucleotides 33 and 34 as primers, followed by digestion with SphI and EcoRI. Oligonucleotides 31, 32, 33 and 34 incorporate HindIII, SphI, SphI and EcoRI restriction sites at their respective ends. In the third step, plasmid pVL-1 is prepared by inserting the SV40 T antigen polyadenylation site into this intermediate plasmid. Plasmid pVL-1 is prepared from two DNA fragments: 1) a vector fragment of 4.0 kB prepared by cutting the intermediate plasmid with BglII and NheI, and 2) a DNA fragment of 0.2 kB containing the SV40 T antigen polyadenylation site (nucleotides 2770 to 2533 of SV40, coordinates by reference to Reddy et al. (1978) *Science* **200**, 494-502) prepared by PCR using plasmid pSV2 (Southern and Berg (1982) *J. Mol. Appl. Gen*. **1**, 327-341) as template with oligonucleotides 35 and 36 as primers, followed by digestion with BglII and NheI. Oligonucleotides 35 and 36 incorporate unique BglII and NheI restriction sites at their respective ends. In the fourth step, plasmid pVL-2 is prepared by inserting the SV40 origin of replication into plasmid pVL-1. Plasmid pVL-2 is prepared from two DNA fragments: 1) a vector fragment of 4.2 kB prepared by digesting plasmid pVL-1 with NheI and XhoI, and 2) a DNA fragment of 0.2 kB containing the SV40 origin of replication (nucleotides 5725 to 5578 of SV40, Ibid) prepared by PCR using plasmid pSV2 as template with oligonucleotides 37 and 38 as primers, followed by digestion with NheI and SalI. Oligonucleotides 37 and 38 incorporate unique NheI and Sall restriction sites at their respective ends.

### Target Host Cells

Target host cells used for resistance tests carried out with resistance test vectors derived from plasmids pLG-lucPP-HS, pLG-lucPP-PB, pCG-lucPP-HS, pCG-lucPP-PB, pLS-lucPP-HS, pLS-lucPP-PB, pCS-lucPP-HS or pCS-lucPP-PB are prepared from the human embryonic kidney cell line 293 and the Jurkat leukemic T cell line (American Type Culture Collection, Rockville, MD). Each cell line is stably transfected with an expression vector encoding a variant phage T7 RNA polymerase. This variant contains an SV40 T antigen nuclear localization signal (NLS) fused in frame to the N-terminus of the T7 RNA polymerase, permitting its transport into, and function in, the cell nucleus (Lieber et al. (1989) *Nucleic Acids Res.* **17**, 8485-8493). The non-functional indicator gene in the resistance test vector is converted into a functional indicator gene by reverse transcriptase upon infection of the target cells, resulting from the repositioning of the T7 promoter relative to the indicator gene coding region. Following the integration of the repaired indicator gene into the target cell chromosome by HIV integrase, the nuclear T7 RNA polymerase expressed by the target cell is capable of functionally transcribing the indicator gene.

Plasmid pVL-T7RNAP-NLS is used to direct the expression of a variant T7 RNA polymerase linked to an NLS, in human and other mammalian cells and cell lines. pVL-T7RNAP-NLS is prepared from three DNA fragments: 1) a vector fragment of 4.3 kB prepared by digestion of the plasmid pVL-2 with EcoRI and BglII, 2) a DNA fragment of 2.6 kB encoding T7 RNA polymerase amino acid residues 34 to 883 (nucleotides 267 to 2817, coordinates by reference to GenBank, accession number M38308, Grachev and Pletnev (1984) *Bioorg.* Khim. **10,** 824-843) prepared by PCR using plasmid pT7-G1 (Deng et al. (1994) *Gene* **143,** 245-249) as a template with oligonucleotides 39 and 40 as primers, followed by digestion with NruI and BglII, and 3) a synthetic DNA fragment encoding the first three amino acids of SV40 T antigen followed by amino acids 118 to 133 of the SV40 large T antigen containing the NLS (Lieber et al., Ibid), prepared by annealing the overlapping oligonucleotides 41 and 42, extending with Klenow DNA polymerase and digesting with EcoRI and NruI. Oligonucleotides 39, 40, 41, 42 incorporate NruI, BglII, EcoRI and NruI restrictions site, respectively.

Plasmid pVL-Neo is employed as a selectable marker for the establishment of stable transfectants of human and other mammalian cells and cell lines by co-transfection. pVL-Neo directs the expression of neomycin phosphotransferase and confers resistance to the antibiotic G418. Plasmid pVL-Neo is prepared from two DNA fragments: 1) a vector fragment of 4.3 kB prepared by digesting plasmid pVL-2 with EcoRI and BglII, and 2) a DNA fragment of 0.8 kB containing the complete Neo coding region (nucleotides 1551 to 2345 of the Tn5 transposon sequence, coordinates given in GenBank accession number U00004, Beck et al. (1982) *Gene* **19,** 327-336) prepared by PCR using plasmid pSV2neo (Southern and Berg (1982) *J. Mol. Appl. Gen.* **1,** 327-341) as a template with oligonucleotides 43 and 44 as primers, followed by digestion with EcoRI and BglII. Oligonucleotide 43 incorporates a unique EcoRI site followed by a consensus sequence for mammalian translation initiation, while oligonucleotide 44 incorporates a unique BglII site.

pVL-T7RNAP is introduced by stable transfection into 293 cells by the calcium phosphate coprecipitation method (Wigler et al. (1979) *Cell* **16**, 777) and into Jurkat cells by electroporation (Irving et al. (1991) *Cell* **64**, 891-901). 293 cells are maintained in DMEM medium (JRH Biosciences) supplemented with 1 g/L glucose, 10% donor calf serum (Tissue Culture Biologics). Jurkat cells are maintained in RPMI 1640 medium supplemented with 10% fetal bovine serum (Irvin Scientific), glutamine, penicillin and streptomycin. Transfection cocktails for 293 cells and Jurkat cells each contain a mixture of 10 micrograms of pVL-T7RNAP and the selectable marker pVL-Neo in a mass ratio of 10:1 to 20:1. Twenty-four to 48 hours following transfection, cells are replated in the same media containing the antibiotic G418 (GIBCO, Grand Island, N.Y.). Independent 293 cell clones resistant to G418 are picked directly from selection plates after two weeks and are expanded for analysis. Independent Jurkat cell clones resistant to G418 are obtained by limiting dilution after two to three weeks of drug selection and are expanded for analysis.

G418-resistant 293 and Jurkat cell clones are screened for their level of expression of T7 RNA polymerase by determining the level of steady-state T7 RNA polymerase-specific mRNA synthesized by the cells using the Northern blot hybridization method (Ausubel et al. (1987) Current Protocols in Molecular Biology, Wiley-Interscience). 293 and Jurkat cell clones expressing optimal levels of the T7 RNA polymerase are then identified by determining their ability to support T7 RNA polymerase-specific transcription in transient transfections with plasmid pEMCLucbgAn (Deng et al. (1991) *Gene* **109,** 193-201) in which the transcription of the luciferase gene is directed by a T7 promoter. 293 and Jurkat clones supporting the highest levels of luciferase gene expression are chosen for further use; these are referred to as 293/T7RNAP-NLS cells and Jurkat/T7RNAP-NLS cells, respectively.

### Drug Susceptibility and Resistance Tests

Resistance tests are carried out with resistance test vectors based on indicator gene viral vectors pLG-lucPP-HS, pLG-lucPP-PB, pCG-lucPP-HS, pCG-lucPP-PB, pLS-lucPP-HS, pLS-lucPP-PB, pCS-lucPP-HS or pCS-lucPP-PB, using either two types of host cell or one type of host cell. In the first type of resistance test, resistance test vector viral particles are produced by a first host cell (the resistance test vector host cell) that is prepared by transfecting a packaging host cell with the resistance test vector and packaging expression vector(s). The resistance test vector viral particles are then used to infect a second host cell (the target host cell) in which the expression of the indicator gene is measured. In the second type of resistance test, a single host cell type (the resistance test vector host cell) serves both purposes: some of the packaging host cells in a given culture are transfected and produce resistance test vector viral particles and some of the host cells in the same culture are the target of infection by the resistance test vector particles thus produced. Resistance tests employing a single host cell type are possible with resistance test vectors comprising a non-functional indicator gene with a permuted promoter: while such indicator genes are efficiently expressed upon infection of a permissive host cell, they are not efficiently expressed upon transfection of the same host cell type, and thus provide an opportunity to measure the effect of the anti-viral agent under evaluation. For similar reasons, resistance tests employing two cell types may be carried out by co-cultivating the two cell types as an alternative to infecting the second cell type with viral particles obtained from the supernatants of the first cell type.

### Susceptibility and Resistance Test - Two Cell

Resistance test vector host cells are prepared by the cotransfection of a resistance test vector and the appropriate packaging expression vector(s) using either the 293 cell line, the tsa54 or tsa201 cell lines (Heinzel et al. (1988) *J. Virol.* **62,** 3738), or the BOSC 23 cell line (Pear et al. (1993) *Proc. Natl. Acad. Sci.* **90,** 8392) as packaging host cells. Resistance test vectors constructed by inserting patient-derived segment into pLG-lucPP-HS, pCG-lucPP-HS, pLG-lucPP-PB and pCG-lucPP-PB are cotransfected with the packaging expression vector pVL-env4070A, while resistance test vectors prepared by inserting patient-derived segments into pLS-lucPP-HS, pCS-lucPP-HS, pLS-lucPP-PB and pCS-lucPP-PB are cotransfected with the packaging expression vectors pVL-env4070A and either pLTR-HIV3' or pCMV-HIV3'. Jurkat/T7RNAP-NLS cells are employed as target host cells.

Packaging host cells are grown in DMEM media, 1 g/L glucose, 10% donor calf serum and passaged at a 1:10 dilution every three days. Cells are plated 48 hours prior to transfection at 1x10⁶ cells per 10 cm plate. Cells are transfected by the calcium phosphate coprecipitation method using 5 to 10 mg each of the resistance test vector and the appropriate packaging expression vector(s) to produce resistance test vector host cells. Individual anti-viral agents, including the reverse transcriptase inhibitors AZT, ddI, ddC, d4T and 3TC, and the protease inhibitors saquinavir, ritonavir and indinavir, as well as combinations thereof, are added to individual plates of transfected cells at the time of their transfection, at an appropriate range of concentrations. Twenty-four to 48 hours after transfection, target host cells are infected by co-cultivation with resistance test vector host cells or with viral particles obtained from filtered supernatants of resistance test vector host cells. Each anti-viral agent, or combination thereof, is added to the target host cells at the time of infection to achieve the same final concentration of the given agent, or agents, present during the transfection.

For infection by co-cultivation, media is removed from a 10 cm plate of resistance test vector host cells prepared by transfection 24 to 48 hours earlier, and 0.5 to 1.0 x 10⁶ Jurkat/T7RNAP-NLS target cells are added to the plate in Jurkat cell media containing the anti-viral agent at the appropriate concentration. Target cells are co-cultivated with the resistance test vector host cells for 24 hours, then removed and added to freshly prepared resistance test vector host cells for a second co-cultivation in Jurkat cell media containing the anti-viral agent(s) at the appropriate concentration. Twenty-four hours later, the target host cells are harvested from the second co-cultivation, collected by centrifugation, washed three times with ice-cold phosphate-buffered saline (PBS), and assayed for luciferase activity. For infection with filtered supernatants, media is removed from a 10 cm plate of resistance test vector host cells prepared by transfection 24 to 48 hours earlier. The media is filtered through a 0.45 mm filter at the time of harvest, frozen at -70°C, and thawed before transduction. Jurkat/T7RNAP-NLS cells (0.5 to 1.0 x 10⁶) are added to 5 ml of an equal mixture of Jurkat cell media and the filtered supernatant, made up to 8 mg/ml of polybrene (Sigma, St. Louis, MI) and the appropriate concentration of the anti-viral agent(s). Twenty-four to 48 hours post-infection, the target host cells are collected by centrifugation, washed three times with ice-cold phosphate-buffered saline, and assayed for indicator gene expression. Target host cells infected by co-cultivation or with filtered viral supernatants are assayed for firefly luciferase activity as described (Ausubel et al. (1987) Current Protocols in Molecular Biology, Wiley-Interscience). The reduction in luciferase activity observed for target host cells infected with a given preparation of resistance test vector viral particles in the presence of a given anti-viral agent, or agents, as compared to a control run in the absence of the anti-viral agent, generally relates to the log of the concentration of the anti-viral agent as a sigmoidal curve. This inhibition curve is used to calculate the apparent inhibitory concentration (IC) of that agent, or combination of agents, for the viral target product encoded by the patient-derived segments present in the resistance test vector.

### Susceptibility and Resistance Test - One Cell

Resistance test vector host cells are prepared by the cotransfection of a resistance test vector and the appropriate packaging expression vector(s) using either 293/T7RNAP-NLS cells or Jurkat/T7RNAP-NLS cells as packaging host cells. Resistance test vectors constructed by inserting patient-derived segments into pLG-lucPP-HS, pCG-lucPP-HS, pLG-lucPP-PB and pCG-lucPP-PB are cotransfected with the packaging expression vector pVL-env4070A, while resistance test vectors prepared by inserting patient-derived segments into pLS-lucPP-HS, pCS-lucPP-HS, pLS-lucPP-PB and pCS-lucPP-PB are cotransfected with the packaging expression vectors pVL-env4070A and either pLTR-HIV3' or pCMV-HIV3'.

Cells are transfected using 5 to 10 mg each of the resistance test vector and the appropriate packaging expression vector(s) to produce resistance test vector host cells. 293/T7RNAP-NLS cells are transfected by the calcium phosphate coprecipitation method and Jurkat/T7RNAP-NLS cells are transfected by electroporation. Individual anti-viral agents, or combinations thereof, are added to individual plates of transfected cells at the time of their transfection, at an appropriate range of concentrations. Twenty-four to 72 hours after transfection, cells are collected by centrifugation, washed three times with ice-cold phosphate-buffered saline, and assayed for firefly luciferase activity as described. As transfected cells in the culture do not efficiently express the indicator gene, transfected cells in the culture, as well superinfected cells in the culture, can serve as target host cells for indicator gene expression. The reduction in luciferase activity observed for cells transfected in the presence of a given anti-viral agent, or agents as compared to a control run in the absence of the anti-viral agent(s), generally relates to the log of the concentration of the anti-viral agent as a sigmoidal curve. This inhibition curve is used to calculate the apparent inhibitory concentration (IC) of that agent, or combination of agents, for the viral target product encoded by the patient-derived segments present in the resistance test vector.

### EXAMPLE 2

### HIV Drug Susceptibility And Resistance Tests Using Resistance Test Vectors Comprising Patient-derived Segments And A Non-Functional Indicator Gene With A Permuted Coding Region

### Indicator Gene Viral Vector - Construction

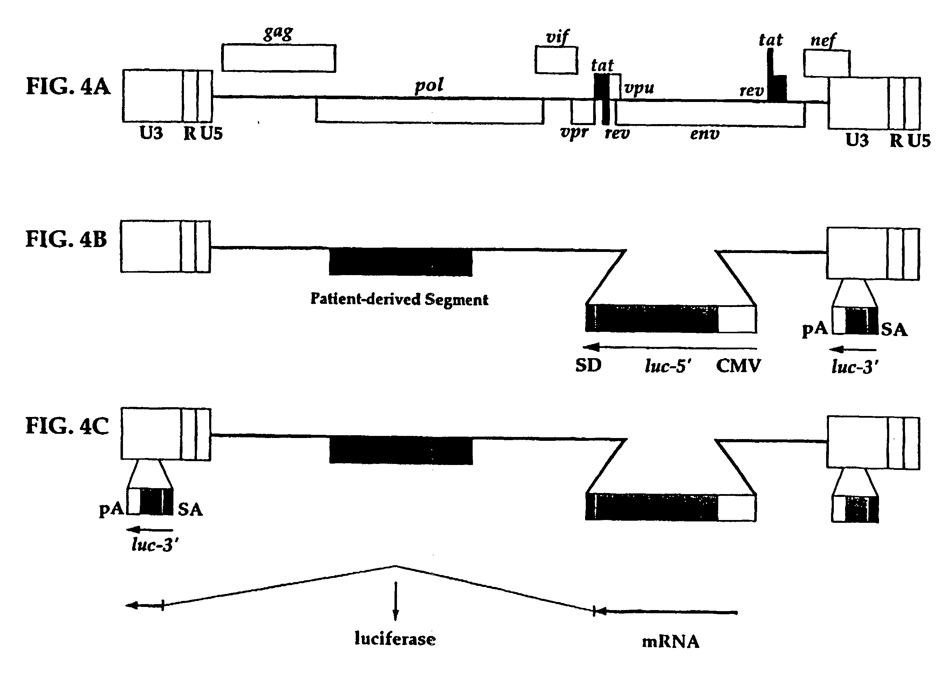

The genomic indicator gene viral vectors with patient sequence acceptor sites, pLG-lucPC-HS, pLG-lucPC-PB, pCG-lucPC-HS and pCG-lucPC-PB, and resistance test vectors derived therefrom, each contain the following elements in a 5' to 3' orientation (Fig 4B): 1) an HIV-LTR U3 region (pLG-lucPC-HS and pLG-lucPC-PB) or a first CMV IE enhancer-promoter (pCG-lucPC-HS and pCG-lucPC-PB), 2) the HIV-LTR R and U5 regions, 3) the coding regions of the HIV *gag-pol, vif, vpr, tat, rev, vpu*, deleted *env*, and *nef* genes, 4) a first indicator gene cassette containing the 5' coding region of the luciferase gene, inserted into the deleted env gene, 5) a second indicator gene cassette containing the 3' coding region of the luciferase gene, inserted into a deleted 3' HIV-LTR U3 region, and 6) a 3' HIV-LTR R and U5 region. pLG-lucPC-HS and pCG-lucPC-HS contain unique HpaI and Sail patient sequence acceptor sites at nucleotides 2243 and 4190 of HXB2, respectively; pLG-lucPC-PB and pCG-lucPC-PB contain unique PvuI and BamHI patient sequence acceptor sites at nucleotides 2221 and 4212 of HXB2, respectively (see Example 1 for details). The first indicator gene cassette contains: 1) a second CMV enhancer-promoter, 2) the 5' coding region of the luciferase gene (amino acids 1 to 446), and 3) a CMV IE splice donor. The second indicator gene cassette contains: 1) an _-globin gene second exon splice acceptor, 2) the 3' coding region of the luciferase gene (amino acids 447 to 550), and 3) an SV40 polyadenylation site. The transcriptional orientation of the luciferase 5' and 3' coding regions are identical to one another, and opposite to that of the first CMV enhancer-promoter and viral LTRs. However, as the luciferase 5' and 3' coding regions are permuted in the resistance test vectors (ie., the 5' coding region is downstream of the 3' coding region), no mRNA is transcribed which can be spliced to generate a functional luciferase coding region. Following reverse transcription and strand transfer, the luciferase 3' coding region is copied from the 3' LTR to the 5' LTR, permitting the transcription of mRNA which can be spliced to generate a functional luciferase coding region (Fig 4C).

The subgenomic indicator gene viral vectors with patient sequence acceptor sites, pLS-lucPC-HS, pLS-lucPC-PB, pCS-lucPC-HS and pCS-lucPC-PB, and resistance test vectors derived therefrom, each contain the following elements in a 5' to 3' orientation (Fig 4B): 1) an HIV-LTR U3 region (pLS-lucPC-HS and pLS-lucPC-PB) or a first CMV IE enhancer-promoter (pCS-lucPC-HS and pCS-lucPC-PB), 2) the HIV-LTR R and U5 regions, 3) the coding region of the HIV *gag-pol* gene, 4) a first indicator gene cassette containing the 5' coding region of the luciferase gene, 5) an RRE element from the HIV *env* gene containing a viral packaging sequence, 6) a second indicator gene cassette containing the 3' coding region of the luciferase gene, inserted into a deleted 3' HIV-LTR U3 region, and 7) a 3' HIV-LTR R and U5 region. pLS-lucPC-HS and pCS-lucPC-HS contain unique HpaI and SalI patient sequence acceptor sites at nucleotides 2243 and 4190 of HXB2, respectively; pLS-lucPC-PB and pCS-lucPC-PB contain unique PvuI and BamHI patient sequence acceptor sites at nucleotides 2221 and 4212 of HXB2, respectively. The first indicator gene cassette contains:
1) a second CMV enhancer-promoter, 2) the 5' coding region of the luciferase gene (amino acids 1 to 446), and 3) a CMV IE splice donor. The second indicator gene cassette contains: 1) an (-globin gene second exon splice acceptor,
2) the 3' coding region of the luciferase gene (amino acids 447 to 550), and 3) an SV40 polyadenylation site. As the luciferase 5' and 3' coding regions are permuted in the resistance test vectors, reverse transcription and strand transfer must occur to generate non-permuted luciferase 5' and 3' coding regions, permitting the transcription of mRNA which can be spliced to generate a functional luciferase coding region (Fig 4C).

Plasmid pVL-luc5', which contains the first indicator gene cassette, is prepared in three steps. In the first two steps, the artificial intron contained in pVL-1 consisting of the CMV IE splice donor and (-globin gene splice acceptor is subjected to site-directed mutagenesis to create restriction sites which upon digestion yield a DNA fragment whose 5' and 3' termini correspond precisely to the start and end of the artificial intron. In step one, site-directed mutagenesis is carried out with pVL-1 using oligonucleotide 45 which contains the following sequences (5' to 3') : 1) the 18 nucleotide sequence which precedes the CMV IE splice donor, 2) a TAC trinucleotide sequence corresponding to first half of the SnaBI restriction site, and 3) the 18 nucleotide sequence at the beginning of the artificial intron. As the sequence of the first three nucleotides of the intron is GTA, the resulting plasmid pVL-SnaBI contains a SnaBI restriction site which upon digestion releases the 5' sequence of the intron as a blunt DNA end. In step two, site-directed mutagenesis is carried out with pVL-SnaBI using oligonucleotide 46 which contains the following sequences (5' to 3'): 1) the 18 nucleotide sequence at the end of the artificial intron, 2) a CTG trinucleotide sequence corresponding to last half of the PvuII restriction site, and 3) the 18 nucleotide sequence following the -globin splice acceptor. As the sequence of the last three nucleotides of the intron is CAG, the resulting plasmid pVL-SnaBI/PvuII contains a PvuII restriction site which upon digestion releases the 3' sequence of the intron as a blunt DNA end. In the third step, plasmid pVL-luc5' is prepared from two DNA fragments: 1) a vector DNA of 5.3 kB prepared by digesting plasmid pVL-luc with EcoRV and NheI, and treating the resulting vector with Klenow DNA polymerase and alkaline phosphatase, and 2) a DNA fragment of 0.1 kB containing the CMV IE splice donor, prepared by digesting plasmid pVL-SnaBI/PvuII with SnaBI and SmaI. Clones corresponding to pVL-luc5', which contain the CMV IE splice donor inserted in the correct orientation into the luciferase coding region, are identified by restriction mapping.

Plasmid pVL-luc3', which contains the second indicator gene cassette, is prepared in three steps. In step one, plasmid pBS-LTR, in which the 3'LTR of pBS-HXB2 is subcloned, is prepared from two DNA fragments: 1) a vector DNA of 3.0 kB prepared by digesting plasmid pBluescript II KS (+) with XhoI and XbaI, and 2) a DNA fragment of 0.8 kB containing the 3'LTR, prepared by digesting pBS-HXB2 with XhoI and XbaI. In step two, plasmid pBS-LTR-luc3', which contains the 3' coding region of luciferase followed by an SV40 polyadenylation site, inserted into the deleted 3'LTR, is prepared from two fragments: 1) a vector DNA of 3.5 kB prepared by digesting pBS-LTR with EcoRV, and treating the resulting vector with alkaline phosphatase, and 2) a DNA fragment of 0.5 kB containing the 3' luciferase coding region and SV40 polyA site, prepared by digesting plasmid pVL-luc with EcoRV and NheI, followed by treatment of the resulting fragment with Klenow DNA polymerase. Clones having the 3' luciferase coding region inserted in the correct orientation (ie., opposite to the direct of transcription in the 3'LTR) are identified by restriction mapping. In step three, plasmid pVL-luc3' is prepared from two DNA fragments: 1) a vector DNA of 4.0 kB prepared by digesting plasmid pBS-LTR-luc3' with EcoRV, followed by treatment of the resulting vector with alkaline phosphatase, and 2) a DNA fragment of 0.1 kB containing the (-globin gene second exon splice acceptor, prepared by digesting plasmid pVL-SnaBI/PvuII with PvuII and SmaI. Clones corresponding to pVL-luc3', which contain the (-globin second exon splice acceptor inserted in the correct orientation into the luciferase coding region, are identified by restriction mapping.

Plasmids pLG-lucPC-HS, pLG-lucPC-PB, pLS-lucPC-HS and pLS-lucPC-PB are prepared by the same three step procedure. In step one, plasmids pLG-lucDP-HS, pLG-lucDP-PB, pLS-lucDP-HS and pLS-lucDP-PB are prepared from two DNA fragments: 1) a vector DNA prepared by digesting plasmids pLG-lucPP-HS, pLG-lucPP-PB, pLS-lucPP-HS, pLS-lucPP-PB, respectively, with SmaI and ClaI, and 2) a DNA fragment of 0.8 kB prepared by digesting plasmid pLG with SmaI and ClaI. In step two, plasmids pLG-luc5'-HS, pLG-luc5'-PB, pLS-luc5'-HS and pLS-luc5'-PB are prepared from two DNA fragments: 1) a vector DNA prepared by digesting plasmids pLG-lucDP-HS, pLG-lucDP-PB, pLS-lucDP-HS and pLS-lucDP-PB, respectively, and treating the resulting vectors with alkaline phosphatase, and 2) a DNA fragment of 2.5 kB containing the first indicator gene cassette prepared by digesting pVL-luc5' with NotI. Clones which contain the first indicator gene cassette inserted into the viral vector with a transcriptional orientation opposite to that of the viral LTRs are identified by restriction mapping. In step three, plasmids pLG-lucPC-HS, pLG-lucPC-PB, pLS-lucPC-HS and pLS-lucPC-PB are prepared from two DNA fragments: 1) a vector DNA prepared by digesting plasmids pLG-luc5'-HS, pLG-luc5'-PB, pLS-luc5'-HS and pLS-luc5'-PB, respectively, with XhoI and XbaI, and 2) a DNA fragment of 1.1 kB containing the second indicator gene cassette, prepared by digesting plasmid pVL-luc3' with XhoI and XbaI.

Plasmids pCG-lucPC-HS, pCG-lucPC-PB, pCS-lucPC-HS and pCS-lucPC-PB are each prepared from two DNA fragments: 1) a vector DNA prepared by digesting either plasmids pLG-lucPC-HS, pLG-lucPC-PB, pLS-lucPC-HS and pLS-lucPC-PB, respectively, with SmaI and ClaI, and 2) a DNA fragment of 1.3 kB prepared by digesting plasmid pCG with SmaI and ClaI.

### Resistance Test Vector - Construction

Resistance test vectors containing a non-functional indicator gene with a permuted coding region were designed using the HIV genomic and subgenomic viral vectors comprising anti-viral target genes described in Example 1.

Resistance test vectors are prepared from plasmids pLG-lucPC-HS, pLG-lucPC-PB, pCG-lucPC-HS, pCG-lucPC-PB, pLS-lucPC-HS, pLSlucPC-PB, pCS-lucPC-HS and pCS-lucPC-PB (Fig. 4B), by the procedure described in Example 1. Resistance test vectors are constructed with vectors prepared from plasmids pLG-lucPC-HS, pCG-lucPC-HS, pLS-lucPC-HS or pCS-lucPC-HS using amplified patient sequences prepared with oligonucleotides 18 and 19, and with oligonucleotides 22 and 23. Resistance test vectors are constructed with vectors prepared from plasmids pLG-lucPC-PB, pCG-lucPC-PB, pLS-lucPC-PB or pCS-lucPC-PB using amplified patient sequences prepared with oligonucleotides 20 and 21, and with oligonucleotides 24 and 25.

### Drug Susceptibility and Resistance Test

Resistance tests are carried out by the procedures described in Example 1 as follows. Resistance test vectors prepared from plasmids pLG-lucPC-HS, pLG-lucPC-PB, pCG-lucPC-HS and pCG-lucPC-PB lack a functional HIV *env* gene, and are used in conjunction with the packaging expression vector pVL-env4070A. Resistance test vectors prepared from plasmids pLS-lucPC-HS, pLS-lucPC-PB, pCS-lucPC-HS and pCS-lucPC-PB encode the HIV *gag-pol* gene products only, and are used in conjunction with pVL-env4070A, and either the pLTR-HIV3' or pCMV-HIV3' packaging expression vectors. In resistance tests carried out using two host cell types, the 293 cell line, the tsa54 cell line, the tsa201 cell line, or the BOSC 23 cell line are employed as packaging host cells, and unmodified Jurkat cells are employed as target cells. As the non-functional indicator genes with permuted coding regions contained within these resistance test vectors are not efficiently expressed upon transfection of the packaging host cells, infection of target host cells is carried out either by co-cultivation with packaging host cells, or by using virus from the packaging host cell supernatant. For similar reasons, resistance tests carried out with these resistance test vectors may employ a single host cell type. Resistance tests using a single host cell type are carried out using either 293, tsa54, tsa201, BOSC 23 or Jurkat cells.

### EXAMPLE 3

### HIV Drug Susceptibility And Resistance Test Using Resistance Test Vectors Comprising Patient-derived Segment(s) And A Non-Functional Indicator Gene With An Inverted Intron

Resistance test vectors containing a non-functional indicator gene with an inverted intron were designed using the HIV genomic and subgenomic viral vectors comprising anti-viral target genes described in Example 1.

### Indicator Gene Viral Vectors - Inverted Intron

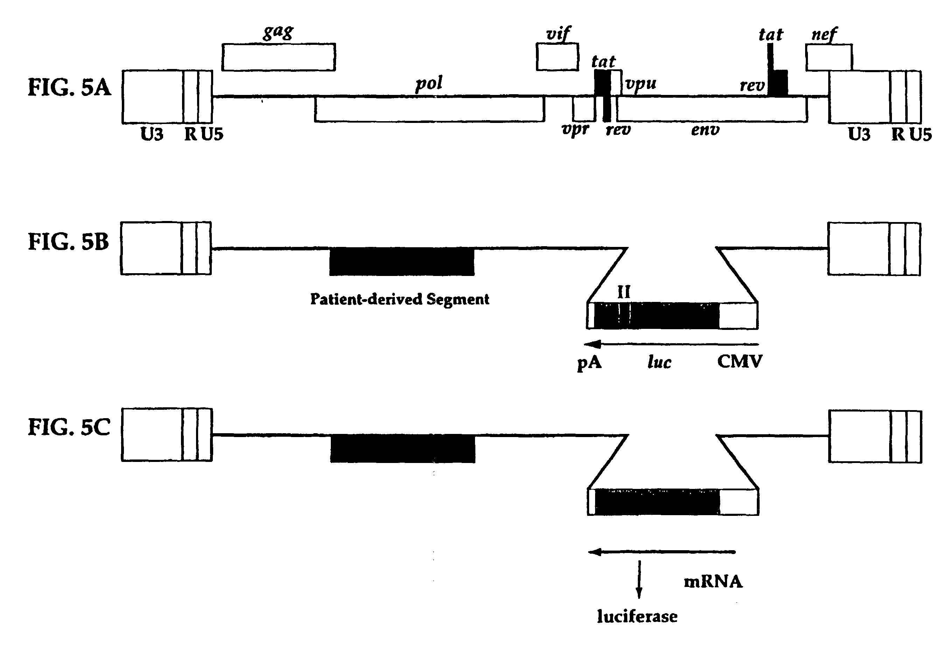

The genomic indicator gene viral vectors with patient sequence acceptor sites, pLG-lucII-HS, pLG-lucII-PB, pCG-lucII-HS and pCG-lucII-PB, and resistance test vectors derived therefrom, each contain the following elements in a 5' to 3' orientation (Fig 5B): 1) an HIV-LTR U3 region (pLG-lucII-HS and pLG-lucII-PB) or a first CMV IE enhancer-promoter (pCG-lucII-HS and pCG-lucII-PB), 2) the HIV-LTR R and U5 regions, 3) the coding regions of the HIV *gag-pol, vif, vpr, tat, rev, vpu,* deleted *env,* and *nef* genes, 4) an indicator gene cassette inserted into the deleted env gene, and 5) a 3' HIV-LTR. pLG-lucII-HS and pCG-lucII-HS contain unique HpaI and SalI patient sequence acceptor sites at nucleotides 2243 and 4190 of HXB2, respectively; pLG-lucII-PB and pCG-lucII-PB contain unique PvuI and BamHI patient sequence acceptor sites at nucleotides 2221 and 4212 of HXB2, respectively (see Example 1 for details). The indicator gene cassette contains 1) a second CMV enhancer-promoter, 2) the coding region of the luciferase gene interrupted by an inverted artificial intron, and 3) an SV40 polyadenylation sequence. The overall transcriptional orientation of the indicator gene cassette is opposite to that of the first CMV enhancer-promoter and viral LTRs, while the orientation of the artificial intron is the same as the latter elements.

Transcription of the indicator gene by the second CMV enhancer-promoter does not lead to the production of functional transcripts as the inverted intron cannot be spliced in this orientation. Transcription of the indicator gene by the 5' viral LTR or the first CMV IE enhancer-promoter, however, leads to the removal of the inverted intron by RNA splicing, although the indicator gene is still not functionally expressed as the resulting transcript has an antisense orientation. Following the reverse transcription of this transcript and integration of the resultant proviral DNA, the indicator gene can be functionally transcribed by the second CMV enhancer-promoter as the inverted intron has been previously removed (Fig 5C).

The subgenomic indicator gene viral vectors with patient sequence acceptor sites, pLS-lucII-HS, pLS-lucII-PB, pCS-lucII-HS and pCS-lucII-PB, and resistance test vectors derived therefrom, each contain the following elements in a 5' to 3' orientation (Fig 5B): 1) an HIV-LTR U3 region (pLS-lucII-HS and pLS-lucII-PB) or a first CMV IE enhancer-promoter (pCS-lucII-HS and pCS-lucII-PB), 2) the HIV-LTR R and U5 regions, 3) the coding region of the HIV *gag-pol* gene, 4) the indicator gene cassette, 5) an RRE element from the HIV *env* gene containing a viral packaging sequence, and 6) a 3' HIV-LTR. pLS-lucII-HS and pCS-lucII-HS contain unique HpaI and SalI patient sequence acceptor sites at nucleotides 2243 and 4190 of HXB2, respectively; pLS-lucII-PB and pCS-lucII-PB contain unique PvuI and BamHI patient sequence acceptor sites at nucleotides 2221 and 4212 of HXB2, respectively. The indicator gene cassette contains 1) a second CMV enhancer-promoter, 2) the coding region of the luciferase gene interrupted by an inverted artificial intron, and 3) an SV40 polyadenylation sequence. As is the case for the pLG-lucII and pCG-lucII genomic viral vectors, the indicator genes of pLS-lucII and pCS-lucII cannot be functionally transcribed until after the inverted intron is removed and reverse transcription and proviral integration occur (Fig 5C).

Plasmid pVL-lucII, which contains the indicator gene cassette, in which the artificial intron from pVL-SnaBI/PvuII is inserted into the luciferase coding region in an inverted orientation, is prepared from two DNA fragments: 1) a vector fragment of 5.8 kB prepared by digesting pVL-luc with EcoRV and treating the resulting vector with alkaline phosphatase, and 2) a DNA fragment of 0.2 kB corresponding precisely to the artificial intron sequence prepared by digesting pVL-SnaBI/PvuII with SnaI and PvuII. Clones corresponding to pVL-lucII which contain the artificial intron inserted into the luciferase coding region in an inverted orientation are identified by restriction mapping.

Plasmids pLG-lucII-HS, pLG-lucII-PB, pLS-lucII-HS and pLS-lucII-PB are prepared from two DNA fragments: 1) a vector DNA prepared by digesting plasmids pLG-lucDP-HS, pLG-lucDP-PB, pLS-lucDP-HS and pLS-lucDP-PB, respectively, and treating the resulting vectors with alkaline phosphatase, and 2) a DNA fragment of 3.2 kB containing the luciferase indicator gene cassette prepared by digesting pVL-lucII with NotI. Clones which contain the correct plucII indicator gene cassette inserted into the viral vector with a transcriptional orientation opposite to that of the viral LTRs are identified by restriction mapping and are used for the preparation of resistance test vectors.

Plasmids pCG-lucII-HS, pCG-lucII-PB, pCS-lucII-HS and pCS-lucII-PB are each prepared from two DNA fragments: 1) a vector DNA prepared by digesting plasmids pLG-lucII-HS, pLG-lucII-PB, pLS-lucII-HS and pLS-lucII-PB, respectively, with SmaI and ClaI, and 2) a DNA fragment of 1.3 kB prepared by digesting plasmid pCG with SmaI and ClaI.

### Resistance Test Vector - Construction

Resistance test vectors are prepared from plasmids pLG-lucII-HS, pLG-lucII-PB, pCG-lucII-HS, pCG-lucII-PB, pLS-lucII-HS, pLS-lucII-PB, pCS-lucII-HS and pCS-lucII-PB (Fig. 5B), by the procedure described in Example 1. Resistance test vectors are constructed with vectors prepared from plasmids pLG-lucII-HS, pCG-lucII-HS, pLS-lucII-HS or pCS-lucII-HS using amplified patient sequences prepared with oligonucleotides 18 and 19, and with oligonucleotides 22 and 23. Resistance test vectors are constructed with vectors prepared from plasmids pLG-lucII-PB, pCG-lucII-PB, pLS-lucII-PB or pCS-lucII-PB using amplified patient sequences prepared with oligonucleotides 20 and 21, and with oligonucleotides 24 and 25.

### Drug Susceptibility and Resistance Test

Resistance tests are carried out by the procedures described in Example 1 as follows. Resistance test vectors prepared from plasmids pLG-lucII-HS, pLG-lucII-PB, pCG-lucII-HS and pCG-lucII-PB lack a functional HIV *env* gene, and are used in conjunction with the packaging expression vector pVL-env4070A. Resistance test vectors prepared from plasmids pLS-lucII-HS, pLS-lucII-PB, pCS-lucII-HS and pCS-lucII-PB encode the HIV *gag-pol* gene products only, and are used in conjunction with pVL-env4070A, and either the pLTR-HIV3' or pCMV-HIV3' packaging expression vectors. In resistance tests carried out using two host cell types, the 293 cell line, the tsa54 cell line, the tsa201 cell line, or the BOSC 23 cell line are employed as packaging host cells, and unmodified Jurkat cells are employed as target cells.

As the non-functional indicator genes with inverted introns contained within these resistance test vectors are not efficiently expressed upon transfection of the packaging host cells, infection of target host cells is carried out either by co-cultivation with packaging host cells, or by using virus from the packaging host cell supernatant. For similar reasons, resistance tests carried out with these resistance test vectors may employ a single host cell type. Resistance tests using a single host cell type are carried out using either 293, tsa54, tsa201, BOSC 23 or Jurkat cells.

### EXAMPLE 4

### Non-Particle Based HIV Drug Susceptibility And Resistance Test Using Resistance Test Vectors Comprising Patient-derived Segment(s) And A Non-functional Indicator Gene.

### Drug Susceptibility and Resistance Test

Non-particle based resistance tests are carried out using the resistance test vectors comprising non-functional indicator genes with either permuted promoters, permuted coding regions or inverted introns, described in Examples 1, 2 and 3. These non-particle based resistance tests are performed by transfection of a single host cell type with each resistance test vector in the absence of packaging expression vectors. Although the non-functional indicator genes contained within these resistance test vectors are not efficiently expressed upon transfection of the host cells, there is detectable indicator gene expression resulting from non-viral particle-based reverse transcription. Reverse transcription and strand transfer results in the conversion of the permuted, non-functional indicator gene to a non-permuted, functional indicator gene. As reverse transcription is completely dependent upon the expression of the *pol* gene contained within each resistance test vector, anti-viral agents may be tested for their ability to inhibit the *pol* gene products encoded by the patient-derived segments contained within the resistance test vectors. Non-particle based resistance tests are carried out by the general procedures described in Example 1 with the following modifications: 1) Resistance test vectors are transfected into the appropriate host cells, 2) anti-viral agents, or combinations thereof, are added at appropriate concentrations to individual cultures of transfected host cells immediately after transfection, 3) host cells are harvested 24 to 72 hours following transfection and assayed for luciferase activity. The reduction in luciferase activity observed for host cells transfected with a given resistance test vector in the presence of a given anti-viral agent, or agents, as compared to a control run in the absence of the anti-viral agent(s) is used to calculate the apparent inhibitory content (Ki) of that agent, or combination of agents, for the viral target gene product encoded by the patient-derived segments present in the resistance test vector.

### Resistance Test Vector - Construction

For non-particle based resistance tests with resistance test vectors comprising a non-functional indicator gene with a permuted promoter, resistance test vectors are prepared as described in Example 1 using plasmids pLG-lucPP-HS, pLG-lucPP-PB, pCG-lucPP-HS, pCG-lucPP-PB, pLS-lucPP-HS, pLS-lucPP-PB, pCS-lucPP-HS or pCS-lucPP-PB. Each resistance test vector is transfected into host cells expressing a cytoplasmic T7 RNA polymerase (eg., 293/T7RNAP cells or Jurkat/T7RNAP cells). Such host cells are prepared by the stable transfection of 293 cells and Jurkat cells as described in Example 1, using plasmid pVL-T7RNAP, which directs the expression of a cytoplasmic phage T7 RNA polymerase in human and other mammalian cells and cell lines. pVL-T7RNAP is constructed from the following two DNA fragments: 1) a vector fragment of 4.3 kB prepared by digestion of the mammalian expression vector pVL-2 with EcoRI and BglII, and 2) a DNA fragment of 2.6 kB containing the complete coding region of the T7 RNA polymerase (nucleotides 166 to 2815, coordinates given in GenBank accession number M38308, Grachev and Pletnev (1984) *Bioorg. Khim,* **10,** 824-843) prepared by PCR using plasmid pT7-G1 as a template with oligonucleotides 47 and 40 as primers, followed by digestion with EcoRI and BglII. Oligonucleotide 47 incorporates a unique EcoRI site followed by a consensus sequence for eukaryotic translation initiation (e.g., Kozak (1991) *J. Biol. Chem,* **266**, 19867-19870), while oligonucleotide 40 incorporates a unique BglII site.

For non-particle based resistance tests with resistance test vectors comprising a non-functional indicator gene with a permuted coding region or inverted intron, resistance test vectors are prepared as described in Example 1 using plasmids pLG-lucPC-HS, pLG-lucPC-PB, pCG-lucPC-HS, pCG-lucPC-PB. pLS-lucPC-HS, pLS-lucPC-PB, pCS-lucPC-HS, pCS-lucPC-PB, pLG-lucII-HS, pLG-lucII-PB, pCG-lucII-HS, pCG-lucII-PB, pLS-lucII-HS, pLS-lucII-PB, pCS-lucII-HS or pCS-lucII-PB. Each resistance test vector is transfected into either 293, tsa54, tsa201, BOSC 23 or Jurkat cells.

### EXAMPLE 5

### HIV Drug Susceptibility And Resistance Test Using Resistance Test Vectors Comprising Patient-derived Segment(s) And A Functional Indicator Gene.

### Indicator Gene Viral Vector - Functional Indicator Gene

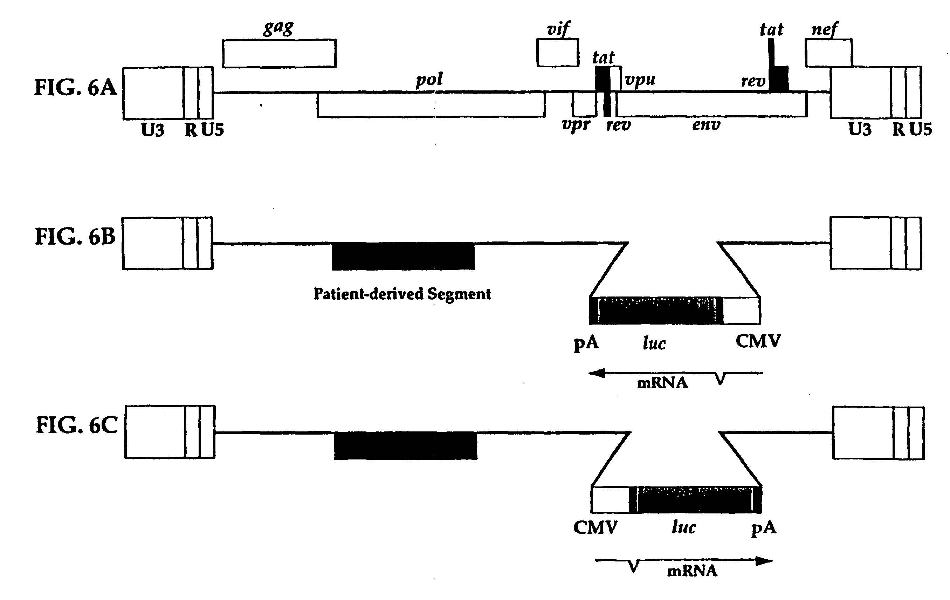

The genomic indicator gene viral vectors with patient sequence acceptor sites, plasmids pLG-luc-HS-1, pLG-luc-HS-2, pLG-luc-PB-1, pLG-luc-PB-2, pCG-luc-HS-1, pCG-luc-HS-2, pCG-luc-PB-1 and pCG-luc-PB-2, and resistance test vectors derived therefrom, each contain the following elements in a 5' to 3' orientation (Fig 6): 1) an HIV-LTR U3 region (pLG-luc-HS-1, pLG-luc-HS-2, pLG-luc-PB-1 and pLG-luc-PB-2) or a first CMV IE enhancer-promoter (pCG-luc-HS-1, pCG-luc-HS-2, pCG-luc-PB-1 and pCG-luc-PB-2), 2) the HIV-LTR R and U5 regions, 3) the coding regions of the HIV *gag-pol, vif, vpr, tat, rev, vpu*, deleted *env*, and *nef* genes, 4) an indicator gene cassette inserted into the deleted *env* gene, and 5) a 3' HIV-LTR. pLG-luc-HS-1, pLG-luc-HS-2, pCG-luc-HS-1 and pCG-luc-HS-2 contain unique HpaI and SalI patient sequence acceptor sites at nucleotides 2243 and 4190 of HXB2, respectively; pLG-luc-PB-1, pLG-luc-PB-2, pCG-luc-PB-1 and pCG-luc-PB-2 contain unique PvuI and BamHI patient sequence acceptor sites at nucleotides 2221 and 4212 of HXB2, respectively (see Example 1 for details). The indicator gene cassettes of each plasmid contain 1) a second CMV enhancer-promoter, 2) the coding region of the luciferase gene, and 3) an SV40 polyadenylation sequence. The indicator gene cassettes of pLG-luc-HS-1, pLG-luc-PB-1, pCG-luc-HS-1 and pCG-luc-PB-1 are inserted into the vector with a transcriptional orientation opposite to the viral LTRs or first CMV enhancer-promoter (Fig. 6B), while the indicator gene cassettes of pLG-luc-HS-2, pLG-luc-PB-2, pCG-luc-HS-2 and pCG-luc-PB-2 are inserted into the vector with the same orientation (Fig. 6C).

The subgenomic indicator gene viral vectors with patient sequence acceptor sites, plasmids pLS-luc-HS-1, pLS-luc-HS-2, pLS-luc-PB-1, pLS-luc-PB-2, pCS-luc-HS-1, pCS-luc-HS-2, pCS-luc-PB-1 and pCS-luc-PB-2, and resistance test vectors derived therefrom, each contain the following elements in a 5' to 3' orientation (Fig 6): 1) an HIV-LTR U3 region (pLS-luc-HS-1, pLS-luc-HS-2, pLS-luc-PB-1 and pLS-luc-PB-2) or a first CMV IE enhancer-promoter (pCS-luc-HS-1, pCS-luc-HS-2, pCS-luc-PB-1 and pCS-luc-PB-2), 2) the HIV-LTR R and U5 regions, 3) the coding region of the HIV *gag-pol* gene, 4) the indicator gene cassette, 5) an RRE element from the HIV env gene containing a viral packaging sequence, and 6) a 3' HIV-LTR. pLS-luc-HS-1, pLS-luc-HS-2, pCS-luc-HS-1 and pCS-luc-HS-2 contain unique HpaI and SalI patient sequence acceptor sites at nucleotides 2243 and 4190 of HXB2, respectively; pLS-luc-PB-1, pLS-luc-PB-2, pCS-luc-PB-1 and pCS-luc-PB-2 contain unique PvuI and BamHI patient sequence acceptor sites at nucleotides 2221 and 4212 of HXB2, respectively. The indicator gene cassettes of each plasmid contain 1) a second CMV enhancer-promoter, 2) the complete coding region of the luciferase gene, and 3) an SV40 polyadenylation sequence. The indicator gene cassettes of pLS-luc-HS-1, pLS-luc-PB-1, pCS-luc-HS-1 and pCS-luc-PB-1 are inserted into the vector with a transcriptional orientation opposite to the viral LTRs or first CMV enhancer-promoter (Fig. 6B), while the indicator gene cassettes of pLS-luc-HS-2, pLS-luc-PB-2, pCS-luc-HS-2 and pCS-luc-PB-2 are inserted into the vector with the same orientation (Fig. 6C).

Plasmids pLG-luc-HS-1 and pLG-luc-HS-2, pLG-luc-PB-1 and pLG-luc-PB-2, pCG-luc-HS-1 and pCG-luc-HS-2, pCG-luc-PB-1 and pCG-luc-PB-2, pLS-luc-HS-1 and pLS-luc-HS-2, pLS-luc-PB-1 and pLS-luc-PB-2, pCS-luc-HS-1 and pCS-luc-HS-2, pCS-luc-PB-1 and pCS-luc-PB-2 are each prepared from two DNA fragments: 1) a vector DNA prepared by digesting either pLG-lucII-HS, pLG-luII-PB, pCG-lucII-HS, pCG-lucII-PB, pLS-luc-HS, pLS-lucII-PB, pCS-lucII-HS and pCS-lucII-PB, respectively with NotI and treating the resulting vectors with alkaline phosphatase, and 2) a DNA fragment of 3.0 kB containing the luciferase indicator gene cassette prepared by digesting pVL-luc with NotI. Clones containing the indicator gene cassette inserted into a given viral vector in both transcriptional orientations relative to the viral LTRs are identified by restriction mapping (eg., pLG-luc-HS-1 and pLG-luc-HS-2).

### Resistance Test Vector - Construction

Resistance test vectors containing a functional indicator gene were designed using the HIV genomic and subgenomic viral vectors comprising anti-viral target genes described in Example 1. Resistance test vectors are prepared from the above plasmids (Fig. 6) by the procedure described in Example 1. Resistance test vectors are constructed with vectors prepared from plasmids pLG-luc-HS-1, pLG-luc-HS-2, pCG-luc-HS-1, pCG-luc-HS-2, pLS-luc-HS-1, pLS-luc-HS-2, pCS-luc-HS-1 or pCS-luc-HS-1 using amplified patient sequences prepared with oligonucleotides 18 and 19, and with oligonucleotides 22 and 23. Resistance test vectors are constructed with vectors prepared from plasmids pLG-luc-PB-1, pLG-luc-PB-2, pCG-luc-PB-1, pCG-luc-PB-2. pLS-luc-PB-1, pLS-luc-PB-2, pCS-luc-PB-1 or pCS-luc-PB-1 using amplified patient sequences prepared with oligonucleotides 20 and 21, and with oligonucleotides 24 and 25.

### Drug Susceptibility and Resistance Test

Resistance tests are carried out by the procedures described in Example 1 as follows. Resistance test vectors prepared from plasmids pLG-luc-HS-1, pLG-luc-HS-2, pLG-luc-PB-1, pLG-luc-PB-2, pCG-luc-HS-1, pCG-luc-HS-2, pCG-luc-PB-1 and pCG-luc-PB-2 lack a functional HIV *env* gene, and are used in conjunction with the packaging expression vector pVL-env4070A. Resistance test vectors prepared from plasmids pLS-luc-HS-1, pLS-luc-HS-2, pLS-luc-PS-1. pLS-luc-PB-2, pCS-luc-HS-1, pCS-luc-HS-2, pCS-luc-PB-1 and pCS-luc-PB-2 encode the HIV *gag-pol* gene products only, and are used in conjunction with pVL-env4070A, and either the pLTR-HIV3' or pCMV-HIV3' packaging expression vectors. Resistance tests are carried out with two host cell types, using the 293 cell line, the tsa54 cell line, the tsa201 cell line, or the BOSC 23 cell line as packaging host cells, and using unmodified Jurkat cells as target cells. The infection of target cells with these or other resistance test vectors containing functional indicator genes is carried out employing the procedure for infection with resistance test vector viral particles from the filtered supernatants obtained from resistance test vector indicator host cells, as described in Example 1. In contrast to resistance test vectors comprising non-functional indicator gene viral vectors with a permuted promoter or an inverted intron, those comprising a functional indicator genes are typically able to express their indicator genes in the transfected packaging host cells. Neither the co-cultivation procedure, nor the resistance test using a single cell type can therefore be readily adapted for the infection of target cells using resistance test vectors with functional indicator genes, as it would be difficult to distinguish between indicator gene expression in infected target host cells and the transfected packaging host cells.

All publications and patent applications cited in this specification are herein incorporated by reference in their entirety as if each individual publication or patent application were specifically and individually indicated to be incorporated by reference.

As will be apparent to those skilled in the art to which the invention pertains, the present invention may be embodied in forms other than those specifically disclosed above, for example to carry out the drug susceptibility and resistance test on other viruses, without departing from the spirit or essential characteristics of the invention. The particular embodiments of the invention described above, are, therefore to be considered as illustrative and not restrictive. The scope of the present invention is as set forth in the appended claims rather than being limited to the examples contained in the foregoing description.

### EXAMPLE 6

### HIV Drug Susceptibility And Resistance Test Using Resistance Teat Vectors Comprising Patient-derived Segment(s) And A Functional Indicator Gene.

### Drug Susceptibility and Resistance Tests

Resistance tests were carried out with resistance test vectors based on the indicator gene viral vectors pCG-CXCN(F-lucP)2, and pCG-CXAT(F-lucP)2, both of which are similar to the pCG-luc-2 described in Example 5, using two host cell types. In the case of pCG-CXCN(F-lucP)2 the indicator gene viral vector was modified in that the indicator gene cassette lacked intron A (CMV/α-globin intron described above), not contain a polyadenylation signal and the downstream 3' U5 sequence was ommitted in the construction. The downstream U5 was replaced with the SV40. poly A signal and origin of replication regions. The indicator gene viral vector pCG-CXAT(F-lucP)2 differed from pCG-CXCN(F-lucP)2 in that the indicator gene cassette contained an artificial intron downstream of the CMV enhancer-promoter and a TK polyadenylation signal region. Resistance test vector viral particles were produced by a first host cell (the resistance test vector host cell) that was prepared by transfecting a packaging host cell with the resistance test vector and the packaging expression vector. The resistance test vector viral particles were then used to infect a second host cell (the target host cell) in which the expression of the indicator gene is measured.

### AZT Drug Susceptibility/Resistance Tests

The resistance test vector pCG-luc-2 containing a functional luciferase gene cassette was constructed and host cells were transfected with the resistance test vector DNA. The resistant test vectors contained "test" patient-derived reverse transcriptase sequences that were either susceptible or resistant to the nucleoside reverse transcriptase inhibitor, AZT (Sigma). The resistance test vector viral particles produced by transfecting the resistance test vector DNA into host cells were used to infect target host cells grown either in the absence of AZT or in the presence of increasing concentrations of the drug (ranging from approximately 0.0001µM to 1000µM). The amount of luciferase activity produced in infected target host cells in the presence of drug was compared to the amount of luciferase produced in infected target host cells in the absence of drug. Drug resistance was measured as the amount of drug required to inhibit by 50% the luciferase activity detected in the absence of drug (inhibitory concentration 50%, IC₅₀). The IC₅₀ values were determined by plotting percent drug inhibition vs. log₁₀ drug concentration.

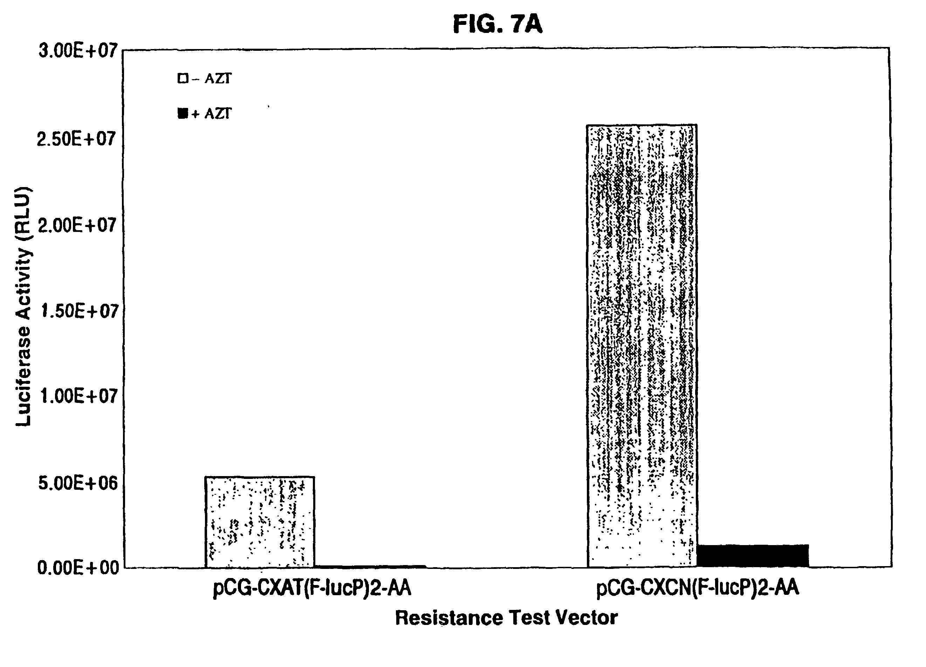

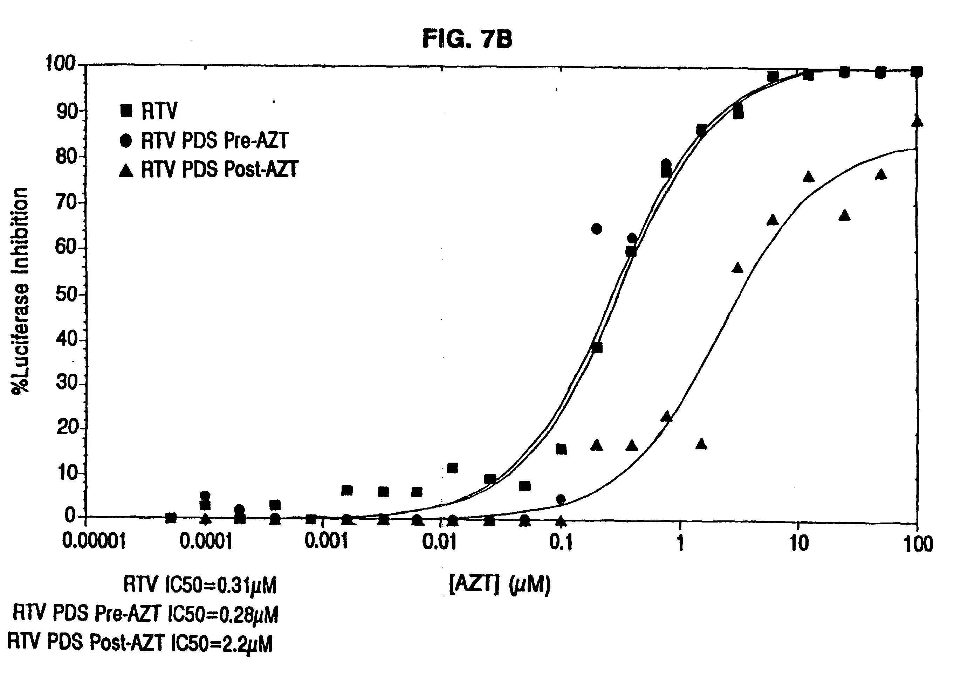

Host cells (293) were seeded in 10-cm-diameter dishes and were transfected several days after plating with resistance test vector plasmid DNA and the envelope expression vector pCXAS(4070A-*env*). Transfections were performed using a calcium-phosphate precipitation procedure. The cell culture media containing the DNA precipitate was replaced with fresh medium, from one to 24 hours, after transfection. Cell culture media containing resistance test vector viral particles was harvested one to four days after transfection and was passed through a 0.45-mm filter before being stored at -80° C. HIV capsid protein (p24) levels in the harvested cell culture media were determined by an EIA method as described by the manufacturer (SIAC; Frederick, MD). Six to forty-eight hours before infection, target cells (293 and 293/T) were plated in cell culture media containing no AZT or serial two fold dilutions of AZT beginning at 100µM and ending at 0.00005µM. The AZT concentrations were maintained throughout the infection. Target host cells were inoculated with 90µl of transfected resistance test vector host cell supernatant. Control infections were performed using cell culture media from mock transfections (no DNA) or transfections containing the resistance test vector plasmid DNA without the envelope expression plasmid DNA (pCXAS(4070A-*env*)). One to twenty-four hours after the inoculation, fresh medium was added to each well. Twelve to thirty-six hours later the media was completely replaced with fresh media. One to three or more days after infection the media was removed and cell lysis buffer (Promega) was added to each well. Cell lysates were diluted 100 fold in lysis buffer and each diluted cell lysate was assayed for luciferase activity (Fig. 7A). The inhibitory effect of AZT was determined using the following equation:${\text{% luciferase inhibition = 1 - (RLUluc}}_{\text{[AZT]}} \text{+ RLUluc)}$ where RLUluc _{[AZT]} is the Relative Light Unit of luciferase activity in infected cells in the presence of AZT and RLUluc is the Relative Light Unit of luciferase activity in infected cells in the absence of AZT. IC₅₀ values were obtained from the sigmoidal curves that were generated from the data by plotting the percent inhibition of luciferase activity vs. the log₁₀ drug concentration. The AZT inhibition curves are shown in (Fig. 7B).

### Nevirapine Drug Susceptibility/Resistance Test

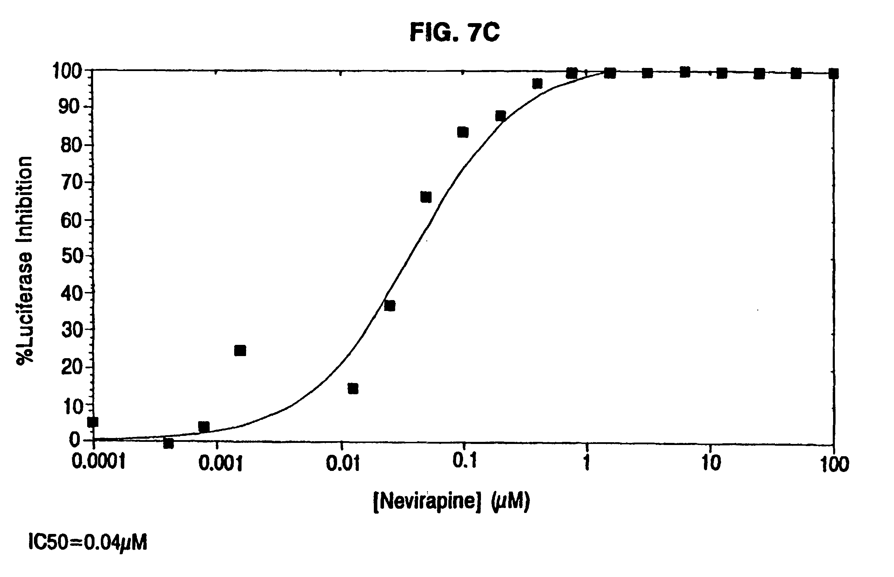

The resistant test vector, based on the indicator gene viral vector pCG-CXCN(F-lucP)2, contained the reverse transcriptase sequence derived from the biologically active proviral clone, pNL4-3, that is susceptible to the non-nucleoside reverse transcriptase inhibitor, nevirapine (BI-RG-587, Boehringer Ingleheim). Transfection of host packaging cells and infection of host target cells were performed as described for the AZT drug susceptibility/resistance tests as described above. Nevirapine susceptibility/resistance was evaluated using nevirapine concentrations ranging from 0.0001µM to 100µM. The nevirapine inhibition curve was determined as described above for AZT and is shown in Figure 7C.

### Indinavir Drug Susceptibility/Resistance Tests

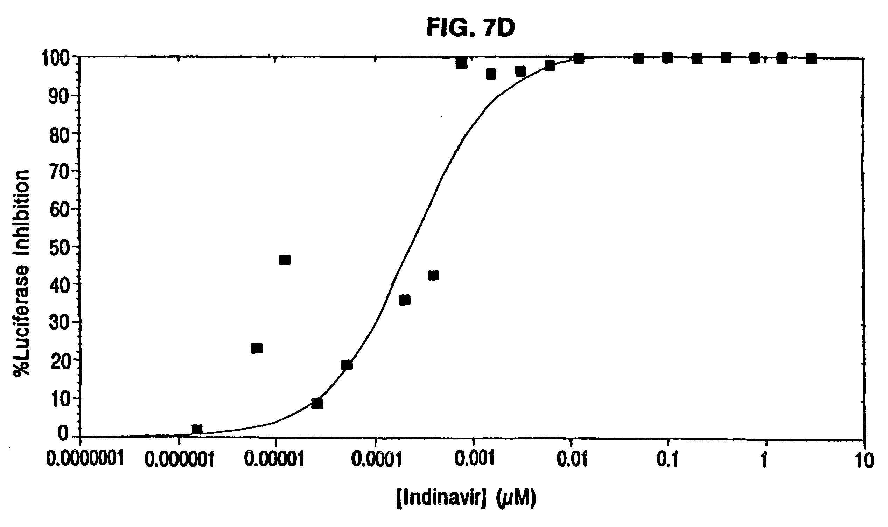

The resistant test vector, based on the indicator gene viral vector pCG-CXCN(F-lucP)2, contained the protease sequence derived from the biologically active proviral clone, pNL4-3, that is susceptible to the protease inhibitor, indinavir (MK-639, Merck). Transfection of host packaging cells and infection of host target cells were performed as described for the AZT drug susceptibility/resistance test except that the protease inhibitor, indinavir, was present in the transfected packaging host cell cultures as well as the infected target host cell cultures, as described above. Indinavir susceptibility/resistance was evaluated using indinavir concentrations ranging from 1.5pM to 3µM. The indinavir inhibition curve was determined as described above for AZT and is shown in Figure 7D.

### Oligonucleotides

1)
2)
3)
4)
5)
6)
7)
8)
9)
10)
11)
12)
13)
14)
15)
16)
17)
18)
19)
20)
21)
22)
23)
24)
25)
26)
27)
28)
29)
30)
31)
32)
33)
34)
35)
36)
37)
38)
39)
40)
41)
42)
43)
44)
45)
46)
47)

## Claims

1. A method for determining susceptibility for an anti-HIV drug comprising:
(a) introducing a resistance test vector comprising a HIV patient-derived segment and an indicator gene into a host cell;
(b) culturing the host cell from (a);
(c) measuring expression of the indicator gene in a target host cell, wherein the expression of the indicator gene is dependent upon the HIV patient-derived segment; and
(d)comparing the expression of the indicator gene from (c) with the expression of the indicator gene measured when steps (a)-(c) are carried out in the absence of the anti-HIV drug, wherein a test concentration of the anti-HIV drug is present at steps (a)-(c); at steps (b)-(c); or at step (c).

2. The method of claim 1, wherein the resistance test vector comprises DNA of a genomic viral vector.

3. The method of claim 1, wherein the resistance test vector comprises DNA of a subgenomic viral vector.

4. A method for determining anti-HIV drug resistance in a patient comprising:
(a) developing a standard curve of drug susceptibility for an anti-HIV drug;
(b) determining anti-HIV drug susceptibility in the patient according to the method of claim 1; and
(c) comparing the anti-HIV drug susceptibility in step (b) with the standard curve determined in step (a), wherein a decrease in anti-HIV susceptibility indicates development of anti-HIV drug resistance in the patient.

5. The method of claim 1, wherein the resistance test vector comprises DNA of HIV.

6. The method of claim 5, wherein the resistance test vector comprises DNA encoding vif, vpr, tat, rev, vpu, and nef.

7. The method of claim 1, wherein the patient-derived segment comprises a functional viral sequence.

8. The method of claim 1, wherein the patient-derived segment encodes one protein that is the target of an anti- HIV drug.

9. The method of claim 1, wherein the patient-derived segment encodes two or more proteins that are the targets of one or more anti- HIVdrugs.

10. A method for determining anti-HIV drug resistance in a patient comprising:
(a) determining anti-HIV drug susceptibility in the patient at a first time according to the method of claim 1, wherein the patient-derived segment is obtained from the patient at about said time;
(b) determining anti-HIV drug susceptibility of the same patient at a later time; and
(c) comparing the anti-HIV drug susceptibilities determined in step (a) and (b), wherein a decrease in anti-HIV drug susceptibility at the later time compared to the first time indicates development or progression of anti-HIV drug resistance in the patient.

11. The method of claim 1, wherein the patient-derived segment comprises an HIV gene.

12. The method of claim 11, wherein the patient-derived segment comprises an HIV gag-pol gene.

13. The method of claim 1, wherein the indicator gene is a functional indicator gene and the host cell is a resistance test vector host cell, including the additional step of infecting the target host cell with resistance test vector viral particles using filtered supernatants from said resistance test vector host cells.

14. The method of claim 1, wherein the indicator gene is a non-functional indicator gene.

15. The method of claim 14, wherein the host cell is a packaging host cell/resistance test vector host cell.

16. The method of claim 15, wherein the culturing is by co-cultivation.

17. The method of claim 15, wherein the target host cell is infected with resistance test vector viral particles using filtered supernatants from said packaging host cell/resistance test vector host cells.

18. The method of claim 1, wherein the indicator gene is a luciferase gene.

19. The method of claim 1, wherein the indicator gene is an E. coli lacZ gene.

20. The method of claim 15, wherein the packaging host cell/resistance test vector host cell is a human cell.

21. The method of claim 15, wherein the packaging host cell/resistance test vector host cell is a human embryonic kidney cell.

22. The method of claim 15, wherein the packaging host cell/resistance test vector host cell is a 293 cell.

23. The method of claim 1, wherein the target host cell is a human T cell.

24. The method of claim 1, wherein the target host cell is a human T cell leukemia cell line.

25. The method of claim 1, wherein the target host cell is a Jurkat cell line.

26. The method of claim 1, wherein the target host cell is a H9 cell line.

27. The method of claim 1, wherein the target host cell is a CEM cell line.

28. The method of claim 1, wherein the target host cell is a human embryonic kidney cell.

29. The method of claim 1, wherein the target host cell is a 293 cell.

30. A resistance test vector comprising an HIV patient-derived segment and an indicator gene, wherein the expression of the indicator gene is dependent upon the patient-derived segment.

31. The resistance test vector of claim 30, wherein the patient-derived segment encodes one protein that is the target of an anti-HIV drug.

32. The resistance test vector of claim 30, wherein the patient-derived segment encodes two or more proteins that are the targets of one or more anti-HIV drugs.

33. The resistance test vector of claim 30, wherein the patient-derived segment comprises an HIV gene.

34. The resistance test vector of claim 30, wherein the patient-derived segment comprises DNA of HIV.

35. The resistance test vector of claim 34, wherein the patient-derived segment comprises DNA encoding vif, vpr, tat, rev, vpu, and nef.

36. The resistance test vector of claim 33, wherein the patient-derived segment comprises an HIV gag-pol gene.

37. The resistance test vector of claim 30, wherein the indicator gene is a functional indicator gene.

38. The resistance test vector of claim 30, wherein the indicator gene is a non-functional indicator gene.

39. The resistance test vector of claim 30, wherein the indicator gene is a luciferase gene.

40. A packaging host cell transfected with a resistance test vector of claim 30.

41. The packaging host cell of claim 40 that is a mammalian host cell.

42. The packaging host cell of claim 40 that is a human host cell.

43. The packaging host cell of claim 40 that is a human embryonic kidney cell.

44. The packaging host cell of claim 40 that is 293 cells.

45. The resistance test vector of claim 30 comprising an indicator gene viral vector and a packaging vector said indicator gene viral vector comprising an indicator gene and said packaging vector comprising a patient-derived segment.

46. A method for determining susceptibility for an anti-HIV drug comprising:
(a) introducing a resistance test vector comprising a HIV patient-derived segment and a nonfunctional indicator is gene into a host cell;
(b) culturing the host cell from (a);
(c) measuring expression of the indicator gene in a target host cell, wherein the expression of the indicator gene is dependent upon the HIV patient- derived segment; and
(d) comparing the expression of the indicator gene from (c) with the expression of the indicator gene measured when steps (a)-(c) are carried out in the absence of the anti-HIV drug,
wherein a test concentration of the anti-HIV drug is present at steps (a)-(c); at steps (b)-(c); or at step (c).

47. The method of claim 46, wherein the resistance test vector comprises DNA of a genomic viral vector.

48. The method of claim 46, wherein the resistance test vector comprises DNA of a subgenomic viral vector.

49. The method of claim 46, wherein the resistance test vector comprises DNA of HIV.

50. The method of claim 49, wherein the resistance test vector comprises DNA encoding vif, vpr, tat, rev, vpu, and nef.

51. The method of claim 46, wherein the patient-derived segment encodes one protein that is the target of an anti-HIV drug.

52. The method of claim 46, wherein the patient-derived segment encodes two or more proteins that are the targets of one or more anti-HIV drugs .

53. The method of claim 46, wherein the patient-derived segment comprises an HIV gene.

54. The method of claim 53, wherein the patient-derived segment comprises an HIV gag-pol gene.

55. The method of claim 46, wherein the indicator gene is a luciferase gene.

56. The method of claim 46, wherein the host cell is a packaging host cell.

57. The method of claim 46, wherein the packaging host cell is a human cell.

58. The method of claim 46, wherein the packaging host cell is a human embryonic kidney cell.

59. The method of claim 46, wherein the packaging host cell is a 293 cell.

60. The method of claim 46, wherein the nonfunctional indicator gene comprises a permuted promoter.

61. The method of claim 46, wherein the nonfunctional indicator gene comprises a permuted coding region.

62. The method of claim 46, wherein the nonfunctional indicator gene comprises an inverted intron.

63. The method of claim 46, wherein the host cell and target host cell are the same cell.

64. The method of claim 46, wherein the target host cell is a human cell.

65. The method of claim 46, wherein the target host cell is a human T cell.

66. The method of claim 46, wherein the target host cell is a human embryonic kidney cell.

67. The method of claim 46, wherein the target host cell is a 293 cell.

68. The method of claim 46, wherein the target host cell is infected with resistance test vector viral particles using filtered supernatants from said packaging host cell/resistance test vector host cell.

69. The method of claim 46, wherein said culturing is by co-cultivation.

70. A method for determining anti-HIV drug resistance in a patient comprising:
(a) developing a standard curve of drug susceptibility for an anti-HIV drug;
(b) determining anti-HIV drug susceptibility in the patient according to the method of claim 1; and
(c) comparing the anti-HIV drug susceptibility in step (b) with the standard curve determined in step (a), wherein a decrease in anti-HIV susceptibility indicates development of anti-HIV drug resistance in the patient.

71. A method for determining anti-HIV drug resistance in a patient comprising:
(a) determining anti-HIV drug susceptibility in the patient at a first time according to the method of claim 46, wherein the patient-derived segment is obtained from the patient at about said time;
(b) determining anti-HIV drug susceptibility of the same patient at a later time; and
(c) comparing the anti-HIV drug susceptibilities determined in step (a) and (b), wherein a decrease in anti-HIV drug susceptibility at the later time compared to the first time indicates development or progression of anti-HIV drug resistance in the patient.

72. A method for evaluating the biological effectiveness of a candidate anti-HIV drug compound comprising:
(a) introducing a resistance test vector comprising a HIV patient-derived segment and an indicator gene into a host cell;
(b) culturing the host cell from step (a);
(c) measuring expression of the indicator gene in a target host cell, wherein the expression of the indicator gene is dependent upon the HIV patient-derived segment; and
(d) comparing the expression of the indicator gene from step (c) with the expression of the indicator gene measured when steps (a)-(c) are carried out in the absence of the candidate anti-HIV drug compound, wherein a test concentration of the candidate anti-HIV drug compound is present at steps (a)-(c); at steps (b)-(c) ; or at step (c).

73. The method of claim 72, wherein the resistance test vector comprises DNA of HIV.

74. The method of claim 72, wherein the resistance test vector comprises DNA encoding HIV gag-pol.

75. The method of claim 72, wherein the patient-derived segment encodes one protein that is the target of an anti-HIV drug.

76. The method of claim 72, wherein the patient-derived segment encodes two or more proteins that are the targets of one or more anti-HIV drugs.

77. The method of claim 72, wherein the patient-derived segment comprises an HIV gene.

78. A method for determining susceptibility for an anti-HIV drug comprising:
(a) introducing a resistance test vector comprising a HIV patient-derived segment and an indicator into a host cell;
(b) culturing the host cell from (a);
(c) measuring the indicator in a target host cell, wherein a change in the indicator is dependent upon the HIV patient-derived segment; and
(d) comparing the measurement of the indicator from (c) with the measurement of the indicator when steps (a)-(c) are carried out in the absence of the anti-HIV drug, wherein a test concentration of the anti-HIV drug is present at steps (a)-(c); at steps (b)-(c); or at step (c) .

79. The method of claim 78, wherein the indicator comprises a DNA structure.

80. The method of claim 79, wherein the indicator comprises a RNA structure.

81. A method for evaluating the biological effectiveness of a candidate anti-HIV drug compound comprising:
(a) introducing a resistance test vector comprising a HIV patient-derived segment and an indicator into a host cell;
(b) culturing the host cell from step (a);
(c) measuring the indicator in a target host cell, wherein a change in the indicator is dependent upon the HIV patient-derived segment; and
(d) comparing the measurement of the indicator from step (c) with the measurement of the indicator measured when steps (a)-(c) are carried out in the absence of the candidate anti-HIV drug compound, wherein a test concentration of the candidate anti-HIV drug compound is present at steps (a)-(c); at steps (b)-(c) ; or at step (c).

82. The method of claim 81, wherein the indicator comprises a DNA structure.

83. The method of claim 81, wherein the indicator comprises a RNA structure.

## Patentansprüche

1. Verfahren zur Bestimmung des Ansprechens auf ein Anti-HIV-Arzneimittel, umfassend:
(a) das Einführen eines Resistenztestvektors, der ein von einem HIV-Patienten stammendes Segment und ein Indikator-Gen umfasst, in eine Wirtszelle;
(b) das Kultivieren der unter (a) beschriebenen Wirtszelle;
(c) das Messen der Expression des Indikator-Gens in einer Ziel-Wirtszelle, wobei die Expression des Indikators von dem vom HIV-Patienten stammenden Segment abhängig ist; und
(d) das Vergleichen der unter (c) beschriebenen Expression des Indikator-Gens mit der Expression des Indikator-Gens, die gemessen wird, wenn die Schritte (a) bis (c) in Abwesenheit des Anti-HIV-Arzneimittels durchgeführt werden, worin eine Testkonzentration des Anti-HIV-Arzneimittels in den Schritten (a) bis (c); in den Schritten (b) und (c); oder in Schritt (c) vorhanden ist.

2. Verfahren nach Anspruch 1, worin der Resistenztestvektor DNA aus einem genomischen viralen Vektor umfasst.

3. Verfahren nach Anspruch 1, worin der Resistenztestvektor DNA aus einem subgenomischen viralen Vektor umfasst.

4. Verfahren zur Bestimmung von Anti-HIV-Arzneimittel-Resistenz bei einem Patienten, umfassend:
(a) das Entwickeln einer Standardkurve des Ansprechens auf ein Arzneimittel für ein Anti-HIV-Arzneimittel;
(b) das Bestimmen des Ansprechens des Patienten auf Anti-HIV-Arzneimittel nach einem Verfahren nach Anspruch 1; und
(c) das Vergleichen des unter Schritt (b) beschriebenen Ansprechens auf Anti-HIV-Arzneimittel mit einer in Schritt (a) ermittelten Standardkurve, worin eine Abnahme des Ansprechens auf Anti-HIV-Arzneimittel die Entwicklung einer Anti-HIV-Arzneimittel-Resistenz beim Patienten anzeigt.

5. Verfahren nach Anspruch 1, worin der Resistenztestvektor DNA von HIV umfasst.

6. Verfahren nach Anspruch 5, worin der Resistenztestvektor DNA umfasst, die für vif, vpr, tat, rev, vpu und nef kodiert.

7. Verfahren nach Anspruch 1, worin das vom Patienten stammende Segment eine funktionelle virale Sequenz umfasst.

8. Verfahren nach Anspruch 1, worin das vom Patienten stammende Segment für ein Protein kodiert, welches das Ziel eines Anti-HIV-Arzneimittels ist.

9. Verfahren nach Anspruch 1, worin das vom Patienten stammende Segment für zwei oder mehr Proteine kodiert, welche die Ziele eines oder mehrerer Anti-HIV-Arzneimittel(s) sind.

10. Verfahren zur Bestimmung von Anti-HIV-Arzneimittel-Resistenz bei einem Patienten, umfassend:
(a) das Bestimmen des Ansprechens des Patienten auf Anti-HIV-Arzneimittel zu einem ersten Zeitpunkt nach einem Verfahren nach Anspruch 1, worin das vom Patienten stammende Segment etwa zu diesem Zeitpunkt vom Patienten erhalten wird;
(b) das Bestimmen des Ansprechens desselben Patienten auf Anti-HIV-Arzneimittel zu einem späteren Zeitpunkt; und
(c) das Vergleichen des jeweils in Schritt (a) und (b) ermittelten Ansprechens auf Anti-HIV-Arzneimittel, wobei eine Verringerung des Ansprechens auf Anti-HIV-Arzneimittel zum späteren Zeitpunkt im Vergleich zum ersten Zeitpunkt die Entwicklung oder das Fortschreiten von Anti-HIV-Arzneimittel-Resistenz beim Patienten anzeigt.

11. Verfahren nach Anspruch 1, worin das vom Patienten stammende Segment ein HIV-Gen umfasst.

12. Verfahren nach Anspruch 11, worin das vom Patienten stammende Segment ein HIV-gag-pol-Gen umfasst.

13. Verfahren nach Anspruch 1, worin das Indikator-Gen ein funktionelles Indikator-Gen ist und die Wirtszelle eine Resistenztestvektor-Wirtszelle ist, umfassend den zusätzlichen Schritt des Infizierens der Ziel-Wirtszelle mit viralen Resistenztestvektor-PartikeIn unter Verwendung von filtrierten Überständen der Resistenztestvektor-Wirtszellen.

14. Verfahren nach Anspruch 1, worin das Indikator-Gen ein nicht-funktionelles Indikator-Gen ist.

15. Verfahren nach Anspruch 14, worin die Wirtszelle eine verpackende Wirtszelle / Resistenztestvektor-Wirtszelle ist.

16. Verfahren nach Anspruch 15, worin das Kultivieren durch Cokultivierung erfolgt.

17. Verfahren nach Anspruch 15, worin die Ziel-Wirtszelle mit viralen Resistenztestvektor-Partikeln unter Verwendung von filtrierten Überständen der verpackenden Wirtszellen / Resistenztestvektor-Wirtszellen infiziert wird.

18. Verfahren nach Anspruch 1, worin das Indikator-Gen ein Luciferase-Gen ist.

19. Verfahren nach Anspruch 1, worin das Indikator-Gen ein E.coli-lacZ-Gen ist.

20. Verfahren nach Anspruch 15, worin die verpackende Wirtszelle / Resistenztestvektor-Wirtszelle eine menschliche Zelle ist.

21. Verfahren nach Anspruch 15, worin die verpackende Wirtszelle / Resistenztestvektor-Wirtszelle eine menschliche embryonale Nierenzelle ist.

22. Verfahren nach Anspruch 15, worin die verpackende Wirtszelle / Resistenztestvektor-Wirtszelle eine 293-Zelle ist.

23. Verfahren nach Anspruch 1, worin die Ziel-Wirtszelle eine menschliche T-Zelle ist.

24. Verfahren nach Anspruch 1, worin die Ziel-Wirtszelle eine menschliche T-Zellen-Leukämie-Zelllinie ist.

25. Verfahren nach Anspruch 1, worin die Ziel-Wirtszelle eine Jurkat-Zelllinie ist.

26. Verfahren nach Anspruch 1, worin die Ziel-Wirtszelle eine H9-Zelllinie ist.

27. Verfahren nach Anspruch 1, worin die Ziel-Wirtszelle eine CEM-Zelllinie ist.

28. Verfahren nach Anspruch 1, worin die Ziel-Wirtszelle eine menschliche embryonale Nierenzelle ist.

29. Verfahren nach Anspruch 1, worin die Ziel-Wirtszelle eine 293-Zelle ist.

30. Resistenztestvektor, umfassend ein von einem HIV-Patienten stammendes Segment und ein Indikator-Gen, worin die Expression des Indikator-Gens von dem vom Patienten stammenden Segment abhängt.

31. Resistenztestvektor nach Anspruch 30, worin das vom Patienten stammende Segment für ein Protein kodiert, welches das Ziel eines Anti-HIV-Arzneimittels ist.

32. Resistenztestvektor nach Anspruch 30, worin das vom Patienten stammende Segment für zwei oder mehr Proteine kodiert, welche die Ziele eines oder mehrerer Anti-HIV-Arzneimittel(s) sind.

33. Resistenztestvektor nach Anspruch 30, worin das vom Patienten stammende Segment ein HIV-Gen umfasst.

34. Resistenztestvektor nach Anspruch 30, worin das vom Patienten stammende Segment DNA von HIV umfasst.

35. Resistenztestvektor nach Anspruch 34, worin das vom Patienten stammende Segment DNA umfasst, die für vif, vpr, tat, rev, vpu und nef kodiert.

36. Resistenztestvektor nach Anspruch 33, worin das vom Patienten stammende Segment ein HIV-gag-pol-Gen umfasst.

37. Resistenztestvektor nach Anspruch 30, worin das Indikator-Gen ein funktionelles Indikator-Gen ist.

38. Resistenztestvektor nach Anspruch 30, worin das Indikator-Gen ein nicht-funktionelles Indikator-Gen ist.

39. Resistenztestvektor nach Anspruch 30, worin das Indikator-Gen ein Luciferase-Gen ist.

40. Verpackende Wirtszelle, die mit einem Resistenztestvektor nach Anspruch 30 transfiziert ist.

41. Verpackende Wirtszelle nach Anspruch 40, die eine Säugetier-Wirtszelle ist.

42. Verpackende Wirtszelle nach Anspruch 40, die eine menschliche Wirtszelle ist.

43. Verpackende Wirtszelle nach Anspruch 40, die eine menschliche embryonale Nierenzelle ist.

44. Verpackende Wirtszelle nach Anspruch 40, die eine 293-Zelle ist.

45. Resistenztestvektor nach Anspruch 30, umfassend einen viralen Indikator-Gen-Vektor und einen verpackenden Vektor, wobei der virale Indikator-Gen-Vektor ein Indikator-Gen umfasst und der verpackende Vektor ein von einem Patienten stammendes Segment umfasst.

46. Verfahren zur Bestimmung des Ansprechens auf ein Anti-HIV-Arzneimittel, umfassend:
(a) das Einführen eines Resistenztestvektors, der ein von einem HIV-Patienten stammendes Segment und ein nichtfunktionelles Indiktator-Gen umfasst, in eine Wirtszelle;
(b) das Kultivieren der unter (a) beschriebenen Wirtszelle;
(c) das Messen der Expression des Indikator-Gens in einer Ziel-Wirtszelle, wobei die Expression des Indikator-Gens von dem vom HIV-Patienten stammenden Segment abhängig ist; und
(d) das Vergleichen der unter (c) beschriebenen Expression des Indikator-Gens mit der Expression des Indikator-Gens, die gemessen wird, wenn die Schritte (a) bis (c) in Abwesenheit des Anti-HIV-Arzneimittels durchgeführt werden, worin eine Testkonzentration des Anti-HIV-Arzneimittels in den Schritten (a) bis (c); in den Schritten (b) und (c); oder in Schritt (c) vorhanden ist.

47. Verfahren nach Anspruch 46, worin der Resistenztestvektor DNA aus einem genomischen viralen Vektor umfasst.

48. Verfahren nach Anspruch 46, worin der Resistenztestvektor DNA aus einem subgenomischen viralen Vektor umfasst.

49. Verfahren nach Anspruch 46, worin der Resistenztestvektor DNA von HIV umfasst.

50. Verfahren nach Anspruch 49, worin der Resistenztestvektor DNA umfasst, die für vif, vpr, tat, rev, vpu und nef kodiert.

51. Verfahren nach Anspruch 46, worin das vom Patienten stammende Segment für ein Protein kodiert, welches das Ziel eines Anti-HIV-Arzneimittels ist.

52. Verfahren nach Anspruch 46, worin das vom Patienten stammende Segment für zwei oder mehr Proteine kodiert, welche die Ziele eines oder mehrerer Anti-HIV-Arzneimittel(s) sind.

53. Verfahren nach Anspruch 46, worin das vom Patienten stammende Segment ein HIV-Gen umfasst.

54. Verfahren nach Anspruch 53, worin das vom Patienten stammende Segment ein HIV-gag-pol-Gen umfasst.

55. Verfahren nach Anspruch 46, worin das Indikator-Gen ein Luciferase-Gen ist.

56. Verfahren nach Anspruch 46, worin die Wirtszelle eine verpackende Wirtszelle ist.

57. Verfahren nach Anspruch 46, worin die verpackende Wirtszelle eine menschliche Zelle ist.

58. Verfahren nach Anspruch 46, worin die verpackende Wirtszelle eine menschliche embryonale Nierenzelle ist.

59. Verfahren nach Anspruch 46, worin die verpackende Wirtszelle eine 293-Zelle ist.

60. Verfahren nach Anspruch 46, worin das nichtfunktionelle Indikator-Gen einen permutierten Promotor umfasst.

61. Verfahren nach Anspruch 46, worin das nichtfunktionelle Indikator-Gen eine permutierte Kodierungsregion umfasst.

62. Verfahren nach Anspruch 46, worin das nichtfunktionelle Indikator-Gen ein invertiertes Intron umfasst.

63. Verfahren nach Anspruch 46, worin die Wirtszelle und die Ziel-Wirtszelle dieselbe Zelle sind.

64. Verfahren nach Anspruch 46, worin die Ziel-Wirtszelle eine menschliche Zelle ist.

65. Verfahren nach Anspruch 46, worin die Ziel-Wirtszelle eine menschliche T-Zelle ist.

66. Verfahren nach Anspruch 46, worin die Ziel-Wirtszelle eine menschliche embryonale Nierenzelle ist.

67. Verfahren nach Anspruch 46, worin die Ziel-Wirtszelle eine 293-Zelle ist.

68. Verfahren nach Anspruch 46, worin die Ziel-Wirtszelle mit viralen Resistenztestvektor-Partikeln infiziert wird, indem filtrierte Überstände der verpackenden Wirtszelle / Resistenztestvektor-Wirtszelle verwendet werden.

69. Verfahren nach Anspruch 46, worin das Kultivieren durch Cokultivierung erfolgt.

70. Verfahren zur Bestimmung von Anti-HIV-Arzneimittel-Resistenz bei einem Patienten, umfassend:
(a) das Entwickeln einer Standardkurve des Ansprechens auf Arzneimittel für ein Anti-HIV-Arzneimittel;
(b) das Bestimmen des Ansprechens des Patienten auf Anti-HIV-Arzneimittel nach einem Verfahren nach Anspruch 1; und
(c) das Vergleichen des in Schritt (b) beschriebenen Ansprechens auf Anti-HIV-Arzneimittel mit der in Schritt (a) ermittelten Standardkurve, worin eine Verringerung des Ansprechens auf Anti-HIV-Arzneimittel die Entwicklung einer Anti-HIV-Arzneimittel-Resistenz beim Patienten anzeigt.

71. Verfahren zur Bestimmung von Anti-HIV-Arzneimittel-Resistenz bei einem Patienten, umfassend:
(a) das Bestimmen des Ansprechens des Patienten auf Anti-HIV-Arzneimittel zu einem ersten Zeitpunkt nach einem Verfahren nach Anspruch 46, wobei das vom Patienten stammende Segment etwa zur selben Zeit vom Patienten erhalten wird;
(b) das Bestimmen des Ansprechens desselben Patienten auf Anti-HIV-Arzneimittel zu einem späteren Zeitpunkt; und
(c) das Vergleichen des in Schritt (a) und Schritt (b) bestimmten Ansprechens auf Anti-HIV-Arzneimittel, worin eine Verringerung des Ansprechens auf Anti-HIV-Arzneimittel zum späteren Zeitpunkt im Vergleich zum früheren Zeitpunkt das Entwickeln oder Fortschreiten von Anti-HIV-Arzneimittel-Resistenz beim Patienten anzeigt.

72. Verfahren zum Bewerten der biologischen Wirksamkeit einer Kandidaten-Anti-HIV-Arzneimittelverbindung, umfassend:
(a) das Einführen eines Resistenztestvektors, der ein von einem HIV-Patienten stammendes Segment und ein Indikator-Gen umfasst, in eine Wirtszelle;
(b) das Kultivieren der unter Schritt (a) beschriebenen Wirtszelle;
(c) das Messen der Expression des Indikator-Gens in einer Ziel-Wirtszelle, worin die Expression des Indikator-Gen vom dem vom HIV-Patienten stammenden Segment abhängt; und
(d) das Vergleichen der unter Schritt (c) beschriebenen Expression des Indikator-Gens mit der Expression des Indikator-Gens, die gemessen wird, wenn die Schritte (a) bis (c) in Abwesenheit der Kandidaten-Anti-HIV-Arzneimittelverbindung durchgeführt werden, worin die Testkonzentration der Kandidaten-Anti-HIV-Arzneimittelverbindung in den Schritten (a) bis (c); in den Schritten (b) und (c); oder in Schritt (c) vorhanden ist.

73. Verfahren nach Anspruch 72, worin der Resistenztestvektor DNA von HIV umfasst.

74. Verfahren nach Anspruch 72, worin der Resistenztestvektor DNA umfasst, die für HIV-gag-pol kodiert.

75. Verfahren nach Anspruch 72, worin das vom Patienten stammende Segment für ein Protein kodiert, welches das Ziel eines Anti-HIV-Arzneimittels ist.

76. Verfahren nach Anspruch 72, worin das vom Patienten stammende Segment für zwei oder mehr Proteine kodiert, welche die Ziele eines oder mehrerer Anti-HIV-Arzneimittel(s) sind.

77. Verfahren nach Anspruch 72, worin das vom Patienten stammende Segment ein HIV-Gen umfasst.

78. Verfahren zur Bestimmung des Ansprechens auf ein Anti-HIV-Arzneimittel, umfassend:
(a) das Einführen eines Resistenztestvektors, der ein von einem HIV-Patienten stammendes Segment und einen Indikator umfasst, in eine Wirtszelle;
(b) das Kultivieren der unter (a) beschriebenen Wirtszelle;
(c) das Messen des Indikators in einer Ziel-Wirtszelle, worin eine Änderung des Indikators von dem vom HIV-Patienten stammenden Segment abhängig ist; und
(d) das Vergleichen der unter (c) beschriebenen Messung des Indikators mit der Messung des Indikators, wenn die Schritte (a) bis (c) in Abwesenheit des Anti-HIV-Arzneimittels durchgeführt werden, worin eine Testkonzentration des Anti-HIV-Arzneimittels in den Schritten (a) bis (c); in den Schritten (b) und (c); oder in Schritt (c) vorhanden ist.

79. Verfahren nach Anspruch 78, worin der Indikator eine DNA-Struktur umfasst.

80. Verfahren nach Anspruch 79, worin der Indikator eine RNA-Struktur umfasst.

81. Verfahren zum Bewerten der biologischen Wirksamkeit einer Kandidaten-Anti-HIV-Arzneimittelverbindung, umfassend:
(a) das Einführen eines Resistenztestvektors, der ein von einem HIV-Patienten stammendes Segment und einen Indikator umfasst, in eine Wirtszelle;
(b) das Kultivieren der unter Schritt (a) beschriebenen Wirtszelle;
(c) das Messen des Indikators in einer Ziel-Wirtszelle, worin eine Änderung des Indikators von dem vom Patienten stammenden Segment abhängig ist; und
(d) das Vergleichen der unter Schritt (c) beschriebenen Messung des Indikators mit der Messung des Indikators, der gemessen wird, wenn die Schritte (a) bis (c) in Abwesenheit der Kandidaten-Anti-HIV-Arzneimittelverbindung durchgeführt werden, worin eine Testkonzentration der Kandidaten-Anti-HIV-Arzneimittelverbindung in den Schritten (a) bis (c); in den Schritten (b) und (c); oder in Schritt (c) vorhanden ist.

82. Verfahren nach Anspruch 81, worin der Indikator eine DNA-Struktur umfasst.

83. Verfahren nach Anspruch 81, worin der Indikator eine RNA-Struktur umfasst.

## Revendications

1. Méthode pour déterminer la sensibilité vis-à-vis d'un médicament anti-VIH comprenant :
(a) l'introduction d'un vecteur de test de résistance comprenant un segment dérivé du patient de VIH et d'un gène indicateur dans une cellule hôte;
(b) la culture de la cellule hôte de (a);
(c) la mesure de l'expression du gène indicateur dans une cellule hôte cible, où l'expression du gène indicateur dépend du segment dérivé du patient de VIH; et
(d) la comparaison de l'expression du gène indicateur de (c) avec l'expression du gène indicateur mesurée quand les étapes (a) - (c) sont effectuées en l'absence du médicament anti-VIH, où une concentration de test du médicament anti-VIH est présente aux étapes (a) - (c); aux étapes (b) - (c); ou à l'étape (c).

2. Méthode de la revendication 1, où le vecteur de test de résistance comprend l'ADN d'un vecteur génomique viral.

3. Méthode de la revendication 1, où le vecteur de test de résistance comprend l'ADN d'un vecteur viral subgénomique.

4. Méthode pour déterminer la résistance à un médicament anti-VIH chez un patient comprenant :
(a) le développement d'une courbe standard de sensibilité vis-à-vis du médicament pour un médicament anti-VIH;
(b) la détermination de la sensibilité vis-à-vis du médicament anti-VIH chez le patient selon la méthode de la revendication 1;
(c) la comparaison de la sensibilité vis-à-vis du médicament anti-VIH à l'étape (b) avec la courbe standard déterminée à l'étape (a), où une diminution de la sensibilité anti-VIH indique le développement d'une résistance au médicament anti-VIH chez le patient.

5. Méthode de la revendication 1, où le vecteur de test de résistance comprend l'ADN de VIH.

6. Méthode de la revendication 5, où le vecteur de test de résistance comprend l'ADN codant pour vif, vpr, tat, rev, vpu et nef.

7. Méthode de la revendication 1, où le segment dérivé du patient comprend une séquence virale fonctionnelle.

8. Méthode de la revendication 1, où le segment dérivé du patient code pour une protéine qui est la cible d'un médicament anti-VIH.

9. Méthode de la revendication 1, où le segment dérivé du patient code pour deux protéines ou plus qui sont les cibles d'un ou plusieurs médicaments anti-VIH.

10. Méthode pour déterminer la résistance à un médicament anti-VIH chez un patient comprenant :
(a) la détermination de la sensibilité vis-à-vis du médicament anti-VIH chez le patient en un premier temps selon la méthode de la revendication 1, où le segment dérivé du patient est obtenu du patient à peu près en même temps;
(b) la détermination de la sensibilité vis-à-vis du médicament anti-VIH du même patient en un temps ultérieur; et
(c) la comparaison des sensibilités vis-à-vis du médicament anti-VIH déterminées aux étapes (a) et (b), où une diminution de la sensibilité vis-à-vis du médicament anti-VIH au temps ultérieur, comparée au premier temps indique un développement ou une progression de la résistance au médicament anti-VIH chez la patient.

11. Méthode de la revendication 1, où le segment dérivé du patient comprend un gène de VIH.

12. Méthode de la revendication 11, où le segment dérivé du patient comprend un gène gag-pol de VIH.

13. Méthode de la revendication 1, où le gène indicateur est un gène indicateur fonctionnel et la cellule hôte est une cellule hôte de vecteur de test de résistance comprenant l'étape additionnelle d'infecter la cellule hôte cible de particules virales du vecteur de test de résistance en utilisant les produits surnageants filtrés desdites cellules hôtes du vecteur de test de résistance.

14. Méthode de la revendication 1, où le gène indicateur est un gène indicateur non fonctionnel.

15. Méthode de la revendication 14, où la cellule hôte est une cellule hôte d'empaquetage/cellule hôte du vecteur de test de résistance.

16. Méthode de la revendication 15, où la culture est par co-culture.

17. Méthode de la revendication 15, où la cellule hôte cible est infectée par des particules virales du vecteur de test de résistance en utilisant les produits surnageants filtrés de ladite cellule hôte d'empaquetage/cellules hôtes de vecteur de test de résistance.

18. Méthode de la revendication 1, où le gène indicateur est un gène de luciférase.

19. Méthode de la revendication 1, où le gène indicateur est un gène lacZ de E.coli.

20. Méthode de la revendication 15, où la cellule hôte d'empaquetage/cellule hôte du vecteur de test de résistance est une cellule humaine.

21. Méthode de la revendication 15, où la cellule hôte d'empaquetage/cellule hôte du vecteur de test de résistance est une cellule de rein embryonnaire humain.

22. Méthode de la revendication 15, où la cellule hôte d'empaquetage/cellule hôte du vecteur de test de résistance est une cellule 293.

23. Méthode de la revendication 1, où la cellule hôte cible est une cellule T humaine.

24. Méthode de la revendication 1, où la cellule hôte cible est une lignée de cellules de leucémie de cellules T humaines.

25. Méthode de la revendication 1, où la cellule hôte cible est une lignée de cellules de Jurkat.

26. Méthode de la revendication 1, où la cellule hôte cible est une lignée de cellules H9.

27. Méthode de la revendication 1, où la cellule hôte cible est une lignée de cellules CEM.

28. Méthode de la revendication 1, où la cellule hôte cible est une cellule de rein embryonnaire humain.

29. Méthode de la revendication, où la cellule hôte cible est une cellule 293.

30. Vecteur de test de résistance comprenant un segment dérivé d'un patient de VIH et un gène indicateur, où l'expression du gène indicateur dépend du segment dérivé du patient.

31. Vecteur de test de résistance de la revendication 30, où le segment dérivé du patient code pour une protéine qui est la cible d'un médicament anti-VIH.

32. Vecteur de test de résistance de la revendication 30, où le segment dérivé du patient code pour deux protéines ou plus qui sont les cibles d'un ou plusieurs médicaments anti-VIH.

33. Vecteur de test de résistance de la revendication 30, où le segment dérivé du patient comprend un gène de VIH.

34. Vecteur de test de résistance de la revendication 30, où le segment dérivé du patient comprend l'ADN de VIH.

35. Vecteur de test de résistance de la revendication 34, où le segment dérivé du patient comprend un ADN codant pour vif, vpr, tat, rev, vpu et nef.

36. Vecteur de test de résistance de la revendication 33, où le segment dérivé du patient comprend un gène gag-pol de VIH.

37. Vecteur de test de résistance de la revendication 30, où le gène indicateur est un gène indicateur fonctionnel.

38. Vecteur de test de résistance de la revendication 30, où le gène indicateur est un gène indicateur non fonctionnel.

39. Vecteur de test de résistance de la revendication 30, où le gène indicateur est un gène de luciférase.

40. Cellule hôte d'empaquetage transfectée avec un vecteur de test de résistance de la revendication 30.

41. Cellule hôte d'empaquetage de la revendication 40 qui est une cellule hôte mammalienne.

42. Cellule hôte d'empaquetage de la revendication 40 qui est une cellule hôte humaine.

43. Cellule hôte d'empaquetage de la revendication 40 qui est une cellule de rein embryonnaire humain.

44. Cellule hôte d'empaquetage de la revendication 40 qui est des cellules 293.

45. Vecteur de test de résistance de la revendication 30 comprenant un vecteur viral d'un gène indicateur et un vecteur d'empaquetage, ledit vecteur viral de gène indicateur comprenant un gène indicateur et ledit vecteur d'empaquetage comprenant un segment dérivé du patient.

46. Méthode pour déterminer la sensibilité vis-vis d'un médicament anti-VIH comprenant :
(a) l'introduction d'un vecteur de test de résistance comprenant un segment dérivé d'un patient de VIH et un indicateur non fonctionnel est un gène dans une cellule hôte;
(b) la culture de la cellule hôte de (a);
(c) la mesure de l'expression du gène indicateur dans une cellule hôte cible, où l'expression du gène indicateur dépend du segment dérivé du patient de VIH; et
(d) la comparaison de l'expression du gène indicateur de (c) avec l'expression du gène indicateur mesurée quand les étapes (a) - (c) sont effectuées en l'absence du médicament anti-VIH,
où une concentration de test du médicament anti-VIH est présente aux étapes (a) - (c); aux étapes (b) - (c); ou à l'étape (c).

47. Méthode de la revendication 46, où le vecteur de test de résistance comprend l'ADN d'un vecteur viral génomique.

48. Méthode de la revendication 46, où le vecteur de test de résistance comprend l'ADN d'un vecteur viral subgénomique.

49. Méthode de la revendication 46, où le vecteur de test de résistance comprend l'ADN de VIH.

50. Méthode de la revendication 49, où le vecteur de test de résistance comprend l'ADN codant pour vif, vpr, tat, rev, vpu, et nef.

51. Méthode de la revendication 46, où le segment dérivé du patient code pour une protéine qui est la cible d'un médicament anti-VIH.

52. Méthode de la revendication 46, où le segment dérivé du patient code pour deux protéines ou plus qui sont les cibles d'un ou plusieurs médicaments anti-VIH.

53. Méthode de la revendication 46, où le segment dérivé du patient comprend un gène de VIH.

54. Méthode de la revendication 53, où le segment dérivé du patient comprend un gène gag-pol de VIH.

55. Méthode de la revendication 46, où le gène indicateur est un gène de luciférase.

56. Méthode de la revendication 46, où la cellule hôte est une cellule hôte d'empaquetage.

57. Méthode de la revendication 46, où la cellule hôte d'empaquetage est une cellule humaine.

58. Méthode de la revendication 46, où la cellule hôte d'empaquetage est une cellule de rein embryonnaire humain.

59. Méthode de la revendication 46, où la cellule hôte d'empaquetage est une cellule 293.

60. Méthode de la revendication 46, où le gène indicateur non fonctionnel comprend un promoteur permuté.

61. Méthode de la revendication 46, où le gène indicateur non fonctionnel comprend une région de codage permutée.

62. Méthode de la revendication 46, où le gène indicateur non fonctionnel comprend un intron inversé.

63. Méthode de la revendication 46, où la cellule hôte et la cellule hôte cible sont sur la même cellule.

64. Méthode de la revendication 46, où la cellule hôte cible est une cellule humaine.

65. Méthode de la revendication 46, où la cellule hôte cible est une cellule T humaine.

66. Méthode de la revendication 46, où la cellule hôte cible est une cellule de rein embryonnaire humain.

67. Méthode de la revendication 46, où la cellule hôte cible est une cellule 293.

68. Méthode de la revendication 46, où la cellule hôte cible est infectée de particules virales d'un vecteur de test de résistance en utilisant les produits surnageants filtrés de ladite cellule hôte d'empaquetage/cellule hôte du vecteur de test de résistance.

69. Méthode de la revendication 46, où ladite culture est par co-culture.

70. Méthode pour déterminer la résistance vis-à-vis d'un médicament anti-VIH chez un patient comprenant :
(a) le développement d'une courbe standard de sensibilité vis-à-vis du médicament pour un médicament anti-VIH.
(b) la détermination d'une sensibilité vis-à-vis d'un médicament anti-VIH chez le patient selon la méthode de la revendication 1; et
(c) la comparaison de la sensibilité vis-à-vis du médicament anti-VIH de l'étape (b) avec la courbe standard déterminée à l'étape (a), où une diminution de la sensibilité anti-VIH indique le développement d'une résistance au médicament anti-VIH chez le patient.

71. Méthode pour déterminer la résistance vis-à-vis du médicament anti-VIH chez un patient comprenant :
(a) la détermination de la sensibilité vis-à-vis du médicament anti-VIH chez le patient en un premier temps selon la méthode de la revendication 46, où le segment dérivé du patient est obtenu du patient à peu près en même temps.
(b) la détermination de la sensibilité vis-à-vis du médicament anti-VIH du même patient en un temps ultérieur; et
(c) la comparaison des sensibilités vis-à-vis du médicament anti-VIH déterminées à l'étape (a) et (b), où une diminution de la sensibilité au médicament anti-VIH au temps ultérieur comparée au premier temps indique le développement
ou la progression de la résistance vis-à-vis du médicament anti-VIH chez le patient.

72. Méthode pour l'évaluation de l'efficacité biologique d'un composé médicament anti-VIH candidat comprenant :
(a) l'introduction d'un vecteur de test de résistance comprenant un segment dérivé d'un patient de VIH et d'un gène indicateur dans une cellule hôte;
(b) la culture de la cellule hôte de l'étape (a);
(c) la mesure de l'expression du gène indicateur dans une cellule hôte cible, où l'expression du gène indicateur dépend du segment dérivé du patient de VIH; et
(d) la comparaison de l'expression du gène indicateur de l'étape (c) avec l'expression du gène indicateur mesuré quand les étapes (a) - (c) sont effectuées en l'absence du composé du médicament anti-VIH candidat, où une concentration de test du composé du médicament anti-VIH candidat est présente aux étapes (a) - (c); aux étapes (b) - (c) ou à l'étape (c) .

73. Méthode de la revendication 72, où le vecteur de test de résistance comprend l'ADN de VIH.

74. Méthode de la revendication 72, où le vecteur de test de résistance comprend l'ADN codant pour gag-pol de VIH.

75. Méthode de la revendication 72, où le segment dérivé du patient code pour une protéine qui est la cible d'un médicament anti-VIH.

76. Méthode de la revendication 72, où le segment dérivé du patient code pour deux protéines ou plus qui sont les cibles d'un ou plusieurs médicaments anti-VIH.

77. Méthode de la revendication 72, où le segment dérivé du patient comprend un gène de VIH.

78. Méthode pour déterminer la sensibilité vis-à-vis d'un médicament anti-VIH comprenant :
(a) l'introduction d'un vecteur de test de résistance comprenant un segment dérivé du patient de VIH et un indicateur dans une cellule hôte ;
(b) la culture de la cellule hôte de (a) ;
(c) la mesure de l'indicateur dans une cellule hôte cible, où un changement de l'indicateur dépend du segment dérivé du patient de VIH ; et
(d) la comparaison de la mesure de l'indicateur de (c) avec la mesure de l'indicateur quand les étapes (a)-(c) sont effectuées en l'absence du médicament anti-VIH, où une concentration de test du médicament anti-VIH est présente aux étapes (a)-(c) ; aux étapes (b)-(c) ; ou à l'étape (c).

79. Méthode de la revendication 78, où l'indicateur comprend une structure d'ADN.

80. Méthode de la revendication 79, où l'indicateur comprend une structure d'ARN.

81. Méthode pour évaluer l'efficacité biologique d'un composé d'un médicament anti-VIH candidat comprenant :
(a) l'introduction d'un vecteur de test de résistance comprenant un segment dérivé du patient de VIH et un indicateur dans une cellule hôte ;
(b) la culture de la cellule hôte de l'étape (a) ;
(c) la mesure de l'indicateur dans une cellule hôte cible, où un changement de l'indicateur dépend du segment dérivé du patient de VIH ; et
(d) la comparaison de la mesure de l'indicateur de l'étape (c) avec la mesure de l'indicateur mesurée quand les étapes (a)-(c) sont effectuées en l'absence du composé du médicament anti-VIH candidat, où une concentration de test du composé du médicament anti-VIH candidat est présente aux étapes (a) - (c) ; aux étapes (b) - (c) ; ou à l'étape (c).

82. Méthode de la revendication 81, où l'indicateur comprend une structure d'ADN.

83. Méthode de la revendication 81, où l'indicateur comprend une structure d'ARN.


### SEQUENCE LISTING

(1) GENERAL INFORMATION:
   (i) APPLICANTS: Capon, Daniel J. and Christos J. Petropoulous
   (ii) TITLE OF INVENTION: Compositions And Methods For Determining Anti-viral Drug Susceptibility And Resistance
   (iii) NUMBER OF SEQUENCES: 47
   (iv) CORRESPONDENCE ADDRESS:
      (A) ADDRESSEE: Cooper & Dunham LLP
      (B) STREET: 1185 Avenue of the Americas
      (C) CITY: New York
      (D) STATE: New York
      (E) COUNTRY: United States
      (F) ZIP: 10036
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE:
      (C) CLASSIFICATION:
   (viii)ATTORNEY/AGENT INFORMATION:
      (A) NAME: White, John P.
      (B) REGISTRATION NUMBER: 28,678
      (C) REFERENCE/DOCKET NUMBER: 50130-A-PCT/JPW/AKC
   (ix) TELECOMMUNICATION INFORMATION:
      (A) TELEPHONE: 212-278-0400
      (B) TELEFAX: 212-391-0526
(2) INFORMATION FOR SEQ ID NO:1:
   (I) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:1:
   AGTGAATTAG CCCTTCCACC CGGGTCGAGC TTGGCGTAAT CA
   42
(2) INFORMATION FOR SEQ ID NO:2:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:2:
   CTGTTGGGAA GGGCGATCTC TAGATGCTAG AGATTTTCCA CA
   42
(2) INFORMATION FOR SEQ ID NO:3:
   (i) SEQUENCE CHARACTERISTICS:
      (A) LENGTH: 44 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:3:
   CTCCTCCTCC AAGTCTGAGC GGCCGCCTTT AGCATCTGAT GCAC
   44
(2) INFORMATION FOR SEQ ID NO:4:
   (i) SEQUENCE CHARACTERISTICS:
      (A) LENGTH: 44 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:4:
   CTCCTCCTCC AAGTCTGAGC GGCCGCCATA TGGTGTTTTA CTAA
   44
(2) INFORMATION FOR SEQ ID NO:5:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:5:
   GGTCTAACCA GAGAGACCCG GTTCACTAAA CGAGCT
   36
(2) INFORMATION FOR SEQ ID NO:6:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:6:
   GAATTCGCGG CCGCAATTCC GCCCCTCTCC CT
   32
(2) INFORMATION FOR SEQ ID NO:7:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:7:
   GTTAACGCGG CCGCGATATA GTTCCTCCTT TC
   32
(2) INFORMATION FOR SEQ ID NO:8:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:8:
   GAATTCTCGC GACCATGGAA GACGCCAAAA AC
   32
(2) INFORMATION FOR SEQ ID NO:9:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:9:
   GTTAACAGAT CTCTCGAGTT ACAATTTGGA CTTTCC
   36
(2) INFORMATION FOR SEQ ID NO:10:
   (i) SEQUENCE CHARACTERISTICS:
      (A) LENGTH: 56 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:10:
(2) INFORMATION FOR SEQ ID NO:11:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:11:
   AAGAGTGACC TGAGGGAAGT TAACGGATAC AGTTCCTTGT CT
   42
(2) INFORMATION FOR SEQ ID NO:12:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:12:
   TCCAGCACTG ACTAATTTGT CGACTTGTTC ATTTCCTCCA AT
   42
(2) INFORMATION FOR SEQ ID NO:13:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:13:
   TAACGCCTAT TCTGCTATGC CGACACCCAA TTCTGAAAAT GG
   42
(2) INFORMATION FOR SEQ ID NO:14:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:14:
   AAGGATACAG TTCCTTGTCG ATCGGCTCCT GCTTCTGAGG GG
   42
(2) INFORMATION FOR SEQ ID NO:15:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:15:
   CTAAAAATAG TACTTTCCGG ATCCCAGCAC TGACTAATTT AT
   42
(2) INFORMATION FOR SEQ ID NO:16:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:16:
   TTAGCTCCTT CGGTCCTCCA ATCGTTGTCA GAAGTAAGTT GG
   42
(2) INFORMATION FOR SEQ ID NO:17:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:17:
   GTCCCAGATA AGTGCCAAGG ATTCGTTCAC TAATCGAATG GA
   42
(2) INFORMATION FOR SEQ ID NO:18:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:18:
   GAATTCGTTA ACTTCCCTCA GATCACTCTT TGG
   33
(2) INFORMATION FOR SEQ ID NO:19:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:19:
   GTTAACGTCG ACTTGTTCAT TTCCTCCAAT
   30
(2) INFORMATION FOR SEQ ID NO:20:
   (i) SEQUENCE CHARACTERISTICS:
      (A) LENGTH: 55 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:20:
   GAATTCCGAT CGACAAGGAA CTGTATCCTT TAACTTCCCT CAGATCACTC TTTGG
   55
(2) INFORMATION FOR SEQ ID NO:21:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:21:
   GTTAACGGAT CCCAGCACTG ACTAATTTAT CTACTTGTTC ATTTCCTCCA AT
   52
(2) INFORMATION FOR SEQ ID NO:22:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:22:
   GAATTCGTTA ACTTCCCTCA RATCMCTCTT TGG
   33
(2) INFORMATION FOR SEQ ID NO:23:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:23:
   GTTAACGTCG ACTTKYTCAT TTCCTCCWAT
   30
(2) INFORMATION FOR SEQ ID NO:24:
   (i) SEQUENCE CHARACTERISTICS:
      (A) LENGTH: 55 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:24:
   GAATTCCGAT CGACAAGGAA CTGTATCCTT TAACTTCCCT CARATCMCTC TTTGG
   55
(2) INFORMATION FOR SEQ ID NO:25:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:25:
   GTTAACGGAT CCCAGCACTG ACTAATTTAT CTACTTKYTC ATTTCCTCCW AT
   52
(2) INFORMATION FOR SEQ ID NO:26:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:26:
   ATCTCTTACC TGTCCTATCT AACAGGCCAG GATTAA
   36
(2) INFORMATION FOR SEQ ID NO:27:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:27:
   GAATTCTCGC GACCACCATG GCGCGTTCAA CGCTC
   35
(2) INFORMATION FOR SEQ ID NO:28:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:28:
   GTTAACAGAT CTTCATGGCT CGTACTCTAT
   30
(2) INFORMATION FOR SEQ ID NO:29:
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:29:
   GAATTCGCGC GCAAGCGGCC GCAACCCGGG AAAAGCTTAA GCATGCAACC CGGGAAGAAT 60
   TCAATCGCGA AA 72
(2) INFORMATION FOR SEQ ID NO:30:
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:30:
   GTTAACGCGC GCTTCTCGAG TTGCGGCCGC TTGCTAGCTT AGATCTTTGG GCCCTTTCGC 60
   GATTGAATTC TT 72
(2) INFORMATION FOR SEQ ID NO:31:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:31:
   GAATTCAAGC TTGGCCATTG CATACGTTGT 30
(2) INFORMATION FOR SEQ ID NO:32:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:32:
   GTTAACGCAT GCATAAGAAG CCAA
   24
(2) INFORMATION FOR SEQ ID NO:33:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:33:
   GAATTCGCAT GCTCCCCTGC TCCGACCCGG
   30
(2) INFORMATION FOR SEQ ID NO:34:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:34:
   GTTAACGAAT TCTCCTGCGG GGAGAAGCAG
   30
(2) INFORMATION FOR SEQ ID NO:35:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:35:
   GAATTCAGAT CTGCCATACC ACATTTGTAG
   30
(2) INFORMATION FOR SEQ ID NO:36:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:36:
   GTTAACGCTA GCTCCAGACA TGATAAGATA
   30
(2) INFORMATION FOR SEQ ID NO:37:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:37:
   GAATTCGCTA GCATCCCGCC CCTAACTCCG
   30
(2) INFORMATION FOR SEQ ID NO:38:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:38:
   GTTAACGTCG ACGCAAAAGC CTAGGCCTCC
   30
(2) INFORMATION FOR SEQ ID NO:39:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:39:
   GAATTCTCGC GAACAGTTGG CCCT
   24
(2) INFORMATION FOR SEQ ID NO:40:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:40:
   GTTAACAGAT CTTTACGCGA ACGCGAAGTC
   30
(2) INFORMATION FOR SEQ ID NO:41:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:41:
   GTTAACGAAT TCTTGCAAAA AGCTTTGCAA GATGGATAAA GTTTTTAGAA ACTCCAGTAG 60
   GACTCC
   66
(2) INFORMATION FOR SEQ ID NO:42:
   (i) SEQUENCE CHARACTERISTICS:
      (A) LENGTH: 63 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:42:
(2) INFORMATION FOR SEQ ID NO:43:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:43:
   GTTAACGAAT TCCCACCATG ATTGAACAAG ATGGA
   35
(2) INFORMATION FOR SEQ ID NO:44:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:44:
   GAATTCAGAT CTTCAGAAGA ACTCGTCAAG
   30
(2) INFORMATION FOR SEQ ID NO:45:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:45:
   CCCCGTGCCA AGAGTGACTA CGTAAGTACC GCCTATAGA
   39
(2) INFORMATION FOR SEQ ID NO:46:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:46:
   CTCTGCTTCT CCCCGCAGCT GGAGAATTCA ATCGCGAAA
   39
(2) INFORMATION FOR SEQ ID NO:47:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:47:

### Annex

### SEQUENCE LISTING

(1) GENERAL INFORMATION:
   (i) APPLICANT:
      (A) NAME: Virologic, Inc.
      (B) STREET: 270 East Grand Avenue
      (C) CITY: South San Francisco
      (D) STATE: California
      (E) COUNTRY: USA
      (F) POSTAL CODE (ZIP): 94080
   (ii) TITLE OF INVENTION: Compositions and methods for determining anti-viral drug susceptibility and resistance and anti-viral drug screening
   (iii) NUMBER OF SEQUENCES: 47
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (v) CURRENT APPLICATION DATA:
      APPLICATION NUMBER: EP 97907549.6
   (vi) PRIOR APPLICATION DATA:
      (A) APPLICATION NUMBER: PCT/US97/01609
      (B) FILING DATE: 29-JAN-1997
   (vi) PRIOR APPLICATION DATA:
      (A) APPLICATION NUMBER: US 08/593,009
      (B) FILING DATE: 29-JAN-1996
(2) INFORMATION FOR SEQ ID NO:1:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:1:
   AGTGAATTAG CCCTTCCACC CGGGTCGAGC TTGGCGTAAT CA 42
(2) INFORMATION FOR SEQ ID NO:2:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:2:
   CTGTTGGGAA GGGCGATCTC TAGATGCTAG AGATTTTCCA CA 42
(2) INFORMATION FOR SEQ ID NO:3:
   (i) SEQUENCE CHARACTERISTICS:
      (A) LENGTH: 44 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:3:
   CTCCTCCTCC AAGTCTGAGC GGCCGCCTTT AGCATCTGAT GCAC 44
(2) INFORMATION FOR SEQ ID NO:4:
   (i) SEQUENCE CHARACTERISTICS:
      (A) LENGTH: 44 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:4:
   CTCCTCCTCC AAGTCTGAGC GGCCGCCATA TGGTGTTTTA CTAA 44
(2) INFORMATION FOR SEQ ID NO:5:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:5:
   GGTCTAACCA GAGAGACCCG GTTCACTAAA CGAGCT 36
(2) INFORMATION FOR SEQ ID NO:6:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:6:
   GAATTCGCGG CCGCAATTCC GCCCCTCTCC CT 32
(2) INFORMATION FOR SEQ ID NO:7:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:7:
   GTTAACGCGG CCGCGATATA GTTCCTCCTT TC 32
(2) INFORMATION FOR SEQ ID NO:8:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:8:
   GAATTCTCGC GACCATGGAA GACGCCAAAA AC 32
(2) INFORMATION FOR SEQ ID NO:9:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:9:
   GTTAACAGAT CTCTCGAGTT ACAATTTGGA CTTTCC 36
(2) INFORMATION FOR SEQ ID NO:10:
   (i) SEQUENCE CHARACTERISTICS:
      (A) LENGTH: 56 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:10:
   AGACGGGCAC ACACTACTTA ATACGACTCA CTATAGGGTG AAGCACTCAA GGCAAG 56
(2) INFORMATION FOR SEQ ID NO:11:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:11:
   AAGAGTGACC TGAGGGAAGT TAACGGATAC AGTTCCTTGT CT 42
(2) INFORMATION FOR SEQ ID NO:12:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:12:
   TCCAGCACTG ACTAATTTGT CGACTTGTTC ATTTCCTCCA AT 42
(2) INFORMATION FOR SEQ ID NO:13:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:13:
   TAACGCCTAT TCTGCTATGC CGACACCCAA TTCTGAAAAT GG 42
(2) INFORMATION FOR SEQ ID NO:14:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:14:
   AAGGATACAG TTCCTTGTCG ATCGGCTCCT GCTTCTGAGG GG 42
(2) INFORMATION FOR SEQ ID NO:15:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) .SEQUENCE DESCRIPTION: SEQ ID NO:15:
(2) INFORMATION FOR SEQ ID NO:16:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:16:
   TTAGCTCCTT CGGTCCTCCA ATCGTTGTCA GAAGTAAGTT GG 42
(2) INFORMATION FOR SEQ ID NO:17:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:17:
   GTCCCAGATA AGTGCCAAGG ATTCGTTCAC TAATCGAATG GA 42
(2) INFORMATION FOR SEQ ID NO:18:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:18:
   GAATTCGTTA ACTTCCCTCA GATCACTCTT TGG 33
(2) INFORMATION FOR SEQ ID NO:19:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:19:
   GTTAACGTCG ACTTGTTCAT TTCCTCCAAT 30
(2) INFORMATION FOR SEQ ID NO:20:
   (i) SEQUENCE CHARACTERISTICS:
      (A) LENGTH: 55 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:20:
   GAATTCCGAT CGACAAGGAA CTGTATCCTT TAACTTCCCT CAGATCACTC TTTGG 55
(2) INFORMATION FOR SEQ ID NO:21:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:21:
   GTTAACGGAT CCCAGCACTG ACTAATTTAT CTACTTGTTC ATTTCCTCCA AT 52
(2) INFORMATION FOR SEQ ID NO:22:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:22:
   GAATTCGTTA ACTTCCCTCA RATCMCTCTT TGG 33
(2) INFORMATION FOR SEQ ID NO:23:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:23:
   GTTAACGTCG ACTTKYTCAT TTCCTCCWAT 30
(2) INFORMATION FOR SEQ ID NO:24:
   (i) SEQUENCE CHARACTERISTICS:
      (A) LENGTH: 55 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:24:
   GAATTCCGAT CGACAAGGAA CTGTATCCTT TAACTTCCCT CARATCMCTC TTTGG 55
(2) INFORMATION FOR SEQ ID NO:25:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:25:
   GTTAACGGAT CCCAGCACTG ACTAATTTAT CTACTTKYTC ATTTCCTCCW AT 52
(2) INFORMATION FOR SEQ ID NO:26:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:26:
   ATCTCTTACC TGTCCTATCT AACAGGCCAG GATTAA 36
(2) INFORMATION FOR SEQ ID NO:27:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:27:
   GAATTCTCGC GACCACCATG GCGCGTTCAA CGCTC 35
(2) INFORMATION FOR SEQ ID NO:28:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:28:
   GTTAACAGAT CTTCATGGCT CGTACTCTAT 30
(2) INFORMATION FOR SEQ ID NO:29:
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:29:
   GAATTCGCGC GCAAGCGGCC GCAACCCGGG AAAAGCTTAA GCATGCAACC CGGGAAGAAT 60
   TCAATCGCGA AA 72
(2) INFORMATION FOR SEQ ID NO:30:
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:30:
   GTTAACGCGC GCTTCTCGAG TTGCGGCCGC TTGCTAGCTT AGATCTTTGG GCCCTTTCGC    60
   GATTGAATTC TT                                                        72
(2) INFORMATION FOR SEQ ID NO:31:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:31:
   GAATTCAAGC TTGGCCATTG CATACGTTGT                                     30
(2) INFORMATION FOR SEQ ID NO:32:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:32:
   GTTAACGCAT GCATAAGAAG CCAA                                           24
(2) INFORMATION FOR SEQ ID NO:33:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:33:
   GAATTCGCAT GCTCCCCTGC TCCGACCCGG 30
(2) INFORMATION FOR SEQ ID NO:34:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:34:
   GTTAACGAAT TCTCCTGCGG GGAGAAGCAG 30
(2) INFORMATION FOR SEQ ID NO:35:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:35:
   GAATTCAGAT CTGCCATACC ACATTTGTAG 30
(2) INFORMATION FOR SEQ ID NO:36:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:36:
   GTTAACGCTA GCTCCAGACA TGATAAGATA 30
(2) INFORMATION FOR SEQ ID NO:37:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:37:
   GAATTCGCTA GCATCCCGCC CCTAACTCCG 30
(2) INFORMATION FOR SEQ ID NO:38:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:38:
   GTTAACGTCG ACGCAAAAGC CTAGGCCTCC 30
(2) INFORMATION FOR SEQ ID NO:39:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:39:
   GAATTCTCGC GAACAGTTGG CCCT 24
(2) INFORMATION FOR SEQ ID NO:40:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:40:
   GTTAACAGAT CTTTACGCGA ACGCGAAGTC 30
(2) INFORMATION FOR SEQ ID NO:41:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:41:
   GTTAACGAAT TCTTGCAAAA AGCTTTGCAA GATGGATAAA GTTTTTAGAA ACTCCAGTAG 60
   GACTCC 66
(2) INFORMATION FOR SEQ ID NO:42:
   (i) SEQUENCE CHARACTERISTICS:
      (A) LENGTH: 63 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:42:
   GAATTCTCGC GATCTAGACG TTCTACCTTT CTCTTCTTTT TTGGAGGAGT CCTACTGGAG 60
   TTT 63
(2) INFORMATION FOR SEQ ID NO:43:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:43:
   GTTAACGAAT TCCCACCATG ATTGAACAAG ATGGA 35
(2) INFORMATION FOR SEQ ID NO:44:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:44:
   GAATTCAGAT CTTCAGAAGA ACTCGTCAAG 30
(2) INFORMATION FOR SEQ ID NO:45:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:45:
   CCCCGTGCCA AGAGTGACTA CGTAAGTACC GCCTATAGA 39
(2) INFORMATION FOR SEQ ID NO:46:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:46:
   CTCTGCTTCT CCCCGCAGCT GGAGAATTCA ATCGCGAAA 39
(2) INFORMATION FOR SEQ ID NO:47:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:47:
   GTTAACGAAT TCCCACCATG AACACGATTA ACATC 35